(19) Europäisches Patentamt
European Patent Office
Office européen des brevets

(11) **EP 4 428 154 A1**

(12) **EUROPEAN PATENT APPLICATION**
published in accordance with Art. 153(4) EPC

(43) Date of publication:
**11.09.2024 Bulletin 2024/37**

(21) Application number: **22889407.7**

(22) Date of filing: **04.11.2022**

(51) International Patent Classification (IPC):
**C07K 16/28** (2006.01)   **A61K 39/395** (2006.01)
**A61P 35/00** (2006.01)

(52) Cooperative Patent Classification (CPC):
**A61K 39/395; A61P 35/00; C07K 16/28**

(86) International application number:
**PCT/CN2022/129834**

(87) International publication number:
**WO 2023/078386 (11.05.2023 Gazette 2023/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **05.11.2021 CN 202111307619**

(71) Applicant: **CHIA TAI TIANQING
PHARMACEUTICAL GROUP CO., LTD.
Lianyungang,
Jiangsu 222062 (CN)**

(72) Inventors:
• **YING, Shusong**
**Lianyungang, Jiangsu 222062 (CN)**
• **LIU, Hui**
**Lianyungang, Jiangsu 222062 (CN)**
• **ZHAO, Kaidi**
**Lianyungang, Jiangsu 222062 (CN)**
• **ZHANG, Zhengping**
**Lianyungang, Jiangsu 222062 (CN)**

(74) Representative: **WSL Patentanwälte Partnerschaft
mbB
Kaiser-Friedrich-Ring 98
65185 Wiesbaden (DE)**

Remarks:
The complete document including Reference
Table(s) and the Sequence Listing(s) can be
downloaded from the EPO website

(54) **ANTI-CLDN18.2 ANTIBODY AND USE THEREOF**

(57)   Provided is a mouse, human, chimeric, or humanized antibody or an antigen-binding fragment thereof specifically binding to CLDN18.2, and also provided are a nucleic acid molecule encoding the antibody or the antigen-binding fragment thereof, as well an expression vector and host cell used to express the antibody or the antigen-binding fragment thereof. Further provided is a use of the antibody or the antigen-binding fragment thereof, comprising treatment of CLDN18.2-positive diseases.

EP 4 428 154 A1

**Description**

**CROSS-REFERENCE TO RELATED APPLICATIONS**

The present application is based on the international application of Chinese patent application No. 202111307619.3 filed on November 5, 2021 and claims priority and benefit of this Chinese patent application. The disclosure of the application is hereby incorporated by reference in its entirety.

**TECHNICAL FIELD**

The present application relates to the field of biotechnology and specifically to an antibody and an antigen-binding fragment thereof binding to claudin18.2 (CLDN18.2) and a method of using such antibodies and antigen-binding fragments.

**BACKGROUND**

Claudin18 (CLDN18) belongs to the family of cell tight junction proteins. It participates in the formation of intercellular tight junction, controls the flow of intercellular molecules, and plays an important role in maintaining the polarity and permeability of cells and in intercellular signal transmission. CLDN18 is a tetraspanin; its N-terminus and C-terminus are both intracellular, and two Loop regions are formed extracellularly. Due to alternative splicing of RNA, two different isoforms are present in tissues, namely claudin18.1 (CLDN18.1, e.g., the Uniprot accession No. for human CLDN18.1 is P56856-1) and claudin18.2 (CLDN18.2, e.g., the Uniprot accession No. for human CLDN18.2 is P56856-2).
CLDN18.1 and CLDN18.2 protein sequences have high similarity, but their expression distribution is different. CLDN18.1 is selectively expressed in normal lung tissues, while CLDN18.2 is expressed in normal tissues at a very limited level and is specifically expressed only in gastric mucosal differentiated epithelial cells. However, research shows that CLDN18.2 exhibits high expression in 70% of primary gastric cancers and metastases thereof, and furthermore, CLDN18.2 is found to be frequently ectopically activated in a variety of malignant tumors, including pancreatic cancer, esophageal cancer, lung cancer, breast cancer, ovarian cancer, and otorhinolaryngological tumors (Niimi et al., (2001) Mol Cell Biol 21 (21): 7380-7390; Sahin, U. et al., (2008) Clin Cancer Res. 14, 7624-34; Tanaka et al., (2011) J Histochem Cytochem 59(10): 942-952; Micke et al., (2014) Int J Cancer 135(9): 2206-2214; Shimobaba et al., (2016) Biochim Biophys Acta 1863(6Pt A): 1170-1178; Sing h et al., (2017) J Hema tol Oncol 10 (1): 105; Tokumitsu et al., (2017) Cytopathology 28(2): 116-121).
Various CLDN18.2 antibodies have been used in the study of cancer treatment, e.g., the CLDN18.2 chimeric IgG1 antibody Claudiximab (IMAB362) developed by Ganymed (Sahin et al., (2018) Eur J Cancer 100: 17-26) and the CLDN18.2 humanized antibody disclosed in PCT application WO2020211792. There is still a need for more antibodies against CLDN18.2 in clinic practice.

**SUMMARY**

In one aspect, the present disclosure provides an isolated antibody or an antigen-binding fragment thereof that binds to CLDN18.2. In some embodiments, the antibody or the antigen-binding fragment thereof binds to human CLDN18.2. In some embodiments, the antibody or the antigen-binding fragment thereof is a murine antibody, a chimeric antibody, a humanized antibody, or a human antibody. In some embodiments, the antibody or the antigen-binding fragment thereof is a monoclonal antibody, a monospecific antibody, a multispecific antibody, a Fab fragment, a F(ab')2 fragment, an Fd fragment, an Fv fragment, a dAb, an isolated CDR, a single-chain Fv molecule, a nanobody, a recombinant polypeptide, a fusion protein, an immunoconjugate, or a combination thereof.
In some embodiments, the anti-CLDN18.2 antibody or the antigen-binding fragment thereof provided herein comprises a heavy chain CDR1 (HCDR1), a heavy chain CDR2 (HCDR2), and a heavy chain CDR3 (HCDR3), wherein

> (1) the heavy chain CDR1 comprises the heavy chain CDR1 amino acid sequence in SEQ ID NO: 11, the heavy chain CDR2 comprises the heavy chain CDR2 amino acid sequence in SEQ ID NO: 11, and the heavy chain CDR3 comprises the heavy chain CDR3 amino acid sequence in SEQ ID NO: 11;
> (2) the heavy chain CDR1 comprises the heavy chain CDR1 amino acid sequence in SEQ ID NO: 12, the heavy chain CDR2 comprises the heavy chain CDR2 amino acid sequence in SEQ ID NO: 12, and the heavy chain CDR3 comprises the heavy chain CDR3 amino acid sequence in SEQ ID NO: 12;
> (3) the heavy chain CDR1 comprises the heavy chain CDR1 amino acid sequence in SEQ ID NO: 13, the heavy chain CDR2 comprises the heavy chain CDR2 amino acid sequence in SEQ ID NO: 13, and the heavy chain CDR3 comprises the heavy chain CDR3 amino acid sequence in SEQ ID NO: 13;

(4) the heavy chain CDR1 comprises the heavy chain CDR1 amino acid sequence in SEQ ID NO: 14, the heavy chain CDR2 comprises the heavy chain CDR2 amino acid sequence in SEQ ID NO: 14, and the heavy chain CDR3 comprises the heavy chain CDR3 amino acid sequence in SEQ ID NO: 14;

(5) the heavy chain CDR1 comprises the heavy chain CDR1 amino acid sequence in SEQ ID NO: 15, the heavy chain CDR2 comprises the heavy chain CDR2 amino acid sequence in SEQ ID NO: 15, and the heavy chain CDR3 comprises the heavy chain CDR3 amino acid sequence in SEQ ID NO: 15;

(6) the heavy chain CDR1 comprises the heavy chain CDR1 amino acid sequence in SEQ ID NO: 29, the heavy chain CDR2 comprises the heavy chain CDR2 amino acid sequence in SEQ ID NO: 29, and the heavy chain CDR3 comprises the heavy chain CDR3 amino acid sequence in SEQ ID NO: 29;

(7) the heavy chain CDR1 comprises the heavy chain CDR1 amino acid sequence in SEQ ID NO: 30, the heavy chain CDR2 comprises the heavy chain CDR2 amino acid sequence in SEQ ID NO: 30, and the heavy chain CDR3 comprises the heavy chain CDR3 amino acid sequence in SEQ ID NO: 30;

(8) the heavy chain CDR1 comprises the heavy chain CDR1 amino acid sequence in SEQ ID NO: 31, the heavy chain CDR2 comprises the heavy chain CDR2 amino acid sequence in SEQ ID NO: 31, and the heavy chain CDR3 comprises the heavy chain CDR3 amino acid sequence in SEQ ID NO: 31;

(9) the heavy chain CDR1 comprises the heavy chain CDR1 amino acid sequence in SEQ ID NO: 32, the heavy chain CDR2 comprises the heavy chain CDR2 amino acid sequence in SEQ ID NO: 32, and the heavy chain CDR3 comprises the heavy chain CDR3 amino acid sequence in SEQ ID NO: 32;

(10) the heavy chain CDR1 comprises the heavy chain CDR1 amino acid sequence in SEQ ID NO: 33, the heavy chain CDR2 comprises the heavy chain CDR2 amino acid sequence in SEQ ID NO: 33, and the heavy chain CDR3 comprises the heavy chain CDR3 amino acid sequence in SEQ ID NO: 33;

(11) the heavy chain CDR1 comprises the heavy chain CDR1 amino acid sequence in SEQ ID NO: 43, the heavy chain CDR2 comprises the heavy chain CDR2 amino acid sequence in SEQ ID NO: 43, and the heavy chain CDR3 comprises the heavy chain CDR3 amino acid sequence in SEQ ID NO: 43;

(12) the heavy chain CDR1 comprises the heavy chain CDR1 amino acid sequence in SEQ ID NO: 51, the heavy chain CDR2 comprises the heavy chain CDR2 amino acid sequence in SEQ ID NO: 51, and the heavy chain CDR3 comprises the heavy chain CDR3 amino acid sequence in SEQ ID NO: 51; or

(13) the heavy chain CDR1 comprises the heavy chain CDR1 amino acid sequence in SEQ ID NO: 59, the heavy chain CDR2 comprises the heavy chain CDR2 amino acid sequence in SEQ ID NO: 59, and the heavy chain CDR3 comprises the heavy chain CDR3 amino acid sequence in SEQ ID NO: 59.

In some embodiments, the anti-CLDN18.2 antibody or the antigen-binding fragment thereof provided herein comprises a heavy chain CDR1, a heavy chain CDR2, and a heavy chain CDR3, wherein

(1) the heavy chain CDR1, the heavy chain CDR2, and the heavy chain CDR3 comprise the amino acid sequences set forth in SEQ ID NOs: 3, 4, and 7, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 3, 4, and 7, respectively;

(2) the heavy chain CDR1, the heavy chain CDR2, and the heavy chain CDR3 comprise the amino acid sequences set forth in SEQ ID NOs: 3, 5, and 7, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 3, 5, and 7, respectively;

(3) the heavy chain CDR1, the heavy chain CDR2, and the heavy chain CDR3 comprise the amino acid sequences set forth in SEQ ID NOs: 3, 6, and 7, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 3, 6, and 7, respectively;

(4) the heavy chain CDR1, the heavy chain CDR2, and the heavy chain CDR3 comprise the amino acid sequences set forth in SEQ ID NOs: 19, 20, and 24, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 19, 20, and 24, respectively;

(5) the heavy chain CDR1, the heavy chain CDR2, and the heavy chain CDR3 comprise the amino acid sequences set forth in SEQ ID NOs: 19, 21, and 24, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 19, 21, and 24, respectively;

(6) the heavy chain CDR1, the heavy chain CDR2, and the heavy chain CDR3 comprise the amino acid sequences set forth in SEQ ID NOs: 19, 22, and 24, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 19, 22, and 24, respectively;

(7) the heavy chain CDR1, the heavy chain CDR2, and the heavy chain CDR3 comprise the amino acid sequences set forth in SEQ ID NOs: 19, 23, and 24, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 19, 23, and 24, respectively;

(8) the heavy chain CDR1, the heavy chain CDR2, and the heavy chain CDR3 comprise the amino acid sequences set forth in SEQ ID NOs: 37, 38, and 39, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 37, 38, and 39, respectively;

(9) the heavy chain CDR1, the heavy chain CDR2, and the heavy chain CDR3 comprise the amino acid sequences set forth in SEQ ID NOs: 45, 46, and 47, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 45, 46, and 47, respectively; or

(10) the heavy chain CDR1, the heavy chain CDR2, and the heavy chain CDR3 comprise the amino acid sequences set forth in SEQ ID NOs: 53, 54, and 55, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 53, 54, and 55, respectively.

In some embodiments, the anti-CLDN18.2 antibody or the antigen-binding fragment thereof provided herein comprises a light chain CDR1 (LCDR1), a light chain CDR2 (LCDR2), and a light chain CDR3 (LCDR3), wherein

(1) the light chain CDR1 comprises the light chain CDR1 amino acid sequence in SEQ ID NO: 16, the light chain CDR2 comprises the light chain CDR2 amino acid sequence in SEQ ID NO: 16, and the light chain CDR3 comprises the light chain CDR3 amino acid sequence in SEQ ID NO: 16;

(2) the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;

(3) the light chain CDR1 comprises the light chain CDR1 amino acid sequence in SEQ ID NO: 18, the light chain CDR2 comprises the light chain CDR2 amino acid sequence in SEQ ID NO: 18, and the light chain CDR3 comprises the light chain CDR3 amino acid sequence in SEQ ID NO: 18;

(4) the light chain CDR1 comprises the light chain CDR1 amino acid sequence in SEQ ID NO: 34, the light chain CDR2 comprises the light chain CDR2 amino acid sequence in SEQ ID NO: 34, and the light chain CDR3 comprises the light chain CDR3 amino acid sequence in SEQ ID NO: 34;

(5) the light chain CDR1 comprises the light chain CDR1 amino acid sequence in SEQ ID NO: 35, the light chain CDR2 comprises the light chain CDR2 amino acid sequence in SEQ ID NO: 35, and the light chain CDR3 comprises the light chain CDR3 amino acid sequence in SEQ ID NO: 35;

(6) the light chain CDR1 comprises the light chain CDR1 amino acid sequence in SEQ ID NO: 36, the light chain CDR2 comprises the light chain CDR2 amino acid sequence in SEQ ID NO: 36, and the light chain CDR3 comprises the light chain CDR3 amino acid sequence in SEQ ID NO: 36;

(7) the light chain CDR1 comprises the light chain CDR1 amino acid sequence in SEQ ID NO: 44, the light chain CDR2 comprises the light chain CDR2 amino acid sequence in SEQ ID NO: 44, and the light chain CDR3 comprises the light chain CDR3 amino acid sequence in SEQ ID NO: 44;

(8) the light chain CDR1 comprises the light chain CDR1 amino acid sequence in SEQ ID NO: 52, the light chain CDR2 comprises the light chain CDR2 amino acid sequence in SEQ ID NO: 52, and the light chain CDR3 comprises the light chain CDR3 amino acid sequence in SEQ ID NO: 52; or

(9) the light chain CDR1 comprises the light chain CDR1 amino acid sequence in SEQ ID NO: 60, the light chain CDR2 comprises the light chain CDR2 amino acid sequence in SEQ ID NO: 60, and the light chain CDR3 comprises the light chain CDR3 amino acid sequence in SEQ ID NO: 60.

In some embodiments, the anti-CLDN18.2 antibody or the antigen-binding fragment thereof provided herein comprises a light chain CDR1, a light chain CDR2, and a light chain CDR3, wherein

(1) the light chain CDR1, the light chain CDR2, and the light chain CDR3 comprise the amino acid sequences set forth in SEQ ID NOs: 8, 9, and 10, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 8, 9, and 10, respectively;

(2) the light chain CDR1, the light chain CDR2, and the light chain CDR3 comprise the amino acid sequences set forth in SEQ ID NOs: 25, 26, and 27, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 25, 26, and 27, respectively;

(3) the light chain CDR1, the light chain CDR2, and the light chain CDR3 comprise the amino acid sequences set forth in SEQ ID NOs: 25, 26, and 28, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 25, 26, and 28, respectively;

(4) the light chain CDR1, the light chain CDR2, and the light chain CDR3 comprise the amino acid sequences set forth in SEQ ID NOs: 40, 41, and 42, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 40, 41, and 42, respectively;

(5) the light chain CDR1, the light chain CDR2, and the light chain CDR3 comprise the amino acid sequences set forth in SEQ ID NOs: 48, 49, and 50, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 48, 49, and 50, respectively; or

(6) the light chain CDR1, the light chain CDR2, and the light chain CDR3 comprise the amino acid sequences set forth in SEQ ID NOs: 56, 57, and 58, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 56, 57, and 58, respectively.

In some embodiments, the anti-CLDN18.2 antibody or the antigen-binding fragment thereof provided herein comprises a heavy chain CDR1, a heavy chain CDR2, a heavy chain CDR3, a light chain CDR1, a light chain CDR2, and a light chain CDR3, wherein

(1) the heavy chain CDR1 comprises the heavy chain CDR1 amino acid sequence in SEQ ID NO: 11, the heavy chain CDR2 comprises the heavy chain CDR2 amino acid sequence in SEQ ID NO: 11, the heavy chain CDR3 comprises the heavy chain CDR3 amino acid sequence in SEQ ID NO: 11, the light chain CDR1 comprises the light chain CDR1 amino acid sequence in SEQ ID NO: 16, the light chain CDR2 comprises the light chain CDR2 amino acid sequence in SEQ ID NO: 16, and the light chain CDR3 comprises the light chain CDR3 amino acid sequence in SEQ ID NO: 16;

(2) the heavy chain CDR1 comprises the heavy chain CDR1 amino acid sequence in SEQ ID NO: 12, the heavy chain CDR2 comprises the heavy chain CDR2 amino acid sequence in SEQ ID NO: 12, the heavy chain CDR3 comprises the heavy chain CDR3 amino acid sequence in SEQ ID NO: 12, the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;

(3) the heavy chain CDR1 comprises the heavy chain CDR1 amino acid sequence in SEQ ID NO: 13, the heavy chain CDR2 comprises the heavy chain CDR2 amino acid sequence in SEQ ID NO: 13, the heavy chain CDR3 comprises the heavy chain CDR3 amino acid sequence in SEQ ID NO: 13, the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;

(4) the heavy chain CDR1 comprises the heavy chain CDR1 amino acid sequence in SEQ ID NO: 14, the heavy chain CDR2 comprises the heavy chain CDR2 amino acid sequence in SEQ ID NO: 14, the heavy chain CDR3 comprises the heavy chain CDR3 amino acid sequence in SEQ ID NO: 14, the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;

(5) the heavy chain CDR1 comprises the heavy chain CDR1 amino acid sequence in SEQ ID NO: 15, the heavy chain CDR2 comprises the heavy chain CDR2 amino acid sequence in SEQ ID NO: 15, the heavy chain CDR3 comprises the heavy chain CDR3 amino acid sequence in SEQ ID NO: 15, the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;

(6) the heavy chain CDR1 comprises the heavy chain CDR1 amino acid sequence in SEQ ID NO: 12, the heavy chain CDR2 comprises the heavy chain CDR2 amino acid sequence in SEQ ID NO: 12, the heavy chain CDR3 comprises the heavy chain CDR3 amino acid sequence in SEQ ID NO: 12, the light chain CDR1 comprises the light chain CDR1 amino acid sequence in SEQ ID NO: 18, the light chain CDR2 comprises the light chain CDR2 amino acid sequence in SEQ ID NO: 18, and the light chain CDR3 comprises the light chain CDR3 amino acid sequence in SEQ ID NO: 18;

(7) the heavy chain CDR1 comprises the heavy chain CDR1 amino acid sequence in SEQ ID NO: 13, the heavy chain CDR2 comprises the heavy chain CDR2 amino acid sequence in SEQ ID NO: 13, the heavy chain CDR3 comprises the heavy chain CDR3 amino acid sequence in SEQ ID NO: 13, the light chain CDR1 comprises the light chain CDR1 amino acid sequence in SEQ ID NO: 18, the light chain CDR2 comprises the light chain CDR2 amino acid sequence in SEQ ID NO: 18, and the light chain CDR3 comprises the light chain CDR3 amino acid sequence in SEQ ID NO: 18;

(8) the heavy chain CDR1 comprises the heavy chain CDR1 amino acid sequence in SEQ ID NO: 14, the heavy chain CDR2 comprises the heavy chain CDR2 amino acid sequence in SEQ ID NO: 14, the heavy chain CDR3 comprises the heavy chain CDR3 amino acid sequence in SEQ ID NO: 14, the light chain CDR1 comprises the light chain CDR1 amino acid sequence in SEQ ID NO: 18, the light chain CDR2 comprises the light chain CDR2 amino acid sequence in SEQ ID NO: 18, and the light chain CDR3 comprises the light chain CDR3 amino acid sequence in SEQ ID NO: 18;

(9) the heavy chain CDR1 comprises the heavy chain CDR1 amino acid sequence in SEQ ID NO: 15, the heavy chain CDR2 comprises the heavy chain CDR2 amino acid sequence in SEQ ID NO: 15, the heavy chain CDR3 comprises the heavy chain CDR3 amino acid sequence in SEQ ID NO: 15, the light chain CDR1 comprises the light chain CDR1 amino acid sequence in SEQ ID NO: 18, the light chain CDR2 comprises the light chain CDR2 amino acid sequence in SEQ ID NO: 18, and the light chain CDR3 comprises the light chain CDR3 amino acid sequence in SEQ ID NO: 18;

(10) the heavy chain CDR1 comprises the heavy chain CDR1 amino acid sequence in SEQ ID NO: 29, the heavy chain CDR2 comprises the heavy chain CDR2 amino acid sequence in SEQ ID NO: 29, the heavy chain CDR3 comprises the heavy chain CDR3 amino acid sequence in SEQ ID NO: 29, the light chain CDR1 comprises the light chain CDR1 amino acid sequence in SEQ ID NO: 34, the light chain CDR2 comprises the light chain CDR2 amino acid sequence in SEQ ID NO: 34, and the light chain CDR3 comprises the light chain CDR3 amino acid sequence in SEQ ID NO: 34;

(11) the heavy chain CDR1 comprises the heavy chain CDR1 amino acid sequence in SEQ ID NO: 30, the heavy chain CDR2 comprises the heavy chain CDR2 amino acid sequence in SEQ ID NO: 30, the heavy chain CDR3 comprises the heavy chain CDR3 amino acid sequence in SEQ ID NO: 30, the light chain CDR1 comprises the light chain CDR1 amino acid sequence in SEQ ID NO: 35, the light chain CDR2 comprises the light chain CDR2 amino acid sequence in SEQ ID NO: 35, and the light chain CDR3 comprises the light chain CDR3 amino acid sequence in SEQ ID NO: 35;

(12) the heavy chain CDR1 comprises the heavy chain CDR1 amino acid sequence in SEQ ID NO: 31, the heavy chain CDR2 comprises the heavy chain CDR2 amino acid sequence in SEQ ID NO: 31, the heavy chain CDR3 comprises the heavy chain CDR3 amino acid sequence in SEQ ID NO: 31, the light chain CDR1 comprises the light chain CDR1 amino acid sequence in SEQ ID NO: 35, the light chain CDR2 comprises the light chain CDR2 amino acid sequence in SEQ ID NO: 35, and the light chain CDR3 comprises the light chain CDR3 amino acid sequence in SEQ ID NO: 35;

(13) the heavy chain CDR1 comprises the heavy chain CDR1 amino acid sequence in SEQ ID NO: 32, the heavy chain CDR2 comprises the heavy chain CDR2 amino acid sequence in SEQ ID NO: 32, the heavy chain CDR3 comprises the heavy chain CDR3 amino acid sequence in SEQ ID NO: 32, the light chain CDR1 comprises the light chain CDR1 amino acid sequence in SEQ ID NO: 35, the light chain CDR2 comprises the light chain CDR2 amino acid sequence in SEQ ID NO: 35, and the light chain CDR3 comprises the light chain CDR3 amino acid sequence in SEQ ID NO: 35;

(14) the heavy chain CDR1 comprises the heavy chain CDR1 amino acid sequence in SEQ ID NO: 33, the heavy chain CDR2 comprises the heavy chain CDR2 amino acid sequence in SEQ ID NO: 33, the heavy chain CDR3 comprises the heavy chain CDR3 amino acid sequence in SEQ ID NO: 33, the light chain CDR1 comprises the light chain CDR1 amino acid sequence in SEQ ID NO: 35, the light chain CDR2 comprises the light chain CDR2 amino acid sequence in SEQ ID NO: 35, and the light chain CDR3 comprises the light chain CDR3 amino acid sequence in SEQ ID NO: 35;

(15) the heavy chain CDR1 comprises the heavy chain CDR1 amino acid sequence in SEQ ID NO: 30, the heavy chain CDR2 comprises the heavy chain CDR2 amino acid sequence in SEQ ID NO: 30, the heavy chain CDR3 comprises the heavy chain CDR3 amino acid sequence in SEQ ID NO: 30, the light chain CDR1 comprises the light chain CDR1 amino acid sequence in SEQ ID NO: 36, the light chain CDR2 comprises the light chain CDR2 amino acid sequence in SEQ ID NO: 36, and the light chain CDR3 comprises the light chain CDR3 amino acid sequence in SEQ ID NO: 36;

(16) the heavy chain CDR1 comprises the heavy chain CDR1 amino acid sequence in SEQ ID NO: 31, the heavy chain CDR2 comprises the heavy chain CDR2 amino acid sequence in SEQ ID NO: 31, the heavy chain CDR3 comprises the heavy chain CDR3 amino acid sequence in SEQ ID NO: 31, the light chain CDR1 comprises the light chain CDR1 amino acid sequence in SEQ ID NO: 36, the light chain CDR2 comprises the light chain CDR2 amino acid sequence in SEQ ID NO: 36, and the light chain CDR3 comprises the light chain CDR3 amino acid sequence in SEQ ID NO: 36;

(17) the heavy chain CDR1 comprises the heavy chain CDR1 amino acid sequence in SEQ ID NO: 32, the heavy chain CDR2 comprises the heavy chain CDR2 amino acid sequence in SEQ ID NO: 32, the heavy chain CDR3 comprises the heavy chain CDR3 amino acid sequence in SEQ ID NO: 32, the light chain CDR1 comprises the light chain CDR1 amino acid sequence in SEQ ID NO: 36, the light chain CDR2 comprises the light chain CDR2 amino acid sequence in SEQ ID NO: 36, and the light chain CDR3 comprises the light chain CDR3 amino acid sequence in SEQ ID NO: 36;

(18) the heavy chain CDR1 comprises the heavy chain CDR1 amino acid sequence in SEQ ID NO: 33, the heavy chain CDR2 comprises the heavy chain CDR2 amino acid sequence in SEQ ID NO: 33, the heavy chain CDR3 comprises the heavy chain CDR3 amino acid sequence in SEQ ID NO: 33, the light chain CDR1 comprises the light chain CDR1 amino acid sequence in SEQ ID NO: 36, the light chain CDR2 comprises the light chain CDR2 amino acid sequence in SEQ ID NO: 36, and the light chain CDR3 comprises the light chain CDR3 amino acid sequence in SEQ ID NO: 36;

(19) the heavy chain CDR1 comprises the heavy chain CDR1 amino acid sequence in SEQ ID NO: 43, the heavy chain CDR2 comprises the heavy chain CDR2 amino acid sequence in SEQ ID NO: 43, the heavy chain CDR3 comprises the heavy chain CDR3 amino acid sequence in SEQ ID NO: 43, the light chain CDR1 comprises the light chain CDR1 amino acid sequence in SEQ ID NO: 44, the light chain CDR2 comprises the light chain CDR2 amino acid sequence in SEQ ID NO: 44, and the light chain CDR3 comprises the light chain CDR3 amino acid sequence in SEQ ID NO: 44;

(20) the heavy chain CDR1 comprises the heavy chain CDR1 amino acid sequence in SEQ ID NO: 51, the heavy chain CDR2 comprises the heavy chain CDR2 amino acid sequence in SEQ ID NO: 51, the heavy chain CDR3 comprises the heavy chain CDR3 amino acid sequence in SEQ ID NO: 51, the light chain CDR1 comprises the light chain CDR1 amino acid sequence in SEQ ID NO: 52, the light chain CDR2 comprises the light chain CDR2 amino acid sequence in SEQ ID NO: 52, and the light chain CDR3 comprises the light chain CDR3 amino acid sequence in SEQ ID NO: 52; or

(21) the heavy chain CDR1 comprises the heavy chain CDR1 amino acid sequence in SEQ ID NO: 59, the heavy chain CDR2 comprises the heavy chain CDR2 amino acid sequence in SEQ ID NO: 59, the heavy chain CDR3 comprises the heavy chain CDR3 amino acid sequence in SEQ ID NO: 59, the light chain CDR1 comprises the light chain CDR1 amino acid sequence in SEQ ID NO: 60, the light chain CDR2 comprises the light chain CDR2 amino acid sequence in SEQ ID NO: 60, and the light chain CDR3 comprises the light chain CDR3 amino acid sequence in SEQ ID NO: 60.

In some embodiments, the anti-CLDN18.2 antibody or the antigen-binding fragment thereof provided herein comprises a heavy chain CDR1, a heavy chain CDR2, a heavy chain CDR3, a light chain CDR1, a light chain CDR2, and a light chain CDR3, wherein

(1) the heavy chain CDR1, the heavy chain CDR2, the heavy chain CDR3, the light chain CDR1, the light chain CDR2, and the light chain CDR3 comprise the amino acid sequences set forth in SEQ ID NOs: 3, 4, 7, 8, 9, and 10, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 3, 4, 7, 8, 9, and 10, respectively;

(2) the heavy chain CDR1, the heavy chain CDR2, the heavy chain CDR3, the light chain CDR1, the light chain CDR2, and the light chain CDR3 comprise the amino acid sequences set forth in SEQ ID NOs: 3, 5, 7, 8, 9, and 10, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 3, 5, 7, 8, 9, and 10, respectively;

(3) the heavy chain CDR1, the heavy chain CDR2, the heavy chain CDR3, the light chain CDR1, the light chain CDR2, and the light chain CDR3 comprise the amino acid sequences set forth in SEQ ID NOs: 3, 6, 7, 8, 9, and 10, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 3, 6, 7, 8, 9, and 10, respectively;

(4) the heavy chain CDR1, the heavy chain CDR2, the heavy chain CDR3, the light chain CDR1, the light chain CDR2, and the light chain CDR3 comprise the amino acid sequences set forth in SEQ ID NOs: 19, 20, 24, 25, 26, and 27, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 19, 20, 24, 25, 26, and 27, respectively;

(5) the heavy chain CDR1, the heavy chain CDR2, the heavy chain CDR3, the light chain CDR1, the light chain CDR2, and the light chain CDR3 comprise the amino acid sequences set forth in SEQ ID NOs: 19, 21, 24, 25, 26, and 27, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 19, 21, 24, 25, 26, and 27, respectively;

(6) the heavy chain CDR1, the heavy chain CDR2, the heavy chain CDR3, the light chain CDR1, the light chain CDR2, and the light chain CDR3 comprise the amino acid sequences set forth in SEQ ID NOs: 19, 22, 24, 25, 26, and 27, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 19, 22, 24, 25, 26, and 27, respectively;

(7) the heavy chain CDR1, the heavy chain CDR2, the heavy chain CDR3, the light chain CDR1, the light chain CDR2, and the light chain CDR3 comprise the amino acid sequences set forth in SEQ ID NOs: 19, 23, 24, 25, 26, and 27, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 19, 23, 24, 25, 26, and 27, respectively;

(8) the heavy chain CDR1, the heavy chain CDR2, the heavy chain CDR3, the light chain CDR1, the light chain CDR2, and the light chain CDR3 comprise the amino acid sequences set forth in SEQ ID NOs: 19, 20, 24, 25, 26, and 28, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 19, 20, 24, 25, 26, and 28, respectively;

(9) the heavy chain CDR1, the heavy chain CDR2, the heavy chain CDR3, the light chain CDR1, the light chain CDR2, and the light chain CDR3 comprise the amino acid sequences set forth in SEQ ID NOs: 19, 21, 24, 25, 26, and 28, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 19, 21, 24, 25, 26, and 28, respectively;

(10) the heavy chain CDR1, the heavy chain CDR2, the heavy chain CDR3, the light chain CDR1, the light chain CDR2, and the light chain CDR3 comprise the amino acid sequences set forth in SEQ ID NOs: 19, 22, 24, 25, 26, and 28, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 19, 22, 24, 25, 26, and 28, respectively;

(11) the heavy chain CDR1, the heavy chain CDR2, the heavy chain CDR3, the light chain CDR1, the light chain CDR2, and the light chain CDR3 comprise the amino acid sequences set forth in SEQ ID NOs: 19, 23, 24, 25, 26, and 28, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 19, 23, 24, 25, 26, and 28, respectively;

(12) the heavy chain CDR1, the heavy chain CDR2, the heavy chain CDR3, the light chain CDR1, the light chain CDR2, and the light chain CDR3 comprise the amino acid sequences set forth in SEQ ID NOs: 37, 38, 39, 40, 41, and 42, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 37, 38, 39, 40, 41, and 42, respectively;

(13) the heavy chain CDR1, the heavy chain CDR2, the heavy chain CDR3, the light chain CDR1, the light chain CDR2, and the light chain CDR3 comprise the amino acid sequences set forth in SEQ ID NOs: 45, 46, 47, 48, 49, and 50, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 45, 46, 47, 48, 49, and 50, respectively; or

(14) the heavy chain CDR1, the heavy chain CDR2, the heavy chain CDR3, the light chain CDR1, the light chain CDR2, and the light chain CDR3 comprise the amino acid sequences set forth in SEQ ID NOs: 53, 54, 55, 56, 57, and 58, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 53, 54, 55, 56, 57, and 58, respectively.

In some embodiments, the anti-CLDN18.2 antibody or the antigen-binding fragment thereof provided herein comprises a heavy chain CDR1, a heavy chain CDR2, a heavy chain CDR3, a light chain CDR1, a light chain CDR2, and a light chain CDR3, wherein

the heavy chain CDR1 comprises the amino acid sequence set forth in SEQ ID NO: 3, 19, 37, 45, or 53;
the heavy chain CDR2 comprises the amino acid sequence set forth in SEQ ID NO: 4, 5, 6, 20, 21, 22, 23, 38, 46, or 54;
the heavy chain CDR3 comprises the amino acid sequence set forth in SEQ ID NO: 7, 24, 39, 47, or 55;
the light chain CDR1 comprises the amino acid sequence set forth in SEQ ID NO: 8, 25, 40, 48, or 56;

the light chain CDR2 comprises the amino acid sequence set forth in SEQ ID NO: 9, 26, 41, 49, or 57; and
the light chain CDR3 comprises the amino acid sequence set forth in SEQ ID NO: 10, 27, 28, 42, 50, or 58.

In some embodiments, the anti-CLDN18.2 antibody or the antigen-binding fragment thereof provided herein comprises a heavy chain CDR1, a heavy chain CDR2, a heavy chain CDR3, a light chain CDR1, a light chain CDR2, and a light chain CDR3, wherein

the heavy chain CDR1 comprises the amino acid sequence set forth in SEQ ID NO: 3, 19, or 53;
the heavy chain CDR2 comprises the amino acid sequence set forth in SEQ ID NO: 4, 5, 6, 20, 21, 22, 23, or 54;
the heavy chain CDR3 comprises the amino acid sequence set forth in SEQ ID NO: 7, 24, or 55;
the light chain CDR1 comprises the amino acid sequence set forth in SEQ ID NO: 8, 25, or 56;
the light chain CDR2 comprises the amino acid sequence set forth in SEQ ID NO: 9, 26, or 57; and
the light chain CDR3 comprises the amino acid sequence set forth in SEQ ID NO: 10, 27, 28, or 58.

In some embodiments, the anti-CLDN18.2 antibody or the antigen-binding fragment thereof provided herein comprises a heavy chain variable region, wherein the heavy chain variable region comprises the amino acid sequence set forth in SEQ ID NO: 11, 12, 13, 14, 15, 29, 30, 31, 32, 33, 43, 51, or 59, or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequence set forth in SEQ ID NO: 11, 12, 13, 14, 15, 29, 30, 31, 32, 33, 43, 51, or 59.

In some embodiments, the anti-CLDN18.2 antibody or the antigen-binding fragment thereof provided herein comprises a light chain variable region, wherein the light chain variable region comprises the amino acid sequence set forth in SEQ ID NO: 16, 17, 18, 34, 35, 36, 44, 52, or 60, or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequence set forth in SEQ ID NO: 16, 17, 18, 34, 35, 36, 44, 52, or 60.

In some embodiments, the anti-CLDN18.2 antibody or the antigen-binding fragment thereof provided herein comprises a heavy chain variable region and a light chain variable region, wherein

(1) the heavy chain variable region and the light chain variable region comprise the amino acid sequences set forth in SEQ ID NOs: 11 and 16, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 11 and 16, respectively;
(2) the heavy chain variable region and the light chain variable region comprise the amino acid sequences set forth in SEQ ID NOs: 12 and 17, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 12 and 17, respectively;
(3) the heavy chain variable region and the light chain variable region comprise the amino acid sequences set forth in SEQ ID NOs: 13 and 17, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 13 and 17, respectively;
(4) the heavy chain variable region and the light chain variable region comprise the amino acid sequences set forth in SEQ ID NOs: 14 and 17, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 14 and 17, respectively;
(5) the heavy chain variable region and the light chain variable region comprise the amino acid sequences set forth in SEQ ID NOs: 15 and 17, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 15 and 17, respectively;
(6) the heavy chain variable region and the light chain variable region comprise the amino acid sequences set forth in SEQ ID NOs: 12 and 18, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 12 and 18, respectively;
(7) the heavy chain variable region and the light chain variable region comprise the amino acid sequences set forth in SEQ ID NOs: 13 and 18, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 13 and 18, respectively;
(8) the heavy chain variable region and the light chain variable region comprise the amino acid sequences set forth in SEQ ID NOs: 14 and 18, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 14 and 18, respectively;

(9) the heavy chain variable region and the light chain variable region comprise the amino acid sequences set forth in SEQ ID NOs: 15 and 18, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 15 and 18, respectively;

(10) the heavy chain variable region and the light chain variable region comprise the amino acid sequences set forth in SEQ ID NOs: 29 and 34, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 29 and 34, respectively;

(11) the heavy chain variable region and the light chain variable region comprise the amino acid sequences set forth in SEQ ID NOs: 30 and 35, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 30 and 35, respectively;

(12) the heavy chain variable region and the light chain variable region comprise the amino acid sequences set forth in SEQ ID NOs: 31 and 35, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 31 and 35, respectively;

(13) the heavy chain variable region and the light chain variable region comprise the amino acid sequences set forth in SEQ ID NOs: 32 and 35, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 32 and 35, respectively;

(14) the heavy chain variable region and the light chain variable region comprise the amino acid sequences set forth in SEQ ID NOs: 33 and 35, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 33 and 35, respectively;

(15) the heavy chain variable region and the light chain variable region comprise the amino acid sequences set forth in SEQ ID NOs: 30 and 36, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 30 and 36, respectively;

(16) the heavy chain variable region and the light chain variable region comprise the amino acid sequences set forth in SEQ ID NOs: 31 and 36, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 31 and 36, respectively;

(17) the heavy chain variable region and the light chain variable region comprise the amino acid sequences set forth in SEQ ID NOs: 32 and 36, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 32 and 36, respectively;

(18) the heavy chain variable region and the light chain variable region comprise the amino acid sequences set forth in SEQ ID NOs: 33 and 36, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 33 and 36, respectively;

(19) the heavy chain variable region and the light chain variable region comprise the amino acid sequences set forth in SEQ ID NOs: 43 and 44, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 43 and 44, respectively;

(20) the heavy chain variable region and the light chain variable region comprise the amino acid sequences set forth in SEQ ID NOs: 51 and 52, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 51 and 52, respectively; or

(21) the heavy chain variable region and the light chain variable region comprise the amino acid sequences set forth in SEQ ID NOs: 59 and 60, or amino acid sequences having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequences set forth in SEQ ID NOs: 59 and 60, respectively.

In some embodiments, the anti-CLDN18.2 antibody or the antigen-binding fragment thereof provided herein comprises a heavy chain variable region and a light chain variable region, wherein the heavy chain variable region comprises the amino acid sequence set forth in SEQ ID NO: 11, 12, 13, 14, 15, 29, 30, 31, 32, 33, 43, 51, or 59, and the light chain variable region comprises the amino acid sequence set forth in SEQ ID NO: 16, 17, 18, 34, 35, 36, 44, 52, or 60.

In some embodiments, the anti-CLDN18.2 antibody or the antigen-binding fragment thereof provided herein comprises a heavy chain variable region and a light chain variable region, wherein the heavy chain variable region comprises the amino acid sequence set forth in SEQ ID NO: 11, 12, 13, 14, 15, 29, 30, 31, 32, 33, or 59, and the light chain variable region comprises the amino acid sequence set forth in SEQ ID NO: 16, 17, 18, 34, 35, 36, or 60.

In some embodiments, the anti-CLDN18.2 antibody or the antigen-binding fragment thereof provided herein comprises a heavy chain comprising a heavy chain variable region and a heavy chain constant region and a light chain comprising a light chain variable region and a light chain constant region, wherein the heavy chain variable region and the light chain variable region comprise the heavy chain variable region and the light chain variable region described above, respectively, the heavy chain constant region comprises a human IgG1, IgG2, or IgG4 constant region or a variant thereof, preferably a human IgG1 constant region or a variant thereof, and the light chain constant region comprises a human κ constant region or a human λ constant region or a variant thereof, preferably a human κ constant region or a variant thereof. Exemplary variants include IgG1, IgG2, or IgG4 heavy chain constant region variants with site-directed engineering and amino acid substitutions of the heavy chain constant region, such as AAA mutations, DLE mutations (Shields et al., 2002; Lazar et al., 2006), YTE mutations, and LS mutations (Ghetie et al, 1997; Zalevsky et al., 2010) known in the art.

In some specific embodiments, the heavy chain constant region comprises the amino acid sequence set forth in SEQ ID NO: 61 or an amino acid sequence comprising 1, 2, 3, 4, or 5 amino acid substitutions, deletions, or additions compared with the amino acid sequence set forth in SEQ ID NO: 61, and the light chain constant region comprises the amino acid sequence set forth in SEQ ID NO: 62 or an amino acid sequence comprising 1, 2, 3, 4, or 5 amino acid substitutions, deletions, or additions compared with the amino acid sequence set forth in SEQ ID NO: 62.

In this context, the amino acid substitution may be a conservative amino acid substitution, i.e. a substitution with another amino acid of the same class (having similar chemical properties or functions). By way of example, amino acids can be classified as follows according to their side chain properties: (1) non-polar amino acids: Ala (A), Val (V), Leu (L), Ile (I), Pro (P), Phe (F), Trp (W), and Met (M); (2) uncharged polar amino acids: Gly (G), Ser (S), Thr (T), Cys (C), Tyr (Y), Asn (N), and Gln (Q); (3) acidic amino acids: Asp (D) and Glu (E); (4) basic amino acids: Lys (K), Arg (R), and His (H). Alternatively, amino acids can be classified as follows based on common side chain properties: (1) hydrophobic amino acids: Met, Ala, Val, Leu, and Ile; (2) neutral hydrophilic amino acids: Cys, Ser, Thr, Asn, and Gln; (3) acidic amino acids: Asp and Glu; (4) basic amino acids: His, Lys, and Arg; (5) amino acids that influence chain orientation: Gly and Pro; (6) aromatic amino acids: Trp, Tyr, and Phe. Herein, the various variants of the anti-CLDN18.2 antibody or the antigen-binding fragment thereof described above retain the ability to specifically bind to the antigen CLDN18.2.

In some embodiments, the anti-CLDN18.2 antibody or the antigen-binding fragment thereof provided herein comprises or consists of two heavy (H) chains and two light (L) chains that are linked by disulfide bonds, wherein each of the heavy chains comprises the heavy chain variable region (VH) and the heavy chain constant region described above, wherein the heavy chain variable region (VH) comprises framework regions (FRs) and the heavy chain complementarity determining regions (HCDRs) described above; each of the light chains comprises the light chain variable region (VL) and the light chain constant region described above, wherein the light chain variable region (VL) comprises FRs and the light chain complementarity determining regions (LCDRs) described above; the C-terminus of the heavy chain variable region is linked to the N-terminus of the heavy chain constant region, and the C-terminus of the light chain variable region is linked to the N-terminus of the light chain constant region.

In some embodiments, the antibody of the present disclosure may be a full-length antibody, e.g., of the IgG1 isotype. In another embodiment, the antibody of the present disclosure may be a single-chain antibody (scFv), a nanobody, or an antibody fragment (e.g., a Fab, a F(ab')$_2$ fragment, an Fd fragment, an Fv fragment, a dAb, or an isolated CDR).

The antibody or the antigen-binding fragment thereof provided herein binds to CLDN18.2, thereby inducing antibody-dependent cellular cytotoxicity (ADCC) and/or complement-dependent cytotoxicity (CDC) against cells expressing CLDN18.2. In some embodiments, the antibody or the antigen-binding fragment thereof provided herein binds to human CLDN18.2, thereby inducing ADCC and/or CDC against cells expressing human CLDN18.2.

In some embodiments, the antibody or the antigen-binding fragment thereof provided herein substantially does not bind to CLDN18.1. In some embodiments, the antibody or the antigen-binding fragment thereof provided herein substantially does not bind to human CLDN18.1. The term "substantially does not bind to" a protein or cell means not binding to the protein or cell or not specifically binding to the protein or cell.

In some embodiments, the antibody or the antigen-binding fragment thereof provided herein does not induce ADCC and/or CDC against cells expressing CLDN18.1. In some embodiments, the antibody or the antigen-binding fragment thereof provided herein does not induce ADCC and/or CDC against cells expressing human CLDN18.1.

In one aspect, the present disclosure provides an isolated antibody or an antigen-binding fragment thereof that binds to CLDN18.2, wherein the antibody or the antigen-binding fragment thereof is produced by a hybridoma selected from the group consisting of the hybridomas designated herein as 28G3, 40F6, 22F12, 34G6, and 10D8. Accordingly, the present disclosure further encompasses antibodies or antigen-binding fragments thereof produced by hybridomas 28G3, 40F6, 22F12, 34G6, and 10D8, and any hybridoma that produces the antibody disclosed herein.

In one aspect, the present disclosure provides an antibody or an antigen-binding fragment thereof that binds to the same epitope on human CLDN18.2 as any exemplary anti-CLDN18.2 antibody or antigen-binding fragment thereof of the present disclosure. In some embodiments, the present disclosure provides an antibody or an antigen-binding fragment thereof that competes for binding to human CLDN18.2 with any exemplary anti-CLDN18.2 antibody or antigen-binding fragment thereof of the present disclosure.

In another aspect, the present disclosure further provides a multispecific antibody comprising one or more anti-CLDN18.2 antibodies or antigen-binding fragments thereof of the present disclosure and at least one antibody or an antigen-binding fragment thereof having a different specificity than the antibody or the antigen-binding fragment of the present disclosure, wherein the multispecific antibody is capable of binding to at least two different binding sites or targets. As used herein, the "multispecific antibody" encompasses an antibody with the specificity against two targets (i.e., a bispecific antibody), three targets (i.e., a trispecific antibody), four targets (i.e., a tetraspecific antibody), or more targets. These multispecific antibodies can be prepared by methods well known in the art.

In another aspect, the present disclosure further provides a recombinant polypeptide or fusion protein comprising one or more anti-CLDN18.2 antibodies or antigen-binding fragments thereof of the present disclosure, wherein the anti-CLDN18.2 antibody or the antigen-binding fragment thereof of the present disclosure is linked to at least one other functional fragment including, but not limited to, another peptide, protein, cytokine, or receptor ligand. These recombinant polypeptides and fusion proteins can be prepared by methods well known in the art.

In another aspect, the present disclosure further provides an immunoconjugate comprising one or more anti-CLDN18.2 antibodies or antigen-binding fragments thereof of the present disclosure, wherein the anti-CLDN18.2 antibody or the antigen-binding fragment thereof of the present disclosure is linked to at least one other therapeutic agent (e.g., a cytotoxic agent, a radioisotope, an immunoadhesion molecule, or an imaging agent), such as an antibody-drug conjugate (ADC). These immunoconjugates can be prepared by methods well known in the art.

In some embodiments, the linkage is a covalent linkage. In some embodiments, the linkage is a non-covalent linkage. In some embodiments, the linkage is a direct linkage. In some embodiments, the linkage is via a linker. A suitable linker includes, but is not limited to, an amino acid and a polypeptide linker.

In another aspect, the anti-CLDN18.2 antibody or the antigen-binding fragment thereof of the present disclosure may be part of a chimeric antigen receptor (CAR). The present disclosure further provides an immune cell comprising the chimeric antigen receptor, e.g., a T cell (i.e., CAR-T cell).

In addition, the present disclosure further provides a gene vector comprising a gene encoding the anti-CLDN18.2 antibody or the antigen-binding fragment thereof of the present disclosure and allowing the entry of the gene into a mammalian cell (preferably a human cell) and the expression therein. Such gene vectors include, but are not limited to, naked plasmid vectors, yeast vectors, adenoviral vectors, adeno-associated viral vectors, retroviral vectors, lentiviral vectors, poxvirus vectors, rhabdovirus vectors, or baculovirus vectors.

In one aspect, the present disclosure further provides an isolated nucleic acid molecule encoding the anti-CLDN18.2 antibody or the antigen-binding fragment thereof of the present disclosure. The present disclosure further provides an expression vector comprising the nucleic acid molecule and a host cell comprising the nucleic acid molecule or the expression vector.

In one aspect, the present disclosure further provides a method for preparing the anti-CLDN18.2 antibody or the antigen-binding fragment thereof, which comprises (i) expressing the anti-CLDN18.2 antibody or the antigen-binding fragment thereof in a host cell and (ii) isolating the anti-CLDN18.2 antibody or the antigen-binding fragment thereof from the host cell or cell culture thereof.

In one aspect, the present disclosure provides a pharmaceutical composition comprising the anti-CLDN18.2 antibody or the antigen-binding fragment thereof of the present disclosure and one or more pharmaceutically acceptable carriers or excipients. In another embodiment, the present disclosure further provides a pharmaceutical composition comprising the recombinant polypeptide, the fusion protein, the multispecific antibody, the immunoconjugate, the chimeric antigen receptor, the nucleic acid molecule, or the genetic vector of the present disclosure, and a pharmaceutically acceptable carrier or excipient.

In another aspect, the present disclosure provides a method for preventing, alleviating, or treating a CLDN18.2-positive disease in a subject, which comprises administering to the subject a therapeutically effective amount of the anti-CLDN18.2 antibody or the antigen-binding fragment thereof or the pharmaceutical composition thereof of the present disclosure. In another aspect, the present disclosure provides use of the anti-CLDN18.2 antibody or the antigen-binding fragment thereof or the pharmaceutical composition thereof of the present disclosure in the preparation of a medicament for preventing, alleviating, or treating a CLDN18.2-positive disease. Alternatively, the present disclosure provides an anti-CLDN18.2 antibody or an antigen-binding fragment thereof for use in preventing, alleviating, or treating a CLDN18.2-positive disease. In some embodiments, the CLDN18.2-positive disease is cancer. In certain embodiments, the subject is a human.

Thus, in some embodiments, the present disclosure provides a method for preventing, alleviating, or treating cancer in a subject, which comprises administering to the subject a therapeutically effective amount of the anti-CLDN18.2 antibody or the antigen-binding fragment thereof or the pharmaceutical composition thereof of the present disclosure. In certain embodiments, the subject is a human.

As used herein, "cancer" refers to a wide variety of diseases characterized by the uncontrolled growth of abnormal cells in the body. "Cancer" or "cancer tissue" may include a tumor. Cancers that can be treated by the method of the present disclosure include, but are not limited to, carcinomas, sarcomas, lymphomas, blastomas, and leukemias. The term "cancer" includes, but is not limited to, a primary cancer originating at a specific site in the body, a metastatic carcinoma that has spread from its initial location to other sites in the body, a recurrent cancer of a primary cancer after remission, and a second primary cancer (a new primary cancer in a patient with a history of a cancer of different type).

In some embodiments, the cancer includes a hematologic tumor and a solid tumor. The hematological tumors include, e.g., leukemia, lymphoma, and myeloma. In some embodiments, the cancer is a CLDN18.2-positive cancer. In some embodiments, the cancer includes, but is not limited to, gastric cancer, esophageal cancer, gastrointestinal cancer (including colon cancer and rectal cancer), pancreatic cancer, liver cancer, lung cancer (including small cell lung cancer and non-small cell lung cancer), bronchial cancer, mesothelioma, kidney cancer, ovarian cancer (including Kunkenberg tumor), breast cancer, bladder cancer, uterus cancer (including endometrial cancer), prostate cancer, testicular cancer, anal cancer, vaginal cancer, bile duct cancer, gall bladder cancer, head and neck cancer, spinal tumor, otorhinolaryngological tumor, thyroid cancer, mesothelioma, bone cancer, skin cancer, melanoma, adenocarcinoma, sarcoma, neuroblastoma, glioblastoma, brain cancer, central nervous system cancer, neuroendocrine tumor, leukemia, lymphoma, and myeloma. The cancer may be in situ, metastatic, recurrent, and/or refractory.

The anti-CLDN18.2 antibody or the antigen-binding fragment thereof of the present disclosure may be administered alone or in combination with one or more other therapeutic agents. When administered in combination with other therapeutic agents, the other therapeutic agent may be administered before or after, or simultaneously with, the administration of the anti-CLDN18.2 antibody or the antigen-binding fragment thereof of the present disclosure.

In some embodiments, the other therapeutic agent is an antibody or an antigen-binding fragment thereof having a different specificity than the antibody or the antigen-binding fragment of the present disclosure, e.g., an anti-PD-1 antibody, an anti-PD-L1 antibody, a CTLA-4 antibody, or an antigen-binding fragment thereof. In some embodiments, the other therapeutic agent is a cytokine, such as interleukin 2. In some embodiments, the other therapeutic agent is an agent that stimulates $\gamma\delta T$ cells, which may be V$\gamma$9V$\delta$2 T cells, and the agent that stimulates $\gamma\delta T$ cells includes, but is not limited to, bisphosphonic acid, or salts or esters thereof, such as zoledronic acid, clodronic acid, ibandronic acid, pamidronic acid, risedronic acid, minodronic acid, olpadronic acid, alendronic acid, incadronic acid, and salts thereof. In some embodiments, the other therapeutic agent is a chemotherapeutic agent including, but not limited to, alkylating agents, podophyllums, camptothecin analogs, taxanes, antimetabolites, and antibiotics, such as platinum agents (cisplatin, carboplatin, or oxaliplatin), anthracyclines (e.g., adriamycin or epirubicin), taxanes (e.g., paclitaxel), fluoropyrimidine derivatives (e.g., 5-fluorouracil or gemcitabine), and prodrugs thereof. In some embodiments, the other therapeutic agent is an agent stabilizing or increasing the expression of CLDN18.2, such as epirubicin, oxaliplatin, 5-fluorouracil, leucovorin, irinotecan, docetaxel, cisplatin, gemcitabine, and prodrugs thereof.

The "treat" or "treatment" refers to a method for alleviating the progression or severity of a symptom, disorder, condition, or disease. The anti-CLDN18.2 antibody or the antigen-binding fragment thereof, the nucleic acid molecule, the pharmaceutical composition, the recombinant polypeptide, the fusion protein, the multispecific antibody, the immunoconjugate, the chimeric antigen receptor, or the genetic vector of the present disclosure may be administered to a subject to treat cancer in the subject. In some embodiments, the subject is a human.

Other features and advantages of the present disclosure will become more apparent with reference to the following detailed description and examples, which shall not be construed as limiting. The content of all of the documents, Genbank records, patents, and published patent disclosures cited in the present disclosure is expressly incorporated herein by reference.

## BRIEF DESCRIPTION OF THE DRAWINGS

FIG. 1 shows the binding activity of the chimeric antibodies xi40F6, xi34G6, xi10D8, xi22F12, and xi28G3 for U2OS-CLDN18.2 cells (A-C), NUGC-4 cells (D-F), and KATOIII cells (G-I).

FIG. 2 shows the ADCC activity of chimeric antibodies xi22F12, xi28G3, xi40F6, and xi34G6, wherein Jurkat-CD16a v158-NFAT-luciferase is the effector cell and KATOIII (A), NUGC-4 (B), and U2OS-CLDN18.2 (C) are target cells.

FIG. 3 shows the ADCC activity of chimeric antibodies xi22F12, xi28G3, xi40F6, and xi34G6, wherein PBMC is the effector cell and KATOIII (A) and NUGC-4 (B) are target cells.

FIG. 4 shows the CDC activity of chimeric antibodies xi22F12, xi28G3, xi40F6, xi34G6, and xi10D8, wherein KATOIII (A) and U2OS-CLDN18.2 (B) are target cells.

FIG. 5 shows the internalization activity of chimeric antibodies xi28G3 and xi40F6, wherein NUGC-4 (A) and NIH-3T3-CLDN18.2 (B) are target cells.

FIG. 6 shows the non-specific ADCC activity of chimeric antibodies xi22F12, xi28G3, xi40F6, xi34G6, and xi10D8, wherein Jurkat-CD16a v158-NFAT-luciferase is the effector cell and NIH-3T3-CLDN18.1 is the target cell.

FIG. 7 shows the binding activity of chimeric antibody xi28G3 and humanized antibodies hz28G3-1.1, hz28G3-1.2, hz28G3-1.3, hz28G3-1.4, hz28G3-2.1, hz28G3-2.2, hz28G3-2.3, and hz28G3-2.4 for the NUGC-4 cell (A) and KATOIII cell (B).

FIG. 8 shows the binding activity of chimeric antibody xi40F6 and humanized antibodies hz40F6-1.1, hz40F6-1.2, hz40F6-1.3, hz40F6-1.4, hz40F6-2.1, hz40F6-2.2, hz40F6-2.3, and hz40F6-2.4 for the NUGC-4 cell (A) and KATOIII cell (B).

FIG. 9 shows the ADCC activity of chimeric antibody xi28G3 and humanized antibodies hz28G3-1.1, hz28G3-1.2, hz28G3-1.3, hz28G3-1.4, hz28G3-2.1, hz28G3-2.2, hz28G3-2.3, and hz28G3-2.4, wherein Jurkat-CD16a v158-NFAT-luciferase is the effector cell and U2OS-CLDN18.2 (A) and KATOIII (B) are target cells.

FIG. 10 shows the ADCC activity of chimeric antibody xi40F6 and humanized antibodies hz40F6-1.1, hz40F6-1.2, hz40F6-1.3, hz40F6-1.4, hz40F6-2.1, hz40F6-2.2, hz40F6-2.3, and hz40F6-2.4, wherein Jurkat-CD16a v158-NFAT-luciferase is the effector cell and NIH-3T3-CLDN18.2 (A) and NUGC-4 (B) are target cells.

FIG. 11 shows the ADCC activity of chimeric antibody xi28G3 and humanized antibodies hz28G3-1.1, hz28G3-1.3, and hz40F6-1.4, wherein PBMC is the effector cell and NIH-3T3-CLDN18.2 (A), KATOIII (B), and NUGC-4 (C) are target cells.

FIG. 12 shows the CDC activity of chimeric antibody xi28G3 and humanized antibodies hz28G3-1.1, hz28G3-1.3, hz40F6-1.1, hz40F6-1.2, hz40F6-1.3, hz40F6-1.4, hz40F6-2.1, hz40F6-2.2, hz40F6-2.3, and hz40F6-2.4, wherein NIH-3T3-CLDN18.2 is the target cell.

FIG. 13 shows the internalization activity of chimeric antibody xi28G3 and humanized antibodies hz28G3-1.1 and hz28G3-1.3, wherein U2OS-CLDN18.2 (A) and NUGC-4 (B) are target cells.

FIG. 14 shows the non-specific ADCC activity of chimeric antibody xi28G3 and humanized antibodies hz28G3-1.1 and hz28G3-1.3, wherein PBMC is the effector cell and NIH-3T3-CLDN18.1 is the target cell.

## DETAILED DESCRIPTION

It should be appreciated that the terms used herein are for the purpose of describing specific embodiments only rather than limiting. Unless otherwise defined, all of the technical and scientific terms used herein have the same meaning as commonly understood by those of ordinary skills in the art to which the present disclosure belongs.

The term "antibody" refers to a binding protein having at least one antigen (e.g., CLDN18.2) binding domain. The antibody or the antigen-binding fragment thereof of the present disclosure may be an intact antibody or any fragment thereof, including a monoclonal antibody or a fragment thereof, and an antibody variant or a fragment thereof. Examples of antibodies or antigen-binding fragments thereof include a monoclonal antibody, a monospecific antibody, a multispecific antibody, a Fab fragment, a F(ab')2 fragment, an Fd fragment, an Fv fragment, a dAb, an isolated CDR region, a single-chain Fv (scFv), a nanobody, and any antibody fragment known in the art. The anti-CLDN18.2 antibody or the antigen-binding fragment thereof disclosed herein may be of the IgG1, IgG2, IgG3, or IgG4 isotype. The term "isotype" refers to the class of antibodies encoded by the heavy chain constant region gene. In some embodiments, the anti-CLDN18.2 antibody or the antigen-binding fragment thereof disclosed herein is of the IgG1 isotype. The anti-CLDN18.2 antibody or the antigen-binding fragment thereof disclosed herein may be derived from any species, including but not limited to mouse, rat, rabbit, non-human primate (e.g., chimpanzee, cynomolgus monkey, spider monkey, or macaque), llama, and human. The anti-CLDN18.2 antibody or the antigen-binding fragment thereof of the present disclosure may be a murine antibody, a chimeric antibody, a humanized antibody, or a human antibody. Unless otherwise stated, the "antibody" in the present disclosure includes a full-length antibody and any antigen-binding portion (or "antigen-binding fragment") or single chain thereof.

A typical "full-length antibody" is a glycoprotein comprising two heavy (H) chains and two light (L) chains, wherein the heavy and light chains are linked by disulfide bonds, each heavy chain consists of a heavy chain variable region (VH) and a heavy chain constant region consisting of three domains, i.e., CH1, CH2, and CH3, and each light chain consists of a light chain variable region (VL) and a light chain constant region consisting of one domain CL, and the VH and VL regions can also be divided into hypervariable regions (i.e., complementarity determining regions (CDRs)) and framework regions (FRs) with relatively conserved sequences. Each VH and VL consists of three CDRs and four FRs, arranged from the amino-terminus to the carboxyl-terminus in the following order: FR1, CDR1, FR2, CDR2, FR3, CDR3, FR4. The variable regions of the antibody comprise binding domains that interact with antigens. The constant regions of the antibody can mediate the binding of immunoglobulins to host tissues or factors, including various cells of the immune system (e.g., effector cells) and the first component (C1q) of the classical complement system.

The "antigen-binding fragment" or "antigen-binding portion" of an antibody refers to one or more fragments of the antibody that retain the functionality of specifically binding to an antigen (e.g., CLDN18.2 protein). It has been demonstrated that the antigen binding functionality of an antibody can be implemented by fragments of a full-length antibody. Examples encompassed within the term "antigen-binding portion/fragment" of an antibody include: (i) a Fab fragment: a monovalent fragment consisting of VL, VH, CL, and CH1 domains; (ii) a F(ab')2 fragment, a bivalent fragment comprising two Fab fragments linked by a disulfide bridge at the hinge region; (iii) an Fd fragment consisting of VH and CH1 domains; (iv) an Fv fragment consisting of VL and VH domains of a single arm of the antibody; (v) a dAb fragment consisting of VH domains (see Ward et al., Nature. 341:544-546 (1989)); (vi) an isolated complementarity determining region (CDR); and (vii) a nanobody, an antibody comprising a single heavy chain variable domain and two constant domains and being free of light chains. Furthermore, although the two domains VL and VH of the Fv fragment are encoded by different genes, the VL and VH domains can be joined via a linker by recombinant means into a single protein chain in which the VL and VH pair to form a monovalent molecule referred to as a single-chain Fv (scFv); see, Bird et al., Science. 242:423-426 (1988); Huston et al., Proc. Natl. Acad. Sci. 85:5879-5883 (1988). Such single-chain antibodies are also encompassed within the term "antigen-binding portion/fragment". Furthermore, a recombinant polypeptide, a fusion protein, and an immunoconjugate comprising the antigen-binding portion/fragment are also encompassed within the term "antigen-binding portion/fragment". These antibody fragments can be obtained using conventional techniques known to those skilled in the art, and the fragments can be subjected to functional screening using the same method as full-length antibodies.

The "mouse antibody" or "murine antibody" refers to an antibody in which the framework regions and CDRs in the variable region are derived from mouse germline immunoglobulin sequences. Furthermore, if the antibody comprises a constant region, the constant region is also derived from mouse germline immunoglobulin sequences. The murine antibody of the present disclosure may comprise amino acid residues not encoded by mouse germline immunoglobulin sequences (e.g., mutations introduced by *in vitro* random mutation or point mutation or by *in vivo* somatic mutation), but "mouse antibody" or "murine antibody" does not include antibodies in which CDR sequences derived from other mammalian species are inserted into mouse framework sequences.

The "chimeric antibody" is an antibody obtained by fusing a variable region of a murine antibody with a constant region of a human antibody and can reduce the immune response induced by the murine antibody. The establishment of a chimeric antibody comprises establishing a hybridoma secreting a murine specific monoclonal antibody first, cloning a variable region gene from the hybridoma cell, then cloning a constant region gene of a human antibody as required, linking the mouse variable region gene and the human constant region gene into a chimeric gene and then inserting the chimeric gene into an expression vector, and finally expressing a chimeric antibody in a eukaryotic system or a prokaryotic system. In the present disclosure, a chimeric antibody is also denoted as "xi".

The "humanized antibody" refers to an antibody comprising complementarity determining regions (CDRs) derived from a non-human antibody and framework and constant regions derived from a human antibody. For example, the humanized antibody that binds to CLDN18.2 provided herein may comprise CDRs derived from one or more murine antibodies as well as human framework and constant regions. Thus, in some embodiments, the humanized antibody provided herein binds to the same epitope on CLDN18.2 as the murine antibody from which the CDRs of the humanized antibody are derived. Exemplary humanized antibodies are provided herein. Additional humanized antibodies that bind to CLDN18.2 or variants thereof comprising the heavy and light chain CDRs provided herein may be generated using any human framework sequences and are also included in the present disclosure. In some embodiments, framework sequences suitable for use in the present disclosure include those similar in structure to the framework sequences provided herein. Additional modifications may be made in the framework regions to alter the properties of the antibodies provided herein. Such additional framework region modifications may include chemical modifications, point mutations for reducing immunogenicity or removing T cell epitopes, or back mutations to residues in original germline sequences. In some embodiments, such additional modifications include those corresponding to the mutations exemplified herein, including back mutations to germline sequences. For example, in some embodiments, one or more amino acids in the human VH and/or VL framework regions of the humanized antibodies provided herein are back mutated to the corresponding amino acids in the parent murine antibodies. In the present disclosure, humanized antibodies are also denoted as "hz".

"Derived" as described herein, when used to refer to a molecule or polypeptide relative to a reference antibody or other binding proteins, means that a molecule or polypeptide can specifically bind to the same epitope as the reference antibody or other binding proteins.

The "isolated" means that a target compound (e.g., an antibody, an antigen-binding fragment, or a nucleic acid molecule) has been isolated from its natural environment.

The "identity" refers to the similarity between two or more nucleic acid sequences or between two or more polypeptide sequences. The sequence identity of the present disclosure is at least 85%, 90% or 95%, preferably at least 95%. Non-limiting examples include 85%, 86%, 87%, 88%, 89%, 90%, 91%, 92%, 93%, 94%, 95%, 96%, 97%, 98%, 99%, or 100%. Sequence comparison and percent identity determination between two sequences can be performed using the BLASTN/BLASTP algorithm on the National Center For Biotechnology Institute with default settings.

The antibody that "competes for binding" refers to an antibody that partially or completely blocks the binding of other antibodies to a target. Whether two antibodies compete with each other for binding to a target, i.e., whether and to what extent one antibody blocks the binding of the other antibody to the target, may be determined using competition assays known in the art, e.g., solid-phase direct or indirect radioimmunoassay (RIA), solid-phase direct or indirect enzyme immunoassay (EIA), sandwich competition assay, and the like. In certain embodiments, one antibody competes with the other antibody for binding to the target and blocks the binding of the other antibody to the target by at least 10%, 20%, 30%, 40%, 50%, 60%, 70%, 80%, 90%, or 100%.

Two or more antibodies "bind to the same epitope" means that the antibodies bind to the same segment of amino acid residues, as determined by a given method. Techniques for determining whether an antibody binds to "the same epitope on CLDN18.2" as the antibodies described herein include, for example, epitope mapping methods, such as epitope mapping by yeast surface display, X-ray diffraction analysis of the crystals of antigen:antibody complexes, and hydrogen/deuterium exchange mass spectrometry (HDX-MS).

The term "$EC_{50}$", also known as half maximal effective concentration, refers to the concentration of antibody that can cause 50% of the maximum effect after a specified exposure time. The term "$IC_{50}$", also known as half maximal inhibitory concentration, refers to the concentration of an antibody that inhibits a specific biological or biochemical function by 50% relative to the case where the antibody is absent. Both $EC_{50}$ and $IC_{50}$ can be measured by ELISA or FACS assay or any other method known in the art.

The "patient" or "subject" includes any human or non-human animal. The term "non-human animal" includes all vertebrates, e.g., mammals and non-mammals, preferably mammals, e.g., non-human primates, sheep, dogs, cats, cows and horses.

The "effective dose" or "effective amount" refers to an amount sufficient to achieve or at least partially achieve a desired effect. The therapeutically "effective amount" or "effective dose" of a drug or therapeutic agent refers to an amount sufficient to prevent or ameliorate symptoms associated with a disease or disorder, preferably an amount that causes a reduction in the severity of the symptoms of the disease or an increase in the frequency and duration of asymptomatic phases, or prevents damage or inability caused by the disease, when used alone or in combination with another therapeutic agent. The therapeutically effective amount is related to the disease to be treated, wherein the actual effective amount can be readily determined by those skilled in the art.

As used herein, "about" means being within an acceptable error range determined by those of ordinary skill in the art for a particular value, which will depend in part on how the value is measured or determined, i.e., the limitation by the measurement system. For example, "about" may mean being within 1 or more than 1 standard deviation, as practiced in the art. Alternatively, "about" may mean a range of up to $\pm 5\%$, for example, fluctuating within a particular numerical range given $\pm 2\%$, $\pm 1\%$, or $\pm 0.5\%$. Where a particular value is given in the present disclosure or in the claims, unless otherwise stated, "about" should be considered to mean being within an acceptable error range for that particular value. In this context, unless otherwise stated, the values of the parameters or conditions in a step are all modified by "about" by default.

The word "a" or "an" means "at least a" or "at least an", the phrase "at least one" means the same as "one or more", and "and/or" is used to refer to "and" or "or", unless otherwise specified.

As described herein, any percentage range, ratio range, or integer range shall be interpreted as including the value of any integer within the listed range, unless otherwise indicated.

In the present disclosure, unless the context dictates otherwise, the words "comprise", "include", and "contain" will be interpreted as including the steps or elements or a group of steps or elements but not excluding any other steps or elements or groups of steps or elements. "Consist of..." means including and being limited to what is defined by the phrase "consist of...". Thus, the phrase "consist of..." means that the listed elements are required or necessary and that no other elements can be present. "Substantially consist of..." means including any element defined by the phrase and being limited to other elements that do not interfere with or are favorable for the listed elements' activity or effects as detailed in the present disclosure. Thus, the phrase "substantially consist of..." means that the listed elements are required or necessary but other elements are optional and can be present or absent depending on whether they affect the listed elements' activity or effects.

As used herein, unless otherwise stated, the terms "nucleic acid", "nucleic acid sequence", and "gene" are used interchangeably to refer to the product of two or more nucleotides joined by phosphodiester bonds.

Unless otherwise specified clearly herein, singular terms encompass plural referents, and vice versa.

Aspects of the present disclosure are described in more detail below.

The amino acid sequence IDs (SEQ ID NOs.) of the heavy chain variable region, the light chain variable region, and the CDRs of the exemplary antibodies or antigen-binding fragments thereof of the present disclosure are provided in Table 1 below. Some antibodies have identical CDRs, and some antibodies have identical VHs or VLs. The heavy chain constant region of the antibody may be a human IgG1, IgG2, or IgG4 constant region or a variant thereof, preferably a human IgG1 constant region or a variant thereof, for example, comprising the amino acid sequence set forth in SEQ ID NO: 61 or an amino acid sequence comprising 1, 2, 3, 4, or 5 amino acid substitutions, deletions, or additions compared with the amino acid sequence set forth in SEQ ID NO: 61. The light chain constant region of the antibody may be a human κ constant region or a human λ constant region or a variant thereof, preferably a human κ constant region or a variant thereof, for example, comprising the amino acid sequence set forth in SEQ ID NO: 62 or an amino acid sequence comprising 1, 2, 3, 4, or 5 amino acid substitutions, deletions, or additions compared with the amino acid sequence set forth in SEQ ID NO: 62. These antibodies may also comprise a mouse IgG1 heavy chain constant region and/or a mouse κ constant region.

Table 1. Amino acid sequence IDs (SEQ ID NOs.) of variable regions and CDRs

| Antibody | HCDR1 | HCDR2 | HCDR3 | VH | LCDR1 | LCDR2 | LCDR3 | VL |
|---|---|---|---|---|---|---|---|---|
| Mouse and chimeric 28G3 | 3 | 4 | 7 | 11 | 8 | 9 | 10 | 16 |
| hz28G3-1.1 | | 5 | | 12 | | | | 17 |
| hz28G3-1.2 | | 6 | | 13 | | | | |
| hz28G3-1.3 | | 5 | | 14 | | | | |
| hz28G3-1.4 | | 6 | | 15 | | | | |
| hz28G3-2.1 | | 5 | | 12 | | | | 18 |
| hz28G3-2.2 | | 6 | | 13 | | | | |
| hz28G3-2.3 | | 5 | | 14 | | | | |
| hz28G3-2.4 | | 6 | | 15 | | | | |
| Mouse and chimeric 40F6 | 19 | 20 | 24 | 29 | 25 | 26 | 27 | 34 |
| hz40F6-1.1 | | 21 | | 30 | | | | 35 |
| hz40F6-1.2 | | 20 | | 31 | | | | |
| hz40F6-1.3 | | 22 | | 32 | | | | |
| hz40F6-1.4 | | 23 | | 33 | | | | |
| hz40F6-2.1 | | 21 | | 30 | | | 28 | 36 |
| hz40F6-2.2 | | 20 | | 31 | | | | |
| hz40F6-2.3 | | 22 | | 32 | | | | |
| hz40F6-2.4 | | 23 | | 33 | | | | |
| Mouse and chimeric 34G6 | 37 | 38 | 39 | 43 | 40 | 41 | 42 | 44 |
| Mouse and chimeric 10D8 | 45 | 46 | 47 | 51 | 48 | 49 | 50 | 52 |
| Mouse and chimeric 22F12 | 53 | 54 | 55 | 59 | 56 | 57 | 58 | 60 |

The term "complementarity determining region", "CDR", or "hypervariable region" refers to one of the 6 hypervariable regions within variable regions of an antibody that primarily contribute to antigen binding. In view of the amino acid sequences of the antibody variable regions, the amino acid sequence boundaries of the CDRs can be determined by those skilled in the art using any of a variety of well-known schemes, including "Kabat" numbering scheme (see Kabat et al. (1991), Sequences of Proteins of Immunological Interest, 5th edition, Public Health Service, National Institutes of Health, Bethesda, MD), "Chothia" numbering scheme (see Al-Lazikani et al. (1997), JMB 273:927-948), IxnMunoGenTics (IMGT) numbering scheme (Lefranc M.P., Immunologist, 7, 132-136 (1999); Lefranc, M.P. et al., Dev. Comp. Immunol., 27, 55-77 (2003)), AbM numbering scheme (defined by Oxford Molecular's AbM antibody modeling software), Contact numbering scheme (based on analysis of available complex crystal structures), and combinations of two or more of the Kabat, Chothia, IMGT, AbM, and Contact definition schemes. For example, for the classical format, according to the Kabat scheme, the CDR amino acid residues in the heavy chain variable region (VH) are numbered 31-35 (HCDR1), 50-65 (HCDR2), and 95-102 (HCDR3); the CDR amino acid residues in the light chain variable region (VL) are numbered 24-34 (LCDR1), 50-56 (LCDR2), and 89-97 (LCDR3). For example, according to the Chothia scheme, the CDR amino acids in VH are numbered 26-32 (HCDR1), 52-56 (HCDR2), and 95-102 (HCDR3); and the amino acid residues in VL are numbered 26-32 (LCDR1), 50-52 (LCDR2), and 91-96 (LCDR3). According to the combination of the Kabat scheme and the Chothia scheme, the CDR is composed of amino acid residues 26-35 (HCDR1), 50-65 (HCDR2), and 95-102 (HCDR3) in the human VH and amino acid residues 24-34 (LCDR1), 50-56 (LCDR2), and 89-97 (LCDR3) in the human VL. According to the IMGT scheme, the CDR amino acid residues in VH are roughly numbered 26-35 (CDR1), 51-57 (CDR2), and 93-102 (CDR3), and the CDR amino acid residues in VL are roughly numbered 27-32 (CDR1), 50-52 (CDR2), and 89-97 (CDR3). According to the IMGT scheme, the CDRs of the antibody can be determined using the program IMGT/DomainGapAlign.

The amino acid sequence boundaries of the CDRs differ among different numbering schemes. It will be appreciated by those skilled in the art that, unless otherwise specified, the term "CDRs" or "complementarity determining regions" of a given antibody or a region thereof (e.g., a variable region) encompasses complementarity determining regions defined by any of the known schemes. Although the CDR sequences shown in Table 1 and Table 11 are defined according to one of the above numbering schemes, the corresponding amino acid sequences defined according to the other CDR numbering schemes shall also fall within the protection scope of the present disclosure.

The VH and/or VL sequences (or CDR sequences) of other antibodies that bind to human CLDN18.2 may be "mixed and paired" with the VH and/or VL sequences (or CDR sequences) of the anti-CLDN18.2 antibody or the antigen-binding fragment thereof of the present disclosure. Preferably, when VH and VL chains (or CDRs thereof) are mixed and paired, the VH sequence in a particular VH/VL pair can be substituted with a structurally similar VH sequence. Likewise, it is preferred to substitute the VL sequence in a particular VH/VL pair with a structurally similar VL sequence.

Thus, in one embodiment, the antibody or the antigen-binding fragment thereof of the present disclosure comprises:

(a) a heavy chain variable region comprising an amino acid sequence listed in Table 1; and
(b) a light chain variable region comprising an amino acid sequence listed in Table 1, or the VL of another anti-CLDN18.2 antibody, wherein the antibody or the antigen-binding fragment thereof specifically binds to human CLDN18.2.

In another embodiment, the antibody or the antigen-binding fragment thereof of the present disclosure comprises:

(a) the HCDR1, HCDR2, and HCDR3 listed in Table 1; and
(b) the LCDR1, LCDR2, and LCDR3 listed in Table 1, or the light chain variable region CDRs of another anti-CLDN18.2 antibody, wherein the antibody or the antigen-binding fragment thereof specifically binds to human CLDN18.2.

In another embodiment, the antibody or the antigen-binding fragment thereof of the present disclosure comprises the HCDR2 listed in Table 1 and the CDR(s) of other anti-CLDN18.2 antibodies, e.g., HCDR1 and/or HCDR3 of an additional anti-CLDN18.2 antibody and/or LCDR1, LCDR2, and/or LCDR3 of an additional anti-CLDN18.2 antibody.

Furthermore, it is well known in the art that the CDR3 domain is independent of the CDR1 and/or CDR2 domains and can independently determine the antibody's binding specificity for an alloantigen, and that multiple antibodies with the same binding specificity can be predicted based on the CDR3 sequence.

In another embodiment, the antibody or the antigen-binding fragment thereof of the present disclosure comprises the HCDR2 listed in Table 1, the HCDR3 listed in Table 1 and/or the LCDR3 listed in Table 1, as well as the CDR(s) of an additional anti-CLDN18.2 antibody, wherein the antibody or the antigen-binding fragment thereof specifically binds to human CLDN18.2. In another embodiment, the antibody or the antigen-binding fragment thereof of the present disclosure further comprises the LCDR2 listed in Table 1 and the CDR(s) of an additional anti-CLDN18.2 antibody, wherein the antibody or the antigen-binding fragment thereof specifically binds to human CLDN18.2. In another embodiment, the antibody or the antigen-binding fragment thereof of the present disclosure further comprises the HCDR1 listed in Table 1 or the LCDR1 listed in Table 1, and CDR(s) of an additional anti-CLDN18.2 antibody, wherein the antibody or the antigen-binding fragment thereof specifically binds to human CLDN18.2. These antibodies preferably (a) compete for binding to human CLDN18.2 with, (b) retain the same functional properties as, (c) binds to the same epitope as, and/or (d) have similar binding affinities as the anti-CLDN18.2 antibody of the present disclosure.

In another embodiment, the heavy chain variable region and/or the light chain variable region of the antibody or the antigen-binding fragment thereof of the present disclosure may comprise one or more conservative modifications. It is understood in the art that some conservative modifications do not eliminate the antigen binding ability of an antibody.

Therefore, in one embodiment, the antibody or the antigen-binding fragment thereof of the present disclosure comprises a heavy chain variable region and/or a light chain variable region each comprising CDR1, CDR2, and CDR3, wherein:

(a) the HCDR1 sequence comprises the sequence listed in Table 1 and/or conservative modifications thereof; and/or
(b) the HCDR2 sequence comprises the sequence listed in Table 1 and/or conservative modifications thereof; and/or

(c) the HCDR3 sequence comprises the sequence listed in Table 1 and/or conservative modifications thereof; and/or
(d) the LCDR1 and/or the LCDR2 and/or the LCDR3 sequences comprise sequences listed in Table 1 and/or conservative modifications thereof; and
(e) the antibody or the antigen-binding fragment thereof specifically binds to human CLDN18.2.

The term "conservative modification" as used herein refers to an amino acid modification that does not significantly affect or alter the binding properties of the antibody. Such conservative modifications include amino acid substitutions, additions, and deletions. Modifications can be introduced into the antibody of the present disclosure using standard techniques known in the art, e.g., point mutation and PCR-mediated mutation. Conservative amino acid substitution refers to substitution of an amino acid residue with an amino acid residue having similar structural or chemical properties (e.g., similar side chains). Families of amino acid residues having similar side chains are known in the art. Such amino acid residue families include amino acids with basic side chains (e.g., lysine, arginine, and histidine), amino acids with acidic side chains (e.g., aspartic acid and glutamic acid), amino acids with uncharged polar side chains (e.g., glycine, asparagine, glutamine, serine, threonine, tyrosine, cysteine, and tryptophan), amino acids with non-polar side chains (e.g., alanine, valine, leucine, isoleucine, proline, phenylalanine, and methionine), amino acids with $\beta$-branched side chains (e.g., threonine, valine, and isoleucine), and amino acids with aromatic side chains (e.g., tyrosine, phenylalanine, tryptophan, and histidine). Thus, one or more amino acid residues in the CDR regions of the anti-CLDN18.2 antibody or the antigen-binding fragment thereof of the present disclosure can be substituted with other amino acid residues of the same side chain family, and the resulting antibody can be tested for functionality using the functional assays described herein.

The antibody or the antigen-binding fragment thereof of the present disclosure comprises variable region (including CDRs and/or framework regions) modifications, or the antibody or the antigen-binding fragment thereof of the present disclosure may further comprise Fc modifications, for example, to alter the effector functionality of the antibody.

Thus, one embodiment of the present disclosure provides an isolated anti-CLDN18.2 monoclonal antibody or an antigen-binding fragment thereof, which comprises a heavy chain variable region comprising an HCDR1, an HCDR2, and an HCDR3 of the sequences described above in the present disclosure and/or a light chain variable region comprising an LCDR1, an LCDR2, and an LCDR3 of the sequences described above in the present disclosure, but comprising framework sequences different from the sequences described above in the present disclosure. Such framework sequences can be found in public DNA databases or public references including germline antibody gene sequences. Such framework sequences are preferably those that are structurally similar to the framework sequences used for the anti-CLDN18.2 antibody of the present disclosure. For example, in some cases, it may be beneficial to mutate residues in the framework regions; such mutations may maintain or enhance the antigen binding ability of the antibody (see, e.g., U.S. Pat. Nos. 5,530,101; 5,585,089; 5,693,762, and 6,180,370).

Another type of variable region modification is to mutate the amino acid residues within the CDR1, CDR2, and/or CDR3 to improve one or more properties (e.g., affinity and physicochemical properties) of the target antibody. Mutations may be introduced by point mutation or PCR-mediated mutations, and the effect of the mutations on antibody binding or other functional properties may be assessed through *in vitro* or *in vivo* assays known in the art. The conservative modifications may be amino acid substitutions, additions, or deletions, preferably substitutions. Furthermore, typically no more than one, two, three, four, or five residues within each CDR are altered.

In one embodiment, the antibody or the antigen-binding fragment thereof provided herein comprises a heavy chain variable region and a light chain variable region, wherein the heavy chain variable region and the light chain variable region comprise: (a) an HCDR1 comprising the HCDR1 sequence of the present disclosure or an amino acid sequence comprising 1, 2, 3, 4, or 5 amino acid substitutions, deletions, or additions; (b) an HCDR2 comprising the HCDR2 sequence of the present disclosure or an amino acid sequence comprising 1, 2, 3, 4, or 5 amino acid substitutions, deletions, or additions; (c) an HCDR3 comprising the HCDR3 sequence of the present disclosure or an amino acid sequence comprising 1, 2, 3, 4, or 5 amino acid substitutions, deletions, or additions; (d) an LCDR1 comprising the LCDR1 sequence of the present disclosure or an amino acid sequence comprising 1, 2, 3, 4, or 5 amino acid substitutions, deletions, or additions; (e) an LCDR2 comprising the LCDR2 sequence of the present disclosure or an amino acid sequence comprising 1, 2, 3, 4, or 5 amino acid substitutions, deletions or additions; and (f) an LCDR3 comprising the LCDR3 sequence of the present disclosure or an amino acid sequence comprising 1, 2, 3, 4, or 5 amino acid substitutions, deletions or additions.

The antibody or the antigen-binding fragment thereof of the present disclosure comprises framework region modifications in the VH and/or the VL to improve antibody properties. In general, such framework region modifications may reduce the immunogenicity of the antibody. For example, one or more framework residues are "back mutated" to the corresponding germline sequence. These residues can be identified by comparing the antibody framework sequence to the germline sequence of the resulting antibody.

Another type of framework region modification comprises mutating one or more residues of the framework regions or even one or more CDRs to remove T cell epitopes, thereby reducing the immunogenicity that an antibody may produce. This method is also known as "deimmunization" and is described in more detail in U.S. Patent Publication No. 20030153043.

Furthermore, the antibody or the antigen-binding fragment thereof of the present disclosure includes Fc modifications, which may be amino acid insertions, deletions, or substitutions and are typically used to alter one or more functional properties of the antibody, e.g., serum half-life, complement binding, Fc receptor binding, and/or antigen-dependent cellular cytotoxicity.

Furthermore, the antibody or the antigen-binding fragment thereof of the present disclosure may also be chemically modified (e.g., by linking one or more chemical functional groups) or modified to alter its glycosylation, so as to alter one or more functional properties of the antibody. In other embodiments, the Fc region is modified by PEGylation (e.g., by reacting the antibody or the fragment thereof with polyethylene glycol (PEG)). In other embodiments, the glycosylation of the antibody is altered.

The present disclosure further provides an isolated nucleic acid molecule encoding the heavy chain variable region and/or light chain variable region or CDRs of the anti-CLDN18.2 antibody or the antigen-binding fragment thereof of the present disclosure. The nucleic acid molecules of the present disclosure may be DNA or RNA and may or may not comprise intron sequences. The nucleic acid molecule of the present disclosure may be single-stranded or double-stranded. In preferred embodiments, the nucleic acid molecule is a cDNA molecule. The nucleic acid of the present disclosure can be obtained using standard molecular biology techniques. For antibodies expressed by hybridomas, light chain and heavy chain cDNAs encoding the antibodies prepared from the hybridomas can be obtained by standard PCR amplification or using cDNA cloning techniques. For antibodies obtained from immunoglobulin gene libraries (e.g., using phage display technology), nucleic acids encoding such antibodies can be recovered from the gene libraries.

Once the DNA fragments encoding the VH and the VL are obtained, further operations, e.g., converting the variable region genes into full-length antibody chain genes, Fab fragment genes, or scFv genes, can be conducted on these DNA fragments using standard recombinant DNA techniques. In these operations, the DNA fragment encoding the VH or the VL is operably linked to the DNA fragment encoding another protein (e.g., an antibody constant region or a flexible linker). The term "operably linked" as used in this case means that two DNA fragments are joined together such that the amino acid sequences encoded by the two DNA fragments are in the reading frame.

Isolated DNA encoding the VH can be converted into a full-length heavy chain gene by operably linking the DNA encoding the VH to another DNA molecule encoding the heavy chain constant regions (CH1, CH2, and CH3). The sequences of the human heavy chain constant region genes are known in the art, and a DNA fragment comprising the human heavy chain constant region gene can be obtained by standard PCR amplification. The heavy chain constant region may be an IgG1, IgG2, IgG3, IgG4, IgA, IgE, IgM, or IgD constant region, but is preferably an IgG1 constant region.

Isolated DNA encoding the VL can be converted into a full-length light chain gene by operably linking the DNA encoding the VL to another DNA molecule encoding the light chain constant region CL. The sequences of the human light chain constant region genes are known in the art, and a DNA fragment comprising the human light chain constant region gene can be obtained by standard PCR amplification. In preferred embodiments, the light chain constant region may be a κ or λ constant region.

The monoclonal antibodies (mAbs) of the present disclosure can be prepared using the somatic hybridization (hybridoma) technique well known in the art in Kohler and Milstein (1975) Nature 256:495. Other embodiments for preparing the monoclonal antibodies include viral or oncogenic transformation of B lymphocytes and phage display techniques. Chimeric or humanized antibodies are also well known in the art. See, e.g., U.S. Pat. 4,816,567; 5,225,539; 5,530,101; 5,585,089; 5,693,762, and 6,180,370, the content of each of which is expressly incorporated herein by reference.

The antibody of the present disclosure may also be prepared in host cell transfectomas using, for example, recombinant DNA techniques in combination with gene transfection methods (e.g., Morrison, S. (1985) Science 229:1202). In one embodiment, DNA encoding partial or full-length light and heavy chains obtained using standard molecular biotechnology is inserted into one or more expression vectors such that the gene is operably linked to transcriptional and translational regulatory sequences. In this case, the term "operably linked" refers to linking the antibody gene into the vector such that the transcriptional and translational regulatory sequences in the vector perform their intended functions of regulating the transcription and translation of the antibody gene. The term "regulatory sequence" includes promoters, enhancers, and other expression control elements (e.g., polyadenylation signals) that control the transcription or translation of the antibody gene.

The antibody light chain gene and the antibody heavy chain gene can be inserted into the same expression vector or different expression vectors. In preferred embodiments, variable regions are inserted into an expression vector that has encoded the heavy chain constant region and the light chain constant region of the desired isotype to construct a full-length antibody gene, such that the VH is operably linked to the CH in the vector, and the VL is operably linked to the CL in the vector. Furthermore, alternatively, the recombinant expression vector can encode a signal peptide that facilitates the secretion of antibody chains from the host cell. The antibody chain gene can be cloned into a vector such that the signal peptide is linked to the amino terminus of the antibody chain gene in the reading frame. The signal peptide may be an immunoglobulin signal peptide or a heterologous signal peptide (i.e., a signal peptide from a non-immunoglobulin protein).

To express the light chain and the heavy chain, host cells are transfected with expression vectors encoding the heavy chain and the light chain using standard techniques. The term "transfect" encompasses a variety of techniques for introducing exogenous DNA into prokaryotic or eukaryotic host cells, such as electroporation, calcium phosphate precipitation, and DEAE-dextran transfection. Although expressing the antibody of the present disclosure in prokaryotic or eukaryotic host cells is theoretically feasible, expressing the antibody in eukaryotic cells is preferred, and expressing the antibody in mammalian host cells is most preferred. This is because eukaryotic cells, particularly mammalian cells, are more likely to assemble and secrete properly folded and immunologically active antibodies than prokaryotic cells. Preferred mammalian host cells for expressing the recombinant antibody of the present disclosure include Chinese hamster ovary cells (CHO cells) (including dhfr-CHO cells used with a DHFR selectable marker, as described in Urlaub and Chasin, (1980) Proc. Natl. Acad. Sci. USA 77:4216-4220, the DHFR selectable marker is described, for example, in R. J. Kaufman and P. A. Sharp (1982) J. Mol. Biol. 159:601-621), NSO myeloma cells, COS cells, and SP2 cells. When a recombinant expression vector encoding an antibody gene is introduced into a mammalian host cell, the antibody is prepared by culturing the host cell for a period of time sufficient to allow the expression of the antibody in the host cell, or preferably, sufficient to allow the secretion of the antibody into the medium in which the host cell grows. Antibodies can be isolated from host cells or cell cultures thereof using standard protein purification methods.

In another aspect, the present disclosure provides a pharmaceutical composition comprising the anti-CLDN18.2 antibody or the antigen-binding fragment thereof, the nucleic acid molecule, the recombinant polypeptide, the fusion protein, the multispecific antibody, the immunoconjugate, the chimeric antigen receptor, or the gene vector of the present disclosure, and a pharmaceutically acceptable carrier or excipient. The term "pharmaceutically acceptable carrier or excipient" refers to carriers or excipients that can be used in the preparation of a pharmaceutical composition and are generally safe, non-toxic, and biologically inactive and non-hazardous, including carriers or excipients for veterinary uses and drugs for humans.

The pharmaceutical composition of the present disclosure is suitable for intravenous, intramuscular, subcutaneous, parenteral, spinal, or epidermal administration (e.g., by injection or infusion). Depending on the route of administration, the active ingredient may be encapsulated in a material to protect it from acids and other natural conditions that may inactivate it. The phrase "parenteral administration" used herein refers to modes of administration other than enteral and topical administration that are typically performed by injection, including but not limited to, intravenous, intramuscular, intraarterial, intrathecal, intracapsular, intraorbital, intracardiac, intradermal, intraperitoneal, transtracheal, subcutaneous, subepidermal, intraarticular, subcapsular, subarachnoid, intraspinal, epidural, and intrasternal injection and infusion. Alternatively, the pharmaceutical composition of the present disclosure may be administered through a non-parenteral route, for example, topical, epidermal, or mucosal route, such as intranasal, oral, vaginal, rectal, sublingual, or topical administration.

The pharmaceutical composition of the present disclosure may be in the form of a sterile aqueous solution or a dispersion. They may also be formulated in microemulsions, liposomes, or other ordered structures suitable for high concentrations of drugs.

The administration regimen is adjusted to provide the best desired response (e.g., therapeutic response). For example, a single large dose may be administered, multiple split doses may be administered over time, or the dose may be reduced or increased in proportion to the criticality of the treatment situation. Alternatively, the antibody may be administered as a sustained-release formulation, in which case the frequency of administration required is reduced.

In the administration of the antibody, the dose may be in the range of about 0.0001 mg/kg to 100 mg/kg of host body weight.

Although the foregoing invention has been described in considerable detail by providing illustrations and examples for the purpose of clear understanding, it will be apparent to those of ordinary skills in the art in light of the teachings of the present disclosure that certain changes and modifications can be made to the present disclosure without departing from the spirit or scope of the appended claims. The present disclosure is further explained by the description of the following examples, which are not intended to be limiting. Those skilled in the art will readily identify a variety of noncritical parameters that may be changed or modified to produce substantially similar results.

Unless otherwise indicated, the practice of the present disclosure will use conventional methods in protein chemistry, biochemistry, recombinant DNA technology, and pharmacology within the art. The reagents, materials, and instruments involved in the following examples can be those commercially available in the art, unless otherwise stated.

Examples

**Example 1. Generation of Anti-CLDN18.2 Hybridoma Monoclonal Antibodies**

**Construction of stable cell lines**

The cDNA encoding human CLDN18.2 (SEQ ID NO: 1) was obtained by gene synthesis and then subcloned into the expression vector pcDNA3.1. The expression vector was transfected into U2OS cells and NIH-3T3 cells, respectively, according to the instructions of Lipofectamine 2000 transfection reagent (Thermo, Cat. No. 11668019) to obtain stable cell lines U2OS-CLDN18.2 and NIH-3T3-CLDN18.2, respectively.

The cDNA encoding human CLDN18.1 (SEQ ID NO: 2) was obtained by gene synthesis and then subcloned into the expression vector pcDNA3.1. The expression vector was transfected into U2OS cells and NIH-3T3 cells, respectively, according to the instructions of Lipofectamine 2000 transfection reagent (Thermo, Cat. No. 11668019) to obtain stable cell lines U2OS-CLDN18.1 and NΠ3-3T3-CLDN18.1, respectively.

**Immunization of mice**

BALB/c mice were immunized with the immunogen, the NIH-3T3-CLDN18.2 cells, by intraperitoneally injecting $5 \times 10^6$ to $10 \times 10^6$ NIH-3T3-CLDN18.2 cells into each mouse, and the immunization was performed once every 2 weeks for a total of 5 times. Three days before fusion, each mouse was intraperitoneally injected with $1 \times 10^7$ NIH-3T3-CLDN18.2 cells for rush immunization. After the completion of the immunization, serum was collected from each mouse for the titer assay of anti-CLDN18.2 specific antibodies. Mice with a higher antibody titer were selected for subsequent spleen cell fusion.

**Cell fusion**

Based on the electrofusion technique, spleen cells ($1 \times 10^8$) from immunized mice were fused with SP2/0 myeloma cells ($5 \times 10^7$) using the electrofusion system (BTX, ECM2001) to obtain hybridoma cells. After the fusion, the cells were resuspended in HAT complete medium, added to a 96-well plate at 0.2 mL/well, and cultured in an incubator at 37 °C with 5% $CO_2$ for 7-10 days, and then hybridoma cell culture supernatants were taken for detection.

**Screening of hybridomas**

The binding activity of the antibodies for CLDN18.2 was detected by ELISA. U2OS-CLDN18.2 cells were added to a 96-well plate at 10000 cells/well and cultured overnight in an incubator at 37 °C with 5% $CO_2$. The cell culture supernatant was discarded, the 96-well plate was washed 3 times with PBS buffer, and 2% polyoxymethylene was added, followed by immobilization at room temperature for 30 min. The 96-well plate was washed 3 times with PBS buffer, the hybridoma cell culture supernatant was added at 100 μL/well, and the mixture was incubated at room temperature for 1 h. The 96 well plate was washed 3 times with PBS buffer, HRP-Goat Anti-Mouse IgG Fcγ (Jackson immunoresearch, Cat. No. 115-035-071) was added at 100 μL/well, and the mixture was incubated at room temperature for 1 h. The 96-well plate was washed 3 times with PBS buffer, TMB (Thermo, Cat. No. 00-4201-56) was added at 100 μL/well, and then the mixture was incubated for 10 min, followed by addition of the stop solution (2N $H_2SO_4$) at 50 μL/well to stop the reaction. OD values at a wavelength of 450 nm were read using a microplate reader (Thermo, Varioskan Flash).

Hybridoma cells positive for ELISA detection were selected, and the binding activity of the antibodies in the hybridoma cell culture supernatants to the cell lines KATOIII and NUGC-4 naturally expressing CLDN18.2 was detected by FACS method (see Example 3 for the method).

**Example 2. Generation of Anti-CLDN18.2 Chimeric Antibodies**

**Acquisition of cDNA**

Hybridoma cells positive for ELISA and FACS detection (28G3, 40F6, 22F12, 34G6, and 10D8) were selected, the total RNA was isolated as the template from the cells using a total RNA extraction kit (Takara, Cat. No. 9767), and the first strand cDNA was synthesized using the superscript III reverse transcriptase according to the kit (Thermo, Cat. No. 18080051) instructions. By using the first chain cDNA as the template, the PCR amplification was performed with the mouse IgG primer and the Kappa primer, thus obtaining the sequences of the heavy chain variable region and the light chain variable region of the antibody. The PCR mixture was electrophoretically separated in a 1% agarose/Tris-borate gel containing 0.5 μg/mL ethidium bromide. A DNA fragment of the expected size was cut off from the gel and purified. The purified PCR product was cloned into pMD-19T vector (Takara, Cat. No. 6013), which was then transfected into DH5α competent *E. coli* cells (Takara, Cat. No. 9057) for culturing on an LB solid culture plate. Single colonies were picked from the LB solid culture plate and subjected to DNA sequencing. The heavy chain variable region sequence and light chain variable region sequence of the antibodies (murine antibodies 28G3, 40F6, 22F12, 34G6, and 10D8) were obtained.

**Construction and expression of chimeric antibodies**

The interaction of IgG1 with FcgRIIIa can be improved by knocking out the fucose expression-related gene FUT8, and thereby the ADCC activity of the antibody is enhanced (Shields et al., 2002; Yamane-Ohnuki et al., 2004). In this example, defucosylated antibodies were prepared using FUT8-knockout CHO-S cells (designated CHO-S Fut8-/-cells).

The mouse VL region gene fragment was linked to a human κ chain constant region to construct a chimeric light chain, and the mouse VH region gene fragment was linked to a human IgG1 constant region to construct a chimeric heavy chain. The chimeric heavy chain expression plasmid and chimeric light chain expression plasmid corresponding to each antibody were co-transfected into CHO-S Fut8-/-cells for protein expression. The transfected cells were cultured at 37 °C with 8% $CO_2$. After 7-10 days of culturing, the cells were centrifuged to obtain the cell culture supernatant, and the chimeric antibodies in the cell culture supernatant were purified using a Protein A column (GE healthcare). The human κ light chain constant region had the amino acid sequence set forth in SEQ ID NO: 62 and the human IgG1 constant region had the amino acid sequence set forth in SEQ ID NO: 61.

**Example 3. Determination of Binding Activity of Anti-CLDN18.2 Antibodies**

Based on the FACS method, the binding activity of anti-CLDN18.2 antibodies for the cell line U2OS-CLDN18.2 overexpressing CLDN18.2 and the cell lines KATOIII (Cell Bank of the Chinese Academy of Sciences) and NUGC-4 (Cell Bank of the Chinese Academy of Sciences) naturally expressing CLDN18.2 was analyzed. IMAB362 (prepared in-house, with the heavy and light chain amino acid sequences as set forth in SEQ ID NOs: 63 and 64 in the present disclosure) was used as the positive control, and the IgG1 isotype control antibody (also designated IgG1 in the examples of the present disclosure, prepared in-house, with the heavy and light chain amino acid sequences as set forth in SEQ ID NOs: 67 and 68 in the present disclosure) was used as the negative control.

The cell concentration was adjusted with PBS, and cells (U2OS-CLDN18.2, KATOIII, or NUGC-4 cells) were added to a 96-well plate at $3 \times 10^5$ cells/well, then the test antibodies at different concentrations were added to each well of the 96-well plate, and the mixture was incubated at 4 °C for 60 min. For KATOIII and NUGC-4 cells, the final concentrations of the xi40F6 and xi34G6 antibodies were 300 nM, 100 nM, 33.33 nM, 11.11 nM, 3.7 nM, 1.23 nM, 0.41 nM, and 0.14 nM, and the final concentrations of the xi10D8, xi22F12, and xi28G3 antibodies were 300 nM, 60 nM, 12 nM, 2.4 nM, 0.48 nM, 0.096 nM, 0.0192 nM, and 0.00384 nM; for U2OS-CLDN18.2 cells, the final concentrations of the test antibodies were 150 nM, 50 nM, 16.67 nM, 5.56 nM, 1.85 nM, 0.62 nM, 0.21 nM, and 0.07 nM. The supernatant was discarded, the cells were washed 3 times with pre-cooled PBS buffer, PE-Goat Anti-Human IgG Fcγ (Jackson immunoresearch, Cat. No. 109-116-170) diluted at 1:200 was added at 100 μL/well, and the mixture was incubated at 4 °C for 30 min. Cells were washed 3 times with pre-cooled PBS buffer and resuspended in 100 μL of PBS buffer, and then the fluorescence signals were detected using a flow cytometer (Sartorius, iQUE3). The binding ability of the anti-CLDN18.2 antibodies for CLDN18.2 was measured by mean fluorescence intensity (MFI) of staining.

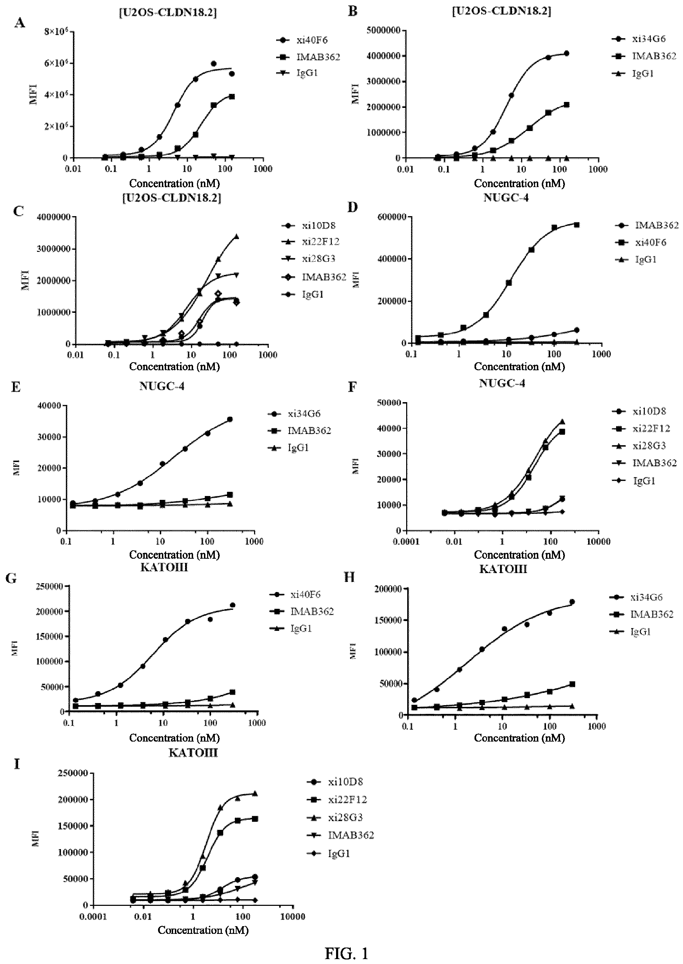

The binding activity of the anti-CLDN18.2 chimeric antibodies for U2OS-CLDN18.2, KATOIII, and NUGC-4 cells is shown in FIG. 1, and the data for binding $EC_{50}$ are shown in Table 2.1, Table 2.2, and Table 2.3. For the three different cells, the binding activity of xi28G3, xi40F6, xi22F12, and xi34G6 was better than that of IMAB362, and the binding activity of xi10D8 was comparable to that of IMAB362.

Table 2.1. $EC_{50}$ for binding of anti-CLDN18.2 chimeric antibodies at the cellular level

| Antibody | EC50 (nM) | | |
|---|---|---|---|
| | U2OS-CLDN18.2 | NUGC-4 | KATOIII |
| xi40F6 | 4.47 | 12.5 | 5.97 |
| IMAB362 | 22.93 | 566.8 | ~26889 |

Table 2.2. EC$_{50}$ for binding of anti-CLDN18.2 chimeric antibodies at the cellular level

| Antibody | EC50 (nM) | | |
|---|---|---|---|
| | U2OS-CLDN18.2 | NUGC-4 | KATOIII |
| xi34G6 | 4.28 | 19.95 | 1.795 |
| IMAB362 | 16.21 | 202.2 | ~1.662e+007 |

Table 2.3. EC$_{50}$ for binding of anti-CLDN18.2 chimeric antibodies at the cellular level

| Antibody | EC50 (nM) | | |
|---|---|---|---|
| | U2OS-CLDN18.2 | NUGC-4 | KATOIII |
| xi10D8 | 19.46 | 512.2 | 13.89 |
| xi22F12 | 25.14 | 20.0 | 3.68 |
| xi28G3 | 7.78 | 21.22 | 3.31 |
| IMAB362 | 16.04 | 1454 | 81.30 |

## Example 4. Non-Specific Binding of Anti-CLDN18.2 Antibodies

Based on the FACS method, the binding activity of anti-CLDN18.2 antibodies for the cell line U2OS-CLDN18.1 overexpressing CLDN18.1 (SEQ ID NO: 2) was analyzed. The cell concentration was adjusted with PBS, and the U2OS-CLDN18.1 cells were added to a 96-well plate at $3 \times 10^5$ cells/well, then the test antibodies were added to each well of the 96-well plate, the final concentration of the antibody was 200 nM, and the mixture was incubated at 4 °C for 60 min. The supernatant was discarded, the cells were washed 3 times with pre-cooled PBS buffer, PE-Goat Anti-Human IgG Fcγ (Jackson immunoresearch, Cat. No. 109-116-170) diluted at 1:200 was added at 100 μL/well, and the mixture was incubated at 4 °C for 30 min. Cells were washed 3 times with pre-cooled PBS buffer and resuspended in 100 μL of PBS buffer, and then the fluorescence signals were detected using a flow cytometer (Sartorius, iQUE3). The nonspecific binding of the anti-CLDN18.2 antibodies for U2OS-CLDN18.1 cells was measured by mean fluorescence intensity (MFI) of staining. The IgG1 isotype control antibody (prepared in-house, with the heavy and light chain amino acid sequences as set forth in SEQ ID NOs: 67 and 68 in the present disclosure) was used as the control, and 2-fold the IgG1 isotype control antibody was used as the benchmark, thus determining the presence or absence of nonspecific binding.

The results are shown in Table 3. The MFI values of the anti-CLDN18.2 chimeric antibodies and the IgG1 isotype control antibody in the control group were similar, indicating that the anti-CLDN18.2 chimeric antibodies did not non-specifically bind to U2OS-CLDN18.1 cells.

Table 3. Non-specific binding of anti-CLDN18.2 chimeric antibodies to U2OS-CLDN18.1 cells

| Antibody | MFI |
|---|---|
| xi10D8 | 8031 |
| xi22F12 | 8943 |
| xi28G3 | 7556 |
| xi40F6 | 7071 |
| xi34G6 | 7633 |
| IMAB362 | 6221 |
| IgG1 | 6035 |

## Example 5. ADCC Activity of Anti-CLDN18.2 Antibodies

Based on the reporter gene method, the antibody-dependent cellular cytotoxicity (ADCC) activity of anti-CLDN18.2 antibodies was determined using KATOIII, NUGC-4, and U2OS-CLDN18.2 as target cells and Jurkat cell stably transfected with CD16a v158 and NFAT-luciferase (Jurkat-CD16a v158-NFAT-luciferase) (BPS Bioscience, Cat. No. 60541) as the effector cell. IMAB362 (prepared in-house, with the heavy and light chain amino acid sequences as set forth in SEQ ID NOs: 63 and 64 in the present disclosure) was used as the positive control, and the IgG1 isotype control antibody (prepared in-house, with the heavy and light chain amino acid sequences as set forth in SEQ ID NOs: 67 and 68 in the present disclosure) was used as the negative control.

KATOIII, NUGC-4, and U2OS-CLDN18.2 cells were each diluted to $2.4 \times 10^5$ cells/mL with the medium, each cell suspension was added to a 96-well plate at 100 $\mu$L/well, and the mixture was incubated overnight in an incubator at 37 °C with 5% $CO_2$. The next day, the culture supernatant was discarded, and the 96-well plate was washed 1-3 times with PBS buffer. The test antibodies at different concentrations were then added to the 96-well plate at 60 $\mu$L/well; for KATOIII cells, the final concentrations of the anti-CLDN18.2 antibodies of the present disclosure were 20 nM, 2 nM, 0.2 nM, 0.02 nM, 0.002 nM, 0.0002 nM, 0.00002 nM, and 0.000002 nM, and the final concentrations of IMAB362 were 2000 nM, 200 nM, 20 nM, 2 nM, 0.2 nM, 0.02 nM, 0.002 nM, and 0.0002 nM; for NUGC-4 cells, the final concentrations of the anti-CLDN18.2 antibodies of the present disclosure and IMAB362 were 1333.33 nM, 133.33 nM, 13.33 nM, 1.33 nM, 0.133 nM, 0.0133 nM, 0.00133 nM, and 0.000133 nM; for U2OS-CLDN18.2 cells, the final concentrations of the anti-CLDN18.2 antibodies of the present disclosure and IMAB362 were 1.334 nM, 0.267 nM, 0.053 nM, 0.011 nM, 0.002 nM, 0.0004 nM, and 0.00009 nM. Jurkat-CD16a v158-NFAT-luciferase cells were then taken out; the Jurkat-CD16a v158-NFAT-luciferase cell suspension was added to the 96-well plate at 40 $\mu$L/well based on the ratio of effector cells:target cells of 5:1. Centrifugation at low speed was performed to allow the effector cells and the target cells to be sufficiently mixed and in contact with each other, and the mixture was incubated in an incubator at 37 °C with 5% $CO_2$ for 5-6 h. The detection reagent was then added at 100 $\mu$L/well according to the instructions of the firefly luciferase detection kit (Vazyme, Cat. No. DD1203-03), and the mixture was left to stand for 15-30 min, and the fluorescence signals were detected by using a microplate reader (Thermo, Varioskan Flash).

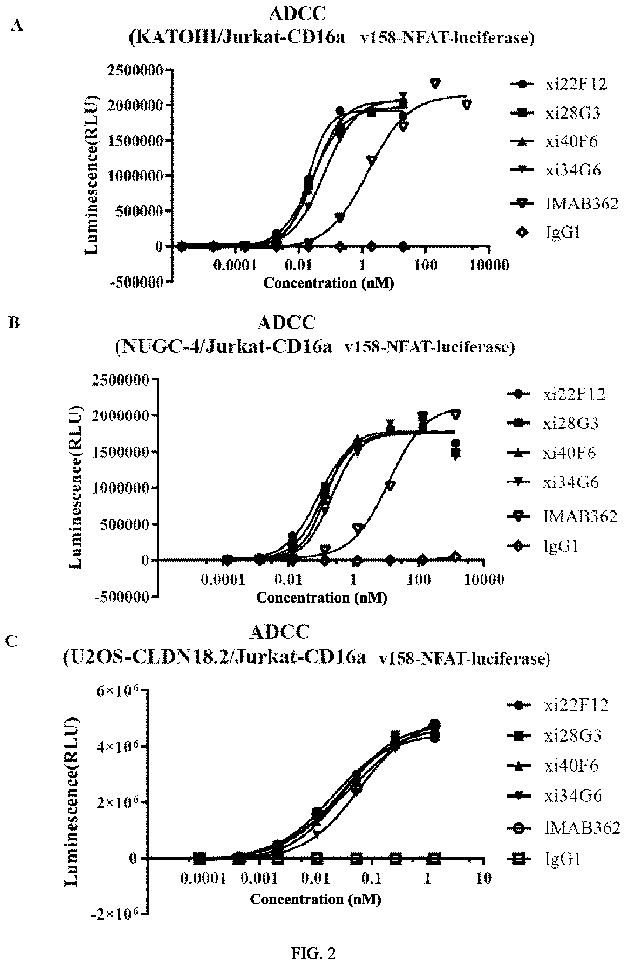

In the reporter gene experiment, the ADCC activity of the anti-CLDN18.2 chimeric antibodies is shown in FIG. 2, and the data for binding $EC_{50}$ are shown in Table 4. For the three different cells, the chimeric antibodies xi28G3, xi40F6, xi34G6, and xi22F12 were able to significantly induce the activation of NFAT signals of Jurkat-CD16a v158-NFAT-luciferase cells and were better than IMAB362.

Table 4. ADCC activity of anti-CLDN18.2 chimeric antibodies (with Jurkat-CD16a v158-NFAT-luciferase as the effector cell

| Antibody | EC50 (nM) | | |
|---|---|---|---|
| | KATOIII | NUGC-4 | U2OS-CLDN18.2 |
| xi28G3 | 0.03 | 0.121 | 0.028 |
| xi40F6 | 0.03 | 0.147 | 0.032 |
| xi34G6 | 0.064 | 0.214 | 0.060 |
| xi22F12 | 0.02 | 0.083 | 0.020 |
| IMAB362 | 1.525 | 11.96 | 0.050 |

Based on the killing effect of PBMC, the ADCC activity of the anti-CLDN18.2 antibodies was detected using KATOIII and NUGC-4 as target cells and PBMC as the effector cell. IMAB362 (prepared in-house, with the heavy and light chain amino acid sequences as set forth in SEQ ID NOs: 63 and 64 in the present disclosure) and 15F9 (prepared in-house, with the heavy and light chain amino acid sequences as set forth in SEQ ID NOs: 65 and 66 in the present disclosure) were used as the positive controls, and the IgG1 isotype control antibody (prepared in-house, with the heavy and light chain amino acid sequences as set forth in SEQ ID NOs: 67 and 68 in the present disclosure) was used as the negative control.

KATOIII and NUGC-4 cells were each diluted to $2 \times 10^5$ cells/mL with the medium, and each cell suspension was added to a 96-well plate at 50 $\mu$L/well. The test antibodies at different concentrations were then added to the wells of the 96-well plate; for KATOIII cells, the final concentrations of the test antibodies were 66.7 nM, 8.34 nM, 1.04 nM, 0.13 nM, 0.016 nM, 0.002 nM, 0.00025 nM, and 0.00003 nM; for NUGC-4 cells, the final concentrations of the test antibodies were 600 nM, 100 nM, 16.67 nM, 2.78 nM, 0.46 nM, 0.077 nM, 0.013 nM, and 0.002 nM. PBMC cells were resuscitated; the PBMC cell suspension (cell density of $4 \times 10^6$/mL) was added to the 96-well plate at 50 $\mu$L/well based on the ratio of effector cells:target cells of 20:1. Centrifugation at low speed was performed to allow the effector cells and the target cells to be sufficiently mixed and in contact with each other, and the mixture was incubated in an incubator at 37 °C with 5% $CO_2$ for 18 h. The 96-well plate was then placed in a plate centrifuge and centrifuged at 250 g/min for 5 min, and 50 $\mu$L of supernatant from each well was added to a 96-well detection plate. The LDH working solution was prepared according to the instructions of the CytoTox96 Non-Radio kit (Promega, Cat. No. PR-G1780) and then added to the 96-well detection plate at 50 μL/well, and after sufficient mixing, the mixture was incubated at room temperature for 25-35 min in the dark. The stop solution was added at 50 μL/well, and after sufficient mixing, OD values at a wavelength of 490 nm were read using a microplate reader (Thermo, Varioskan Flash).

$$\text{Killing rate (\%)} = \frac{\text{OD value of experimental group - OD value of target cell control group - OD value of effector cell control group}}{\text{maximum OD value of target cell plus lysis solution group - OD value of target cell control group}} \times 100\%$$

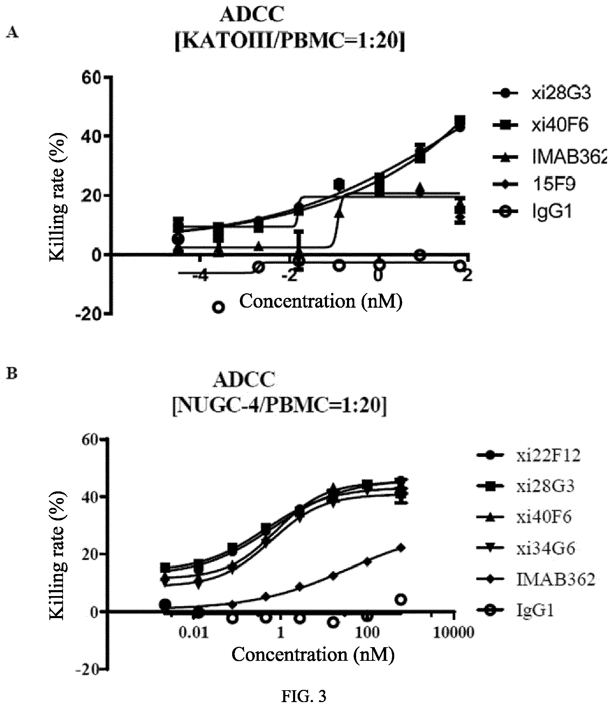

As shown in FIG. 3 and Table 5, in the PBMC killing experiment, the ADCC activity of the anti-CLDN18.2 chimeric antibodies xi22F12, xi28G3, xi40F6, and xi34G6 was better than that of IMAB362 and 15F9.

Table 5. ADCC activity of anti-CLDN18.2 chimeric antibodies with PBMC as the effector cell

| Antibody | KATOIII | | NUGC-4 | |
|---|---|---|---|---|
| | EC50 (nM) | Maximum killing (%) | EC50 (nM) | Maximum killing (%) |
| xi22F12 | / | / | 0.68 | 46.13 |
| xi28G3 | 38.44 | 76.77 | 0.44 | 43.29 |
| xi40F6 | NA | NA | 0.8 | 45.16 |
| xi34G6 | / | / | 0.62 | 40.92 |
| IMAB362 | ~ 0.1227 | 20.7 | 37.13 | 29.15 |
| 15F9 | ~ 0.01554 | 19.5 | / | / |
| "NA" indicates not applicable, and "f" indicates not detectable | | | | |

## Example 6. CDC Activity of Anti-CLDN18.2 Antibodies

Based on the method of CellTiter-Glo Luminescent Cell Viability Assay, the complement-dependent cytotoxicity (CDC) activity of the antibodies was detected using KATOIII and U2OS-CLDN18.2 as target cells. IMAB362 (prepared in-house, with the heavy and light chain amino acid sequences as set forth in SEQ ID NOs: 63 and 64 in the present disclosure) was used as the positive control, and the IgG1 isotype control antibody (prepared in-house, with the heavy and light chain amino acid sequences as set forth in SEQ ID NOs: 67 and 68 in the present disclosure) was used as the negative control.

KATOIII and U2OS-CLDN18.2 were each diluted to $8 \times 10^5$ cells/mL with the medium, and each cell suspension was added to a 96-well plate at 50 μL/well. The test antibodies at different concentrations were then added to the 96-well plate at 50 μL/well; for KATOIII cells, the final concentrations of the test antibodies were 50 nM, 10 nM, 2 nM, 0.4 nM, 0.08 nM, 0.016 nM, 0.0032 nM, and 0.00064 nM; for U2OS-CLDN18.2 cells, the final concentrations of the test antibodies were 333.33 nM, 66.67 nM, 13.33 nM, 2.67 nM, 0.53 nM, 0.11 nM, 0.02 nM, and 0.004 nM. Complement (Quidel, Cat. No. A113) was then added to the 96-well plate at 50 μL/well to allow the final concentration of the complement to be 10%. The mixture was centrifuged at low speed, and then the 96-well plate was incubated at 37 °C for 30 min with shaking to allow the complement and the target cells to be sufficiently mixed and in contact with each other. Then the mixture was incubated in an incubator at 37 °C with 5% $CO_2$ for 2 h. After the incubation, the CellTiter-Glo Luminescent Cell Viability Assay (Promega, Cat. No. G7572) was added at 110 μL/well, the mixture was then incubated at room temperature for 5-15 min in the dark, and finally the fluorescence values were measured by using a microplate reader (Thermo, Varioskan Flash).

$$\text{Killing rate (\%)} = 1 - \frac{\text{fluorescence value of experimental group - fluorescence value of blank control group}}{\text{fluorescence value of target cell control group - fluorescence value of blank control group}} \times 100\%$$

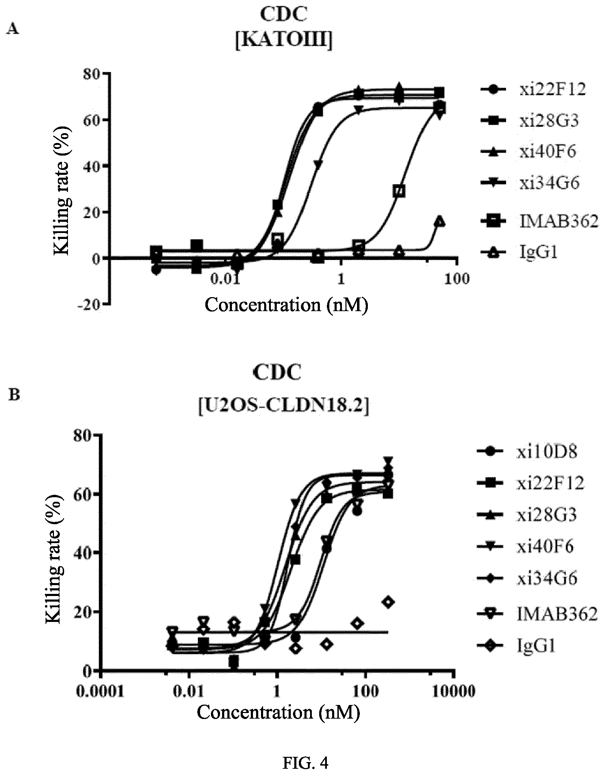

The results are shown in FIG. 4 and Table 6. The anti-CLDN18.2 chimeric antibodies xi22F12, xi28G3, xi40F6, xi34G6, and xi10D8 were all able to induce strong CDC effect, and the activity of xi22F12, xi28G3, xi40F6, and xi34G6 was stronger than that of IMAB362.

Table 6. CDC activity of anti-CLDN18.2 chimeric antibodies

| Antibody | EC50 (nM) | |
|---|---|---|
| | KATOIII | U2OS-CLDN18.2 |
| xi22F12 | 0.103 | 2.089 |
| xi28G3 | 0.112 | 1.584 |
| xi40F6 | 0.127 | 1.069 |
| xi34G6 | 0.302 | 1.772 |
| xi10D8 | / | 11.44 |
| IMAB362 | 12.85 | 9.79 |
| "/" indicates not detectable | | |

**Example 7. InternalizationActivity of Anti-CLDN18.2 Antibodies**

According to the instructions of the Antibody Internalization Human Reagent (Sartorius, Cat. No. 90564), the internalization activity of the anti-CLDN18.2 antibodies was detected using NUGC-4 and NIH-3T3-CLDN18.2 as target cells.

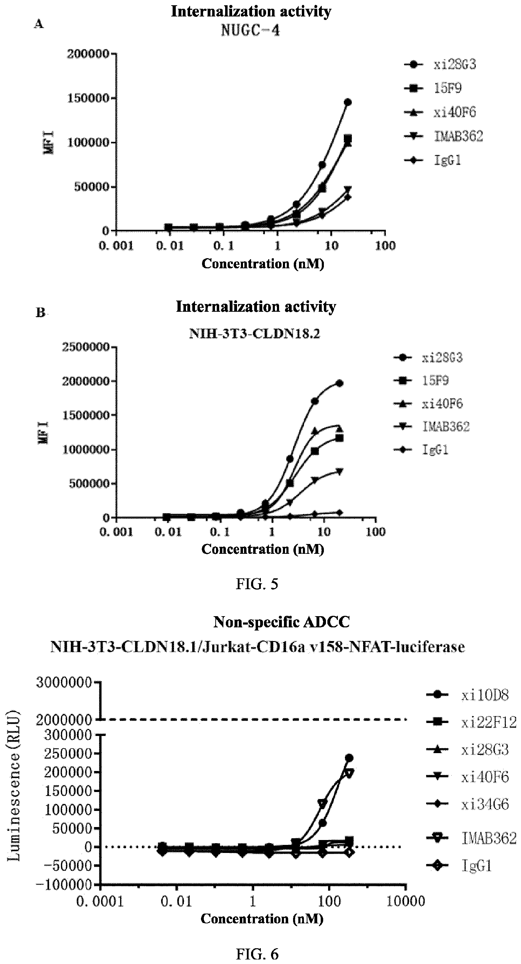

NUGC-4 and NIH-3T3-CLDN18.2 cells were each diluted to $2 \times 10^6$ cells/mL with the medium, and each cell suspension was added to a 96-well plate at 20 μL/well. The test antibodies were each sufficiently mixed with the internalization reagent, and the mixture of the test antibody and the internalization reagent was incubated at 37 °C for 15 min in the dark and then added to the 96-well plate at 20 μL/well, and the resulting mixture was incubated in an incubator at 37 °C with 5% $CO_2$ for 2 h; the final concentrations of the test antibodies were 20 nM, 6.67 nM, 2.22 nM, 0.74 nM, 0.25 nM, 0.08 nM, 0.027 nM, and 0.009 nM. After the incubation, the fluorescence signals (MFI) were detected using a flow cytometer (Sartorius, iQUE3). The internalization activity of the antibodies was evaluated by the fluorescence values, and the results are shown in FIG. 5.

**Example 8. Non-specific ADCC Activity of Anti-CLDN18.2 Antibodies**

Based on the reporter gene method, the non-specific ADCC activity of anti-CLDN18.2 antibodies for NIH-3T3-CLAN18.1 cells was analyzed using NIH-3T3-CLDN18.1 cell as the target cell and Jurkat-CD16a v158-NFAT-luciferase as the effector cell, and the final concentrations of the test antibodies were 333.33 nM, 66.67 nM, 13.33 nM, 2.67 nM, 0.53 nM, 0.107 nM, 0.021 nM, and 0.004 nM. See Example 5 for the specific method.

The results are shown in FIG. 6. The anti-CLDN18.2 chimeric antibodies xi22F12, xi28G3, xi40F6, and xi34G6 all had no ADCC effect for NIH-3T3-CLDN18.1 cells, while xi10D8 and IMAB362 had weak ADCC activity for NIH-3T3-CLDN18.1 cells.

**Example 9. Humanization of Anti-CLDN18.2 Monoclonal Antibodies**

Murine antibodies 28G3 (heavy chain variable region: SEQ ID NO:11; light chain variable region: SEQ ID NO: 16) and 40F6 (heavy chain variable region: SEQ ID NO: 29; light chain variable region: SEQ ID NO: 34) were selected for humanization.

The murine antibodies 28G3 and 40F6 were humanized by CDR-grafting in combination with computer-aided design. Human germline antibody sequences with the highest sequence homology to the heavy and the light chain variable region sequences of the murine antibodies were selected from the protein databases by alignment. The complementarity determining regions (CDRs) of the murine antibodies 28G3 and 40F6 were grafted between the framework regions of the selected human germline antibody sequences, and the CDRs and/or amino acid residues in the framework regions were further mutated to obtain more candidate variable region sequences.

A humanized VL region gene fragment was linked to a human κ chain constant region to construct a humanized light chain, and a humanized VH region gene fragment was linked to a human IgG1 constant region to construct a humanized heavy chain. The transfection and expression were performed according to the method in Example 2, and then the humanized antibodies in the cell culture supernatant were purified using a Protein A column. The human κ light chain constant region had the amino acid sequence set forth in SEQ ID NO: 62 and the human IgG1 constant region had the amino acid sequence set forth in SEQ ID NO: 61.

**Example 10. Characterization of Anti-CLDN18.2 Humanized Antibodies**

**Binding activity**

Based on the FACS method, the binding activity of anti-CLDN18.2 humanized antibodies for the cell lines KATOIII (Cell Bank of the Chinese Academy of Sciences) and NUGC-4 (Cell Bank of the Chinese Academy of Sciences) naturally expressing CLDN18.2 was analyzed. The final concentrations of hz28G3-1.1, hz28G3-1.2, hz28G3-1.3, hz28G3-1.4, hz28G3-2.1, hz28G3-2.2, hz28G3-2.3, hz28G3-2.4, and xi28G3 were 150 nM, 50 nM, 16.67 nM, 5.56 nM, 1.85 nM, 0.62 nM, 0.21 nM, and 0.07 nM, and the final concentrations of hz40F6-1.1, hz40F6-1.2, hz40F6-1.3, hz40F6-1.4, hz40F6-2.1, hz40F6-2.2, hz40F6-2.3, hz40F6-2.4, and xi40F6 were 300 nM, 100 nM, 33.33 nM, 11.11 nM, 3.7 nM, 1.23 nM, 0.41 nM, and 0.14 nM. See Example 3 for the specific method.

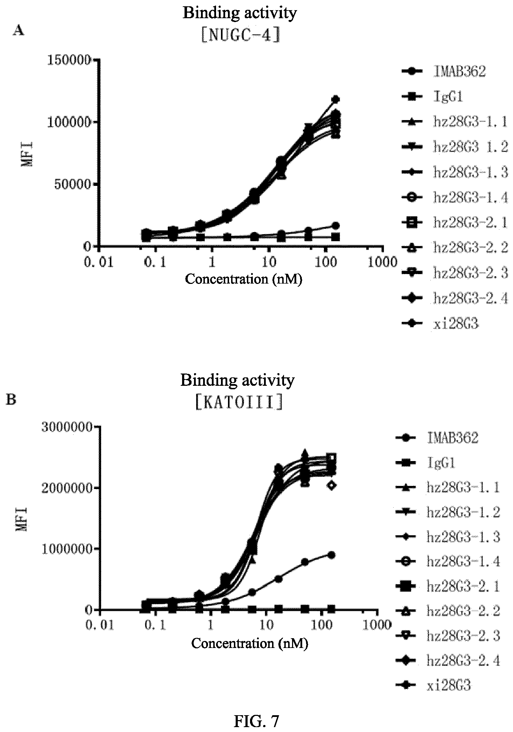

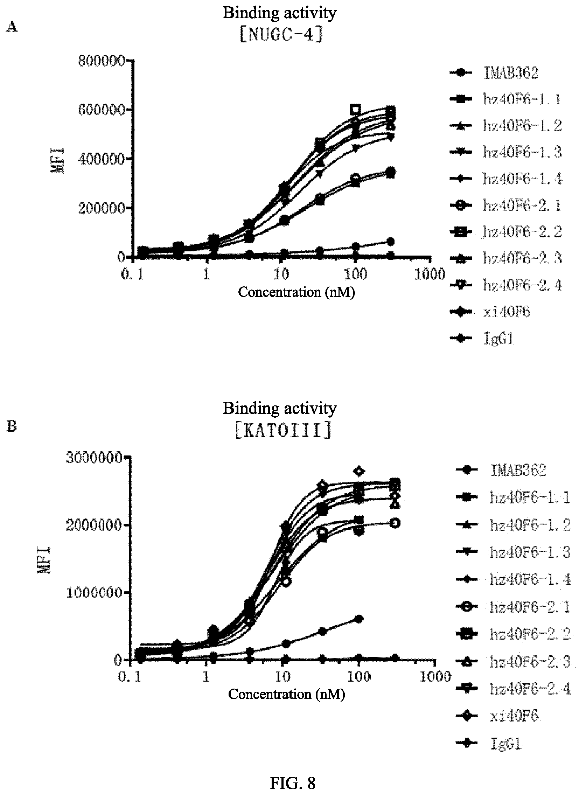

The binding activity of the anti-CLDN18.2 humanized antibodies is shown in FIGs. 7-8, and the data for binding $EC_{50}$ are shown in Table 7.1 and Table 7.2. Except that the binding activity of hz40F6-1.1 and hz40F6-2.1 was weaker than that of the chimeric antibody xi40F6, the binding activity of the remaining humanized antibodies was comparable to or better than that of the corresponding chimeric antibodies, and the binding activity of all humanized antibodies was better than that of IMAB362.

Table 7.1. Binding activity of anti-CLDN18.2 humanized antibodies

| Antibody | EC50 (nM) | |
|---|---|---|
| | NUGC-4 | KATOIII |
| hz28G3-1.1 | 11.14 | 8.11 |
| hz28G3-1.2 | 14.07 | 5.84 |
| hz28G3-1.3 | 10.47 | 7.01 |
| hz28G3-1.4 | 14.03 | 5.83 |
| hz28G3-2.1 | 12.08 | 7.25 |
| hz28G3-2.2 | 12.43 | 5.92 |
| hz28G3-2.3 | 11.95 | 6.54 |
| hz28G3-2.4 | 14.17 | 5.62 |
| xi28G3 | 44.10 | 6.63 |
| IMAB362 | 293.4 | 16.32 |

Table 7.2. Binding activity of anti-CLDN18.2 humanized antibodies

| Antibody | EC50 (nM) | |
|---|---|---|
| | NUGC-4 | KATOIII |
| hz40F6-1.1 | 19.99 | 8.24 |
| hz40F6-1.2 | 16.03 | 7.42 |
| hz40F6-1.3 | 17.71 | 7.9 |
| hz40F6-1.4 | 10.59 | 6.44 |
| hz40F6-2.1 | 18.69 | 8.77 |
| hz40F6-2.2 | 14.86 | 7.54 |
| hz40F6-2.3 | 16.21 | 6.13 |
| hz40F6-2.4 | 13.35 | 6.38 |
| xi40F6 | 12.5 | 7.02 |
| IMAB362 | 566.8 | 38.22 |

**ADCC Activity**

Based on the reporter gene method, the ADCC activity of anti-CLDN18.2 humanized antibodies was analyzed using KATOIII, NUGC-4, NIH-3T3-CLDN18.2, and U2OS-CLDN18.2 as target cells and Jurkat-CD16a v158-NFAT-luciferase as the effector cell. For NIH-3T3-CLDN18.2 and U2OS-CLDN18.2 cells, the final concentrations of the test antibodies were 13.33 nM, 2.22 nM, 0.37 nM, 0.062 nM, 0.01 nM, 0.0017 nM, 0.00029 nM, and 0.000048 nM; for NUGC-4 cells, the final concentrations of the test antibodies were 1333.33 nM, 133.33 nM, 13.33 nM, 1.33 nM, 0.133 nM, 0.0133 nM, 0.00133 nM and 0.000133 nM; for KATOIII cells, the final concentrations of the anti-CLDN18.2 antibodies of the present disclosure were 20 nM, 2 nM, 0.2 nM, 0.02 nM, 0.002 nM, 0.0002 nM, 0.00002 nM, and 0.000002 nM, and the final concentrations of IMAB362 were 2000 nM, 200 nM, 20 nM, 2 nM, 0.2 nM, 0.02 nM, 0.002 nM, and 0.0002 nM. See Example 5 for the specific method.

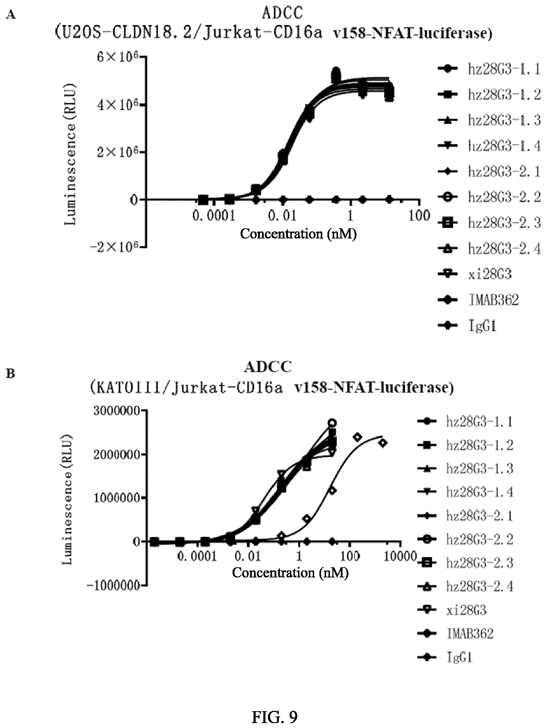

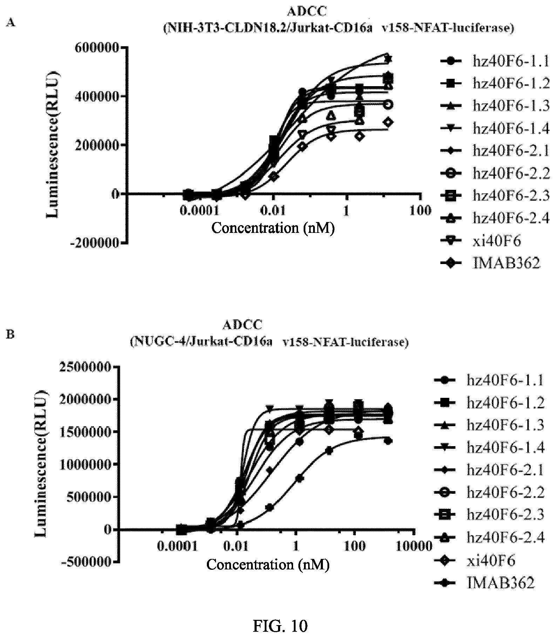

In the reporter gene experiment, the ADCC activity of the anti-CLDN18.2 humanized antibodies is shown in FIG. 9 and FIG. 10, and the data for $EC_{50}$ binding are shown in Table 8.1 and Table 8.2. For the two different cells, the anti-CLDN18.2 humanized antibodies were all able to significantly induce the activation of NFAT signals of Jurkat-CD16a v158-NFAT-luciferase cells, and the ADCC activity of the humanized antibodies was comparable to that of corresponding chimeric antibodies and was better than that of IMAB362.

Table 8.1. ADCC activity of anti-CLDN18.2 humanized antibodies (with Jurkat-CD16a v158-NFAT-luciferase as the effector cell)

| Antibody | EC50 (nM) | |
| --- | --- | --- |
| | KATOIII | U2OS-CLDN18.2 |
| hz28G3-1.1 | 0.139 | 0.016 |
| hz28G3-1.2 | 0.396 | 0.015 |
| hz28G3-1.3 | 0.217 | 0.023 |
| hz28G3-1.4 | 0.184 | 0.017 |
| hz28G3-2.1 | 0.184 | 0.019 |
| hz28G3-2.2 | 0.861 | 0.017 |
| hz28G3-2.3 | 0.209 | 0.019 |
| hz28G3-2.4 | 0.275 | 0.017 |
| xi28G3 | 0.043 | 0.018 |
| IMAB362 | 16.87 | 0.014 |

Table 8.2. ADCC activity of anti-CLDN18.2 humanized antibodies (with Jurkat-CD16a v15 8-NFAT-luciferase as the effector cell

| Antibody | EC50 (nM) | |
| --- | --- | --- |
| | NUGC-4 | NIH-3T3-CLDN18.2 |
| hz40F6-1.1 | 0.045 | 0.013 |
| hz40F6-1.2 | 0.019 | 0.03 |
| hz40F6-1.3 | 0.023 | 0.017 |
| hz40F6-1.4 | 0.017 | 0.024 |
| hz40F6-2.1 | 0.149 | 0.012 |
| hz40F6-2.2 | 0.019 | 0.01 |
| hz40F6-2.3 | 0.028 | 0.014 |
| hz40F6-2.4 | 0.030 | 0.014 |
| xi40F6 | -0.014 | 0.014 |

(continued)

| Antibody | EC50 (nM) | |
|---|---|---|
| | NUGC-4 | NIH-3T3-CLDN18.2 |
| IMAB362 | 0.856 | 0.023 |

Based on the PBMC killing method, the ADCC activity of the anti-CLDN18.2 humanized antibodies was analyzed using KATOIII, NUGC-4, and NIH-3T3-CLDN18.2 as target cells and PBMC as the effector cell. The final concentrations of the test antibodies were 66.7 nM, 8.34 nM, 1.04 nM, 0.13 nM, 0.016 nM, 0.002 nM, 0.00025 nM, and 0.000032 nM. See example 5 for the specific method.

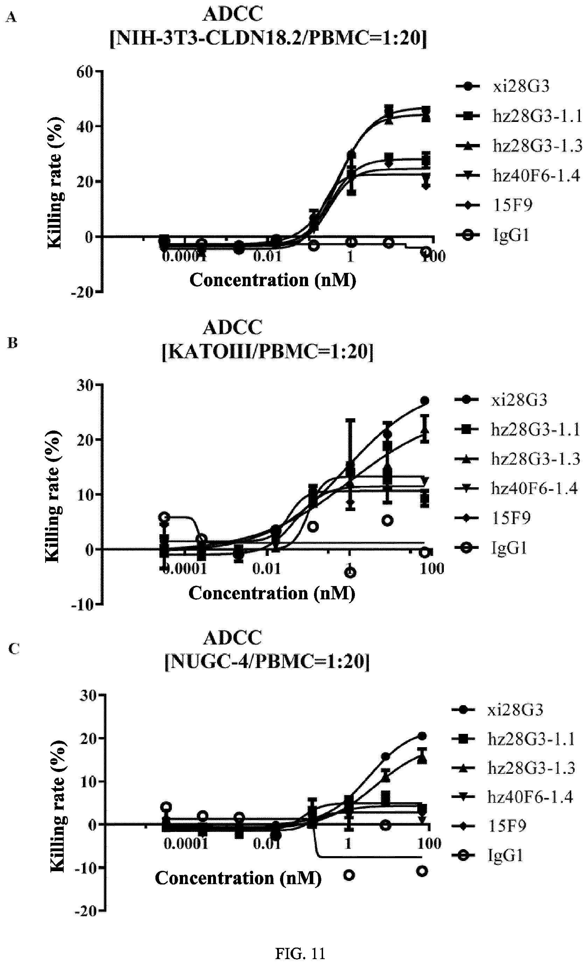

Results as shown in FIG. 11 and Table 9. In the PBMC killing experiment, the ADCC activity of the anti-CLDN18.2 humanized antibody hz28G3-1.3 was comparable to that of xi28G3 and was significantly better than that of 15F9.

Table 9. ADCC activity of anti-CLDN18.2 humanized antibodies with PBMC as the effector cell

| Antibody | NIH-3T3-CLDN18.2 | | KATOIII | | NUGC-4 | |
|---|---|---|---|---|---|---|
| | EC50 (nM) | Maximum killing (%) | EC50 (nM) | Maximum killing (%) | EC50 (nM) | Maximum killing (%) |
| xi28G3 | 0.54 | 46.96 | 0.82 | 29.88 | 3.26 | 22.49 |
| hz28G3-1.1 | 0.33 | 28.14 | 0.048 | 11.5 | 0.07 | 4.9 |
| hz28G3-1.3 | 0.51 | 44.26 | 1.03 | 25.26 | 4.6 | 18.98 |
| hz40F6-1.4 | 0.31 | 24.57 | 0.1 | 13.29 | 0.12 | 2.83 |
| 15F9 | 0.18 | 22.55 | 0.03 | 10.64 | 0.3 | 4.34 |

**CDC Activity**

Based on the method of CellTiter-Glo Luminescent Cell Viability Assay, the CDC activity of the antibodies was detected using NIH-3T3-CLDN18.2 as the target cell. IMAB362 (prepared in-house, with the heavy and light chain amino acid sequences as set forth in SEQ ID NOs: 63 and 64 in the present disclosure) and 15F9 (prepared in-house, with the heavy and light chain amino acid sequences as set forth in SEQ ID NOs: 65 and 66 in the present disclosure) were used as the positive controls, and the IgG1 isotype control antibody (prepared in-house, with the heavy and light chain amino acid sequences as set forth in SEQ ID NOs: 67 and 68 in the present disclosure) was used as the negative control. NIH-3T3-CLDN18.2 cells were diluted to $8 \times 10^5$ cells/mL with the medium containing 0.1% BSA, and cell suspension was added to a 96-well plate at 50 μL/well. The test antibodies at different concentrations were then added to the 96-well plate at 50 μL/well. The final concentrations of hz28G3-1.1, hz28G3-1.3, and xi28G3 were 100 nM, 25 nM, 6.25 nM, 1.56 nM, 0.39 nM, 0.098 nM, 0.024 nM, and 0.006 nM, and the final concentrations of hz40F6-1.1, hz40F6-1.2, hz40F6-1.3, hz40F6-1.4, hz40F6-2.1, hz40F6-2.2, hz40F6-2.3, hz40F6-2.4, and xi40F6 were 333.33 nM, 66.67 nM, 13.33 nM, 2.67 nM, 0.53 nM, 0.11 nM, 0.02 nM, and 0.004 nM. See Example 6 for the specific method.

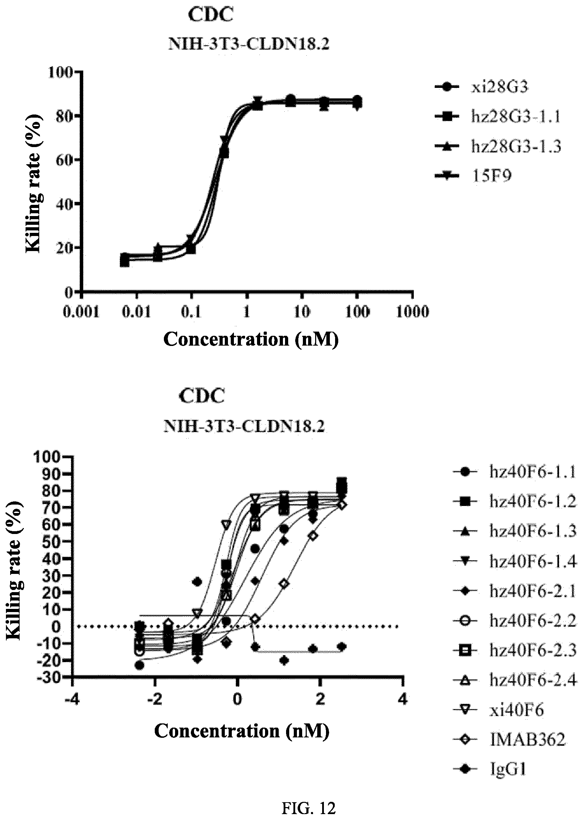

The results are shown in FIG. 12 and Table 10.1 and Table 10.2. The anti-CLDN18.2 humanized antibodies were all able to induce strong CDC effect, and except for a few antibodies, the CDC activity of the other humanized antibodies was comparable to that of corresponding chimeric antibodies.

Table 10.1. CDC activity of anti-CLDN18.2 humanized antibodies

| Antibody | EC50 (nM) |
|---|---|
| xi28G3 | 0.27 |
| hz28G3-1.1 | 0.29 |
| hz28G3-1.3 | 0.31 |
| 15F9 | 0.25 |

Table 10.2. CDC activity of anti-CLDN18.2 humanized
antibodies

| Antibody | EC50 (nM) |
|---|---|
| xi40F6 | 0.29 |
| hz40F6-1.1 | 1.47 |
| hz40F6-1.2 | 0.54 |
| hz40F6-1.3 | 0.84 |
| hz40F6-1.4 | 0.55 |
| hz40F6-2.1 | 3.8 |
| hz40F6-2.2 | 0.53 |
| hz40F6-2.3 | 0.9 |
| hz40F6-2.4 | 0.89 |
| IMAB362 | 24.43 |

**Internalization activity**

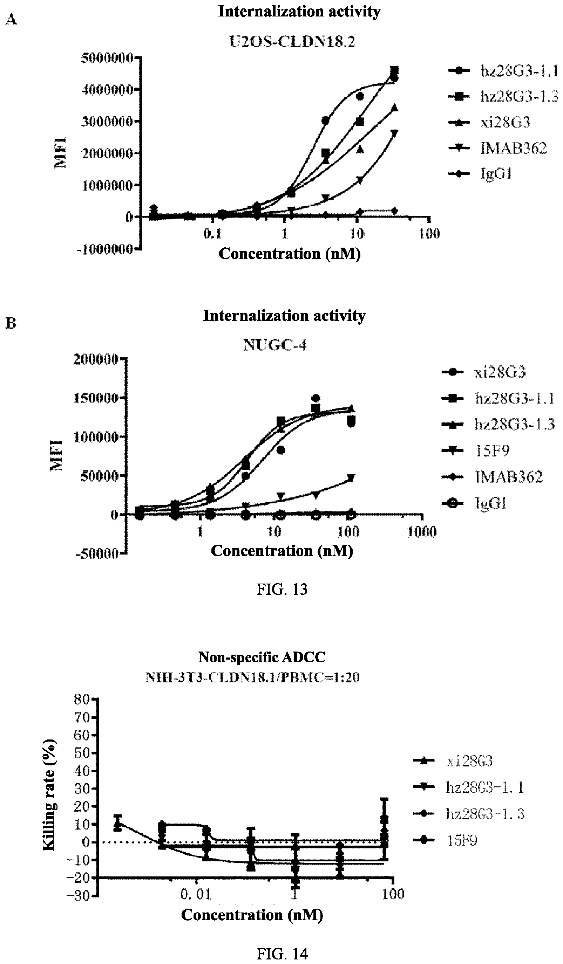

According to the instructions of the Antibody Internalization Human Reagent (Sartorius, Cat. No. 90564), the internalization activity of the anti-CLDN18.2 humanized antibodies was detected using NUGC-4 and U2OS-CLDN18.2 as target cells. The final concentrations of the test antibodies in NUGC-4 cells were 111.11 nM, 37.04 nM, 12.35 nM, 4.12 nM, 1.37 nM, 0.46 nM, and 0.15 nM, and in U2OS-CLDN18.2 cells, the final concentrations of the test antibodies were 33.33 nM, 11.11 nM, 3.7 nM, 1.23 nM, 0.41 nM, 0.14 nM, 0.046 nM, and 0.015 nM. See Example 7 for the specific method. The internalization activity of the antibodies was evaluated by the fluorescence values, and the results are shown in FIG. 13.

**Non-specific ADCC activity**

Based on the PBMC killing method, the non-specific ADCC activity of the anti-CLDN18.2 humanized antibodies for NIH-3T3-CLDN18.1 cells was analyzed using NIH-3T3-CLDN18.1 as the target cell and PBMC as the effector cell. The final concentrations of the test antibodies were 66.7 nM, 8.34 nM, 1.04 nM, 0.13 nM, 0.016 nM, 0.002 nM, and 0.00025 nM. See Example 5 for the specific method.

The results are shown in FIG. 14. The anti-CLDN18.2 humanized antibodies hz28G3-1.1 and hz28G3-1.3 had no ADCC effect on NIH-3T3-CLDN18.1 cells.

The sequence information of the present disclosure is summarized in Table 11 below.

Table 11. Sequence information

| Description Sequence/SEQ ID NO: |
|---|
| Human CLDN18.2: MAVTACQGLGFVVSLIGIAGIIAATCMDQWSTQDLYNNPVTAVFNYQGLWRSCVRESSGFTECRGY FTLLGLPAMLQAVRALMIVGIVLGAIGLLVSIFALKCIRIGSMEDSAKANMTLTSGIMFIVSGLCAIAG VSVFANMLVTNFWMSTANMYTGMGGMVQTVQTRYTFGAALFVGWVAGGLTLIGGVMMCIACR GLAPEETNYKAVSYHASGHSVAYKPGGFKASTGFGSNTKNKKIYDGGARTEDEVQSYPSKHDYV (SEQ ID NO:1) |
| Human CLDN18.1: MSTTTCQVVAFLLSILGLAGCIAATGMDMWSTQDLYDNPVTSVFQYEGLWRSCVRQSSGFTECRP YFTILGLPAMLQAVRALMIVGIVLGAIGLLVSIFALKCIRIGSMEDSAKANMTLTSGIMFIVSGLCAIA GVSVFANMLVTNFWMSTANMYTGMGGMVQTVQTRYTFGAALFVGWVAGGLTLIGGVMMCIAC RGLAPEETNYKAVSYHASGHSVAYKPGGFKASTGFGSNTKNKKIYDGGARTEDEVQSYPSKHDYV (SEQ ID NO:2) |
| HCDR1 of mouse, chimeric, and humanized 28G3 DYLML    (SEQ ID NO:3) |
| HCDR2 of mouse and chimeric 28G3 NINPYYGRTTYNLKFKG    (SEQ ID NO:4) |
| HCDR2 of hz28G3-1.1, hz28G3-1.3, hz28G3-2.1, and hz28G3-2.3 NINPYYGRTTYALKFKG    (SEQ ID NO:5) |
| HCDR2 of hz28G3-1.2, hz28G3-1.4, hz28G3-2.2, and hz28G3-2.4 NINPYYGRTYYALKFQG    (SEQ ID NO:6) |
| HCDR3 of mouse, chimeric, and humanized 28G3 DGYSLRNAMDY    (SEQ ID NO:7) |
| LCDR1 of mouse, chimeric, and humanized 28G3 KSSQSLFNSGNQKNYLA    (SEQ ID NO:8) |
| LCDR2 of mouse, chimeric, and humanized 28G3 GASTRES    (SEQ ID NO:9) |
| LCDR3 of mouse, chimeric, and humanized 28G3 QNAHSYPYT    (SEQ ID NO:10) |
| VH of mouse and chimeric 28G3: EIQLQQTGPELVKPGASVTISCKTSGYSFTDYLMLWVKQSHGKSLEWIGNINPYYGRTTYNLKFKG KATLTVDRSSSTAYMQLNSLTSEDSAVYYCARDGYSLRNAMDYWGQGTSVTVSS    (SEQ ID NO:11) |
| VH of hz28G3-1.1 and hz28G3-2.1: |

| |
|---|
| QVQLVQSGAEVKKPGASVKVSCKTSGYSFTDYLMLWVRQAPGQGLEWMGNINPYYGRTTYALKF KGRVTMTVDRSTSTVYMELSSLRSEDTAVYYCARDGYSLRNAMDYWGQGTLVTVSS (SEQ ID NO:12) |
| VH of hz28G3-1.2 and hz28G3-2.2: QVQLVQSGAEVKKPGASVKVSCKASGYTFTDYLMLWVRQAPGQGLEWMGNINPYYGRTYYALK FQGRVTMTVDRSTSTVYMELSSLRSEDTAVYYCARDGYSLRNAMDYWGQGTLVTVSS (SEQ ID NO:13) |
| VH of hz28G3-1.3 and hz28G3-2.3: QVQLVQSGAEVKKPGASVKVSCKTSGYSFTDYLMLWVRQAHGQSLEWMGNINPYYGRTTYALKF KGRVTMTVDRSTSTVYMELSSLRSEDTAVYYCARDGYSLRNAMDYWGQGTLVTVSS (SEQ ID NO:14) |
| VH of hz28G3-1.4 and hz28G3-2.4: QVQLVQSGAEVKKPGASVKVSCKASGYTFTDYLMLWVRQAHGQSLEWMGNINPYYGRTYYALK FQGRVTMTVDRSTSTVYMELSSLRSEDTAVYYCARDGYSLRNAMDYWGQGTLVTVSS (SEQ ID NO:15) |
| VL of mouse and chimeric 28G3: DIVMTQSPYSLSVSTGEKVTMSCKSSQSLFNSGNQKNYLAWYQQKPGQPPKLLIYGASTRESGVPD RFTGSGSGTDFTLTISSVQAEDLALYYCQNAHSYPYTFGGGTKLEIK (SEQ ID NO:16) |
| VL of hz28G3-1.1, hz28G3-1.2, hz28G3-1.3, and hz28G3-1.4: DIVMTQSPDSLAVSLGERATINCKSSQSLFNSGNQKNYLAWYQQKPGQPPKLLIYGASTRESGVPDR FSGSGSGTDFTLTISSLQAEDVAVYYCQNAHSYPYTFGQGTKVEIK (SEQ ID NO:17) |
| VL of hz28G3-2.1, hz28G3-2.2, hz28G3-2.3, and hz28G3-2.4: DIVMTQSPYSLAVSLGERATINCKSSQSLFNSGNQKNYLAWYQQKPGQPPKLLIYGASTRESGVPDR FTGSGSGTDFTLTISSLQAEDVAVYYCQNAHSYPYTFGQGTKVEIK (SEQ ID NO:18) |
| HCDR1 of mouse, chimeric, and humanized 40F6 SYGVY (SEQ ID NO:19) |
| HCDR2 of mouse and chimeric 40F6, hz40F6-1.2, and hz40F6-2.2 VVWAGGNTNYNSALMS (SEQ ID NO:20) |
| HCDR2 of hz40F6-1.1 and hz40F6-2.1 VVWAGGNTNYADSVKG (SEQ ID NO:21) |
| HCDR2 of hz40F6-1.3 and hz40F6-2.3 VVWAGGNTNYADALKG (SEQ ID NO:22) |
| HCDR2 of hz40F6-1.4 and hz40F6-2.4 VVWAGGNTNYNSALKG (SEQ ID NO:23) |
| HCDR3 of mouse, chimeric, and humanized 40F6 DRGRLLAMDY (SEQ ID NO:24) |
| LCDR1 of mouse, chimeric, and humanized 40F6 KSSQSLLNSGNQKNYLA (SEQ ID NO:25) |
| LCDR2 of mouse, chimeric, and humanized 40F6 GASTRES (SEQ ID NO:26) |
| LCDR3 of mouse and chimeric 40F6, hz40F6-1.1, hz40F6-1.2, hz40F6-1.3, and hz40F6-1.4 QNDHFFPFT (SEQ ID NO:27) |
| LCDR3 of hz40F6-2.1, hz40F6-2.2, hz40F6-2.3, and hz40F6-2.4 QNDYFFPFT (SEQ ID NO:28) |
| VH of mouse and chimeric 40F6: QVQLKESGPGLVAPSQSLSITCTVSGFSLTSYGVYWVRQSPGKGLEWLGVVWAGGNTNYNSALMS RLSISKDNSKSQVFLKMNSLQTDDTAMYYCARDRGRLLAMDYWGQGTSVTVSS (SEQ ID NO:29) |
| VH of hz40F6-1.1 and hz40F6-2.1: QVQLVESGGGVVQPGRSLRLSCAASGFTLTSYGVYWVRQAPGKGLEWVAVVWAGGNTNYADSV KGRFTISKDNSKSTLYLQMNSLRAEDTAVYYCARDRGRLLAMDYWGQGTLVTVSS (SEQ ID NO:30) |
| VH of hz40F6-1.2 and hz40F6-2.2: QVQLVESGGGVVQPGRSLRLSCAASGFTLTSYGVYWVRQAPGKGLEWVAVVWAGGNTNYNSAL MSRFTISKDNSKSTLYLQMNSLRAEDTAVYYCARDRGRLLAMDYWGQGTLVTVSS (SEQ ID NO:31) |
| VH of hz40F6-1.3 and hz40F6-2.3: QVQLVESGGGVVQPGRSLRLSCAASGFTLTSYGVYWVRQAPGKGLEWVAVVWAGGNTNYADAL KGRFTISKDNSKSTLYLQMNSLRAEDTAVYYCARDRGRLLAMDYWGQGTLVTVSS (SEQ ID |

| |
|---|
| NO:32) |
| VH of hz40F6-1.4 and hz40F6-2.4:<br>QVQLVESGGGVVQPGRSLRLSCTVSGFTLTSYGVYWVRQAPGKGLEWVAVVWAGGNTNYNSALK<br>GRFTISKDNSKSTLYLQMNSLRAEDTAVYYCARDRGRLLAMDYWGQGTLVTVSS   (SEQ ID<br>NO:33) |
| VL of mouse and chimeric 40F6:<br>DVVMTQSPSSLSVSAGEKVTMSCKSSQSLLNSGNQKNYLAWYQQKPGQPPKLLIYGASTRESGVP<br>DRFTGSGSGTDFTLTISSVQAEDLAVYYCQNDHFFPFTFGSGTKLEIK   (SEQ ID NO:34) |
| VL of hz40F6-1.1, hz40F6-1.2, hz40F6-1.3, and hz40F6-1.4:<br>DIVMTQSPDSLAVSLGERATINCKSSQSLLNSGNQKNYLAWYQQKPGQPPKLLIYGASTRESGVPD<br>RFTGSGSGTDFTLTISSLQAEDVAVYYCQNDHFFPFTFGQGTKVEIK   (SEQ ID NO:35) |
| VL of hz40F6-2.1, hz40F6-2.2, hz40F6-2.3, and hz40F6-2.4:<br>DIVMTQSPDSLAVSLGERATINCKSSQSLLNSGNQKNYLAWYQQKPGQPPKLLIYGASTRESGVPD<br>RFSGSGSGTDFTLTISSLQAEDVAVYYCQNDYFFPFTFGQGTKVEIK   (SEQ ID NO:36) |
| HCDR1 of mouse and chimeric 34G6<br>SYGVH   (SEQ ID NO:37) |
| HCDR2 of mouse and chimeric 34G6<br>VIWSDGSTTYNSALKS   (SEQ ID NO:38) |
| HCDR3 of mouse and chimeric 34G6<br>HAYYGNSFDY   (SEQ ID NO:39) |
| LCDR1 of mouse and chimeric 34G6<br>KSSQSLLNSGNQKSYLT   (SEQ ID NO:40) |
| LCDR2 of mouse and chimeric 34G6<br>WASTRES   (SEQ ID NO:41) |
| LCDR3 of mouse and chimeric 34G6<br>QNVYIFPLT   (SEQ ID NO:42) |
| VH of mouse and chimeric 34G6:<br>QVQLKESGPDLVAPSQSLSITCTVSGFSLTSYGVHWVRQPPGKGLEWLVVIWSDGSTTYNSALKSR<br>LSISKDNSKSQVFLKMNSLQTDDTAIYYCARHAYYGNSFDYWGQGTTLTVSS   (SEQ ID NO:43) |
| VL of mouse and chimeric 34G6:<br>DIVMTQSPSSLTVTAGEKVTMSCKSSQSLLNSGNQKSYLTWYQQKPGQPPKLLIYWASTRESGVPD<br>RFTGSGSGTDFTLTISSVQAEDLAVYYCQNVYIFPLTFGAGTKLELK   (SEQ ID NO:44) |
| HCDR1 of mouse and chimeric 10D8<br>NYGMN   (SEQ ID NO:45) |
| HCDR2 of mouse and chimeric 10D8<br>WININTGEPRYAEEFKG   (SEQ ID NO:46) |
| HCDR3 of mouse and chimeric 10D8<br>YGYGNSFPY   (SEQ ID NO:47) |
| LCDR1 of mouse and chimeric 10D8<br>KSSQSLLNSGNQRNYLT   (SEQ ID NO:48) |
| LCDR2 of mouse and chimeric 10D8<br>WASTRES   (SEQ ID NO:49) |
| LCDR3 of mouse and chimeric 10D8<br>QNDYSYPLT   (SEQ ID NO:50) |
| VH of mouse and chimeric 10D8:<br>QIQLAQSGPELKKPGETVKVSCKASGYTFKNYGMNWVKQAPGKGLKWMGWININTGEPRYAEEF<br>KGRIAFSLETSASTAYLLINNLKNEDTATYFCARYGYGNSFPYWGQGTLVTVSA   (SEQ ID NO:51) |
| VL of mouse and chimeric 10D8:<br>DIVMTQSPSSLTVTVGEKVTMSCKSSQSLLNSGNQRNYLTWYQQKPGQPPKLLIYWASTRESGVPD<br>RFTGSGSGTAFTLTVSSVQAEDLAVYYCQNDYSYPLTFGAGTKLEMK   (SEQ ID NO:52) |
| HCDR1 of mouse and chimeric 22F12<br>SYGVY   (SEQ ID NO:53) |
| HCDR2 of mouse and chimeric 22F12<br>VIWAGGSTNYNSALMS   (SEQ ID NO:54) |
| HCDR3 of mouse and chimeric 22F12<br>DRGRLLSMDY   (SEQ ID NO:55) |
| LCDR1 of mouse and chimeric 22F12<br>RSSQSLLNSGNQKNYLA   (SEQ ID NO:56) |
| LCDR2 of mouse and chimeric 22F12 |

| |
|---|
| GASTRES (SEQ ID NO:57) |
| LCDR3 of mouse and chimeric 22F12<br>QNVHFFPFT (SEQ ID NO:58) |
| VH of mouse and chimeric 22F12:<br>QVQLKESGPGLVAPSQSLSITCTVSGFSLTSYGVYWVRQPPGKGLEWLGVIWAGGSTNYNSALMSR<br>LSINKDNSKSQVFLKMNSLQTDDTAMYYCARDRGRLLSMDYWGQGTSVTVSS (SEQ ID NO:59) |
| VL of mouse and chimeric 22F12:<br>DIVMTQSPSSLSVSAGEKVTMSCRSSQSLLNSGNQKNYLAWYQQKPGQPPKLLIYGASTRESGVPD<br>RFTGSGSGTDFTLTIGSVQAEDLAVYYCQNVHFFPFTFGSGTKLEIK (SEQ ID NO:60) |
| Heavy chain constant region of chimeric and humanized antibodies<br>ASTKGPSVFPLAPSSKSTSGGTAALGCLVKDYFPEPVTVSWNSGALTSGVHTFPAVLQSSGLYSLSSV<br>VTVPSSSLGTQTYICNVNHKPSNTKVDKKVEPKSCDKTHTCPPCPAPELLGGPSVFLFPPKPKDTLM<br>ISRTPEVTCVVVDVSHEDPEVKFNWYVDGVEVHNAKTKPREEQYNSTYRVVSVLTVLHQDWLNG<br>KEYKCKVSNKALPAPIEKTISKAKGQPREPQVYTLPPSRDELTKNQVSLTCLVKGFYPSDIAVEWES<br>NGQPENNYKTTPPVLDSDGSFFLYSKLTVDKSRWQQGNVFSCSVMHEALHNHYTQKSLSLSPGK<br>(SEQ ID NO:61) |
| Light chain constant region of chimeric and humanized antibodies<br>RTVAAPSVFIFPPSDEQLKSGTASVVCLLNNFYPREAKVQWKVDNALQSGNSQESVTEQDSKDSTY<br>SLSSTLTLSKADYEKHKVYACEVTHQGLSSPVTKSFNRGEC (SEQ ID NO:62) |
| Heavy chain of IMAB362:<br>QVQLQQPGAELVRPGASVKLSCKASGYTFTSYWINWVKQRPGQGLEWIGNIYPSDSYTNYNQKFK<br>DKATLTVDKSSSTAYMQLSSPTSEDSAVYYCTRSWRGNSFDYWGQGTTLTVSSASTKGPSVFPLAPS<br>SKSTSGGTAALGCLVKDYFPEPVTVSWNSGALTSGVHTFPAVLQSSGLYSLSSVVTVPSSSLGTQTYI<br>CNVNHKPSNTKVDKKVEPKSCDKTHTCPPCPAPELLGGPSVFLFPPKPKDTLMISRTPEVTCVVVD<br>VSHEDPEVKFNWYVDGVEVHNAKTKPREEQYNSTYRVVSVLTVLHQDWLNGKEYKCKVSNKAL<br>PAPIEKTISKAKGQPREPQVYTLPPSRDELTKNQVSLTCLVKGFYPSDIAVEWESNGQPENNYKTTPP<br>VLDSDGSFFLYSKLTVDKSRWQQGNVFSCSVMHEALHNHYTQKSLSLSPGK (SEQ ID NO:63) |
| Light chain of IMAB362:<br>DIVMTQSPSSLTVTAGEKVTMSCKSSQSLLNSGNQKNYLTWYQQKPGQPPKLLIYWASTRESGVPD<br>RFTGSGSGTDFTLTISSVQAEDLAVYYCQNDYSYPFTFGSGTKLEIKRTVAAPSVFIFPPSDEQLKSGT<br>ASVVCLLNNFYPREAKVQWKVDNALQSGNSQESVTEQDSKDSTYSLSSTLTLSKADYEKHKVYAC<br>EVTHQGLSSPVTKSFNRGEC (SEQ ID NO:64) |
| Heavy chain of 15F9:<br>QVQLVQSGSELKKPGASVKVSCKASGYTFTNYGINWVRQAPGQGLEWMGWINPNTGETTYAEEF<br>KGRFVFSLDTSVSTAYLQISSLKAEDTAVYFCARLYYGNRFDSWGQGTLVTVSSASTKGPSVFPLAP<br>SSKSTSGGTAALGCLVKDYFPEPVTVSWNSGALTSGVHTFPAVLQSSGLYSLSSVVTVPSSSLGTQT<br>YICNVNHKPSNTKVDKKVEPKSCDKTHTCPPCPAPELLGGPSVFLFPPKPKDTLMISRTPEVTCVVV<br>DVSHEDPEVKFNWYVDGVEVHNAKTKPREEQYNSTYRVVSVLTVLHQDWLNGKEYKCKVSNKA<br>LPAPIEKTISKAKGQPREPQVYTLPPSRDELTKNQVSLTCLVKGFYPSDIAVEWESNGQPENNYKTTP<br>PVLDSDGSFFLYSKLTVDKSRWQQGNVFSCSVMHEALHNHYTQKSLSLSPGK (SEQ ID NO:65) |
| Light chain of 15F9:<br>DIVMTQSPDSLAVSLGERATINCKSSQSLLNSGNQRNYLTWYQQKPGQPPKLLIYWASTRESGVPDR<br>FSGSGSGTDFTLTISSLQAEDVAVYYCQNAYYYPLTFGGGTKVEIKRTVAAPSVFIFPPSDEQLKSGT<br>ASVVCLLNNFYPREAKVQWKVDNALQSGNSQESVTEQDSKDSTYSLSSTLTLSKADYEKHKVYAC<br>EVTHQGLSSPVTKSFNRGEC (SEQ ID NO:66) |
| Heavy chain of IgG1 isotype control antibody:<br>EVQLEQSGAELMKPGASVKISCKATGYTFTTYWIEWIKQRPGHSLEWIGEILPGSDSTYYNEKVKG<br>KVTFTADASSNTAYMQLSSLTSEDSAVYYCARGDGFYVYWGQGTTLTVSSASTKGPSVFPLAPSSK<br>STSGGTAALGCLVKDYFPEPVTVSWNSGALTSGVHTFPAVLQSSGLYSLSSVVTVPSSSLGTQTYICN<br>VNHKPSNTKVDKKVEPKSCDKTHTCPPCPAPELLGGPSVFLFPPKPKDTLMISRTPEVTCVVVDVSH<br>EDPEVKFNWYVDGVEVHNAKTKPREEQYNSTYRVVSVLTVLHQDWLNGKEYKCKVSNKALPAPI<br>EKTISKAKGQPREPQVYTLPPSRDELTKNQVSLTCLVKGFYPSDIAVEWESNGQPENNYKTTPPVLD<br>SDGSFFLYSKLTVDKSRWQQGNVFSCSVMHEALHNHYTQKSLSLSPGK (SEQ ID NO:67) |
| Light chain of IgG1 isotype control antibody:<br>DIELTQSPATLSVTPGDSVSLSCRASQSISNNLHWYQQKSHESPRLLIKYTSQSMSGIPSRFSGSGSGT<br>DFTLSINSVETEDFGVYFCQQSGSWPRTFGGGTKLDIKRTVAAPSVFIFPPSDEQLKSGTASVVCLLN<br>NFYPREAKVQWKVDNALQSGNSQESVTEQDSKDSTYSLSSTLTLSKADYEKHKVYACEVTHQGLS<br>SPVTKSFNRGEC (SEQ ID NO:68) |

All patents, patent disclosures and other identified publications are explicitly incorporated herein by reference for the purpose of description and disclosure. These publications are provided solely because they were disclosed prior to the filing date of the present disclosure. All statements as to the dates of these documents or descriptions as to the contents of these documents are based on the information available to the applicant and do not constitute any admission as to the correctness of the dates or the content of these documents. Moreover, in any country, any reference to these publications herein is not to be construed as an admission that the publications form part of the commonly recognized knowledge in the art.

Although the present disclosure has been described in detail herein above through general explanations and specific embodiments, it will be apparent to those skilled in the art that modifications or improvements can be made based on the present disclosure. Accordingly, such modifications and improvements made without departing from the spirit of the present disclosure all fall within the protection scope of the present disclosure.

**Claims**

1. An isolated anti-CLDN18.2 antibody or an antigen-binding fragment thereof, wherein the antibody or the antigen-binding fragment thereof comprises:

   (i) a heavy chain CDR1, a heavy chain CDR2, and a heavy chain CDR3, wherein

   (1) the heavy chain CDR1 comprises the heavy chain CDR1 amino acid sequence in SEQ ID NO: 11, the heavy chain CDR2 comprises the heavy chain CDR2 amino acid sequence in SEQ ID NO: 11, and the heavy chain CDR3 comprises the heavy chain CDR3 amino acid sequence in SEQ ID NO: 11;
   (2) the heavy chain CDR1 comprises the heavy chain CDR1 amino acid sequence in SEQ ID NO: 12, the heavy chain CDR2 comprises the heavy chain CDR2 amino acid sequence in SEQ ID NO: 12, and the heavy chain CDR3 comprises the heavy chain CDR3 amino acid sequence in SEQ ID NO: 12;
   (3) the heavy chain CDR1 comprises the heavy chain CDR1 amino acid sequence in SEQ ID NO: 13, the heavy chain CDR2 comprises the heavy chain CDR2 amino acid sequence in SEQ ID NO: 13, and the heavy chain CDR3 comprises the heavy chain CDR3 amino acid sequence in SEQ ID NO: 13;
   (4) the heavy chain CDR1 comprises the heavy chain CDR1 amino acid sequence in SEQ ID NO: 14, the heavy chain CDR2 comprises the heavy chain CDR2 amino acid sequence in SEQ ID NO: 14, and the heavy chain CDR3 comprises the heavy chain CDR3 amino acid sequence in SEQ ID NO: 14;
   (5) the heavy chain CDR1 comprises the heavy chain CDR1 amino acid sequence in SEQ ID NO: 15, the heavy chain CDR2 comprises the heavy chain CDR2 amino acid sequence in SEQ ID NO: 15, and the heavy chain CDR3 comprises the heavy chain CDR3 amino acid sequence in SEQ ID NO: 15;
   (6) the heavy chain CDR1 comprises the heavy chain CDR1 amino acid sequence in SEQ ID NO: 29, the heavy chain CDR2 comprises the heavy chain CDR2 amino acid sequence in SEQ ID NO: 29, and the heavy chain CDR3 comprises the heavy chain CDR3 amino acid sequence in SEQ ID NO: 29;
   (7) the heavy chain CDR1 comprises the heavy chain CDR1 amino acid sequence in SEQ ID NO: 30, the heavy chain CDR2 comprises the heavy chain CDR2 amino acid sequence in SEQ ID NO: 30, and the heavy chain CDR3 comprises the heavy chain CDR3 amino acid sequence in SEQ ID NO: 30;
   (8) the heavy chain CDR1 comprises the heavy chain CDR1 amino acid sequence in SEQ ID NO: 31, the heavy chain CDR2 comprises the heavy chain CDR2 amino acid sequence in SEQ ID NO: 31, and the heavy chain CDR3 comprises the heavy chain CDR3 amino acid sequence in SEQ ID NO: 31;
   (9) the heavy chain CDR1 comprises the heavy chain CDR1 amino acid sequence in SEQ ID NO: 32, the heavy chain CDR2 comprises the heavy chain CDR2 amino acid sequence in SEQ ID NO: 32, and the heavy chain CDR3 comprises the heavy chain CDR3 amino acid sequence in SEQ ID NO: 32;
   (10) the heavy chain CDR1 comprises the heavy chain CDR1 amino acid sequence in SEQ ID NO: 33, the heavy chain CDR2 comprises the heavy chain CDR2 amino acid sequence in SEQ ID NO: 33, and the heavy chain CDR3 comprises the heavy chain CDR3 amino acid sequence in SEQ ID NO: 33;
   (11) the heavy chain CDR1 comprises the heavy chain CDR1 amino acid sequence in SEQ ID NO: 43, the heavy chain CDR2 comprises the heavy chain CDR2 amino acid sequence in SEQ ID NO: 43, and the heavy chain CDR3 comprises the heavy chain CDR3 amino acid sequence in SEQ ID NO: 43;
   (12) the heavy chain CDR1 comprises the heavy chain CDR1 amino acid sequence in SEQ ID NO: 51, the heavy chain CDR2 comprises the heavy chain CDR2 amino acid sequence in SEQ ID NO: 51, and the heavy chain CDR3 comprises the heavy chain CDR3 amino acid sequence in SEQ ID NO: 51; or
   (13) the heavy chain CDR1 comprises the heavy chain CDR1 amino acid sequence in SEQ ID NO: 59, the heavy chain CDR2 comprises the heavy chain CDR2 amino acid sequence in SEQ ID NO: 59, and the heavy chain CDR3 comprises the heavy chain CDR3 amino acid sequence in SEQ ID NO: 59; and/or

(ii) a light chain CDR1, a light chain CDR2, and a light chain CDR3, wherein

(1) the light chain CDR1 comprises the light chain CDR1 amino acid sequence in SEQ ID NO: 16, the light chain CDR2 comprises the light chain CDR2 amino acid sequence in SEQ ID NO: 16, and the light chain CDR3 comprises the light chain CDR3 amino acid sequence in SEQ ID NO: 16;
(2) the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;
(3) the light chain CDR1 comprises the light chain CDR1 amino acid sequence in SEQ ID NO: 18, the light chain CDR2 comprises the light chain CDR2 amino acid sequence in SEQ ID NO: 18, and the light chain CDR3 comprises the light chain CDR3 amino acid sequence in SEQ ID NO: 18;
(4) the light chain CDR1 comprises the light chain CDR1 amino acid sequence in SEQ ID NO: 34, the light chain CDR2 comprises the light chain CDR2 amino acid sequence in SEQ ID NO: 34, and the light chain CDR3 comprises the light chain CDR3 amino acid sequence in SEQ ID NO: 34;
(5) the light chain CDR1 comprises the light chain CDR1 amino acid sequence in SEQ ID NO: 35, the light chain CDR2 comprises the light chain CDR2 amino acid sequence in SEQ ID NO: 35, and the light chain CDR3 comprises the light chain CDR3 amino acid sequence in SEQ ID NO: 35;
(6) the light chain CDR1 comprises the light chain CDR1 amino acid sequence in SEQ ID NO: 36, the light chain CDR2 comprises the light chain CDR2 amino acid sequence in SEQ ID NO: 36, and the light chain CDR3 comprises the light chain CDR3 amino acid sequence in SEQ ID NO: 36;
(7) the light chain CDR1 comprises the light chain CDR1 amino acid sequence in SEQ ID NO: 44, the light chain CDR2 comprises the light chain CDR2 amino acid sequence in SEQ ID NO: 44, and the light chain CDR3 comprises the light chain CDR3 amino acid sequence in SEQ ID NO: 44;
(8) the light chain CDR1 comprises the light chain CDR1 amino acid sequence in SEQ ID NO: 52, the light chain CDR2 comprises the light chain CDR2 amino acid sequence in SEQ ID NO: 52, and the light chain CDR3 comprises the light chain CDR3 amino acid sequence in SEQ ID NO: 52; or
(9) the light chain CDR1 comprises the light chain CDR1 amino acid sequence in SEQ ID NO: 60, the light chain CDR2 comprises the light chain CDR2 amino acid sequence in SEQ ID NO: 60, and the light chain CDR3 comprises the light chain CDR3 amino acid sequence in SEQ ID NO: 60.

2. The antibody or the antigen-binding fragment thereof according to claim 1, wherein the antibody or the antigen-binding fragment thereof comprises:

(i) a heavy chain CDR1, a heavy chain CDR2, and a heavy chain CDR3, wherein

(1) the heavy chain CDR1, the heavy chain CDR2, and the heavy chain CDR3 comprise the amino acid sequences set forth in SEQ ID NOs: 3, 4, and 7, or amino acid sequences having at least 80% identity to the amino acid sequences set forth in SEQ ID NOs: 3, 4, and 7, respectively;
(2) the heavy chain CDR1, the heavy chain CDR2, and the heavy chain CDR3 comprise the amino acid sequences set forth in SEQ ID NOs: 3, 5, and 7, or amino acid sequences having at least 80% identity to the amino acid sequences set forth in SEQ ID NOs: 3, 5, and 7, respectively;
(3) the heavy chain CDR1, the heavy chain CDR2, and the heavy chain CDR3 comprise the amino acid sequences set forth in SEQ ID NOs: 3, 6, and 7, or amino acid sequences having at least 80% identity to the amino acid sequences set forth in SEQ ID NOs: 3, 6, and 7, respectively;
(4) the heavy chain CDR1, the heavy chain CDR2, and the heavy chain CDR3 comprise the amino acid sequences set forth in SEQ ID NOs: 19, 20, and 24, or amino acid sequences having at least 80% identity to the amino acid sequences set forth in SEQ ID NOs: 19, 20, and 24, respectively;
(5) the heavy chain CDR1, the heavy chain CDR2, and the heavy chain CDR3 comprise the amino acid sequences set forth in SEQ ID NOs: 19, 21, and 24, or amino acid sequences having at least 80% identity to the amino acid sequences set forth in SEQ ID NOs: 19, 21, and 24, respectively;
(6) the heavy chain CDR1, the heavy chain CDR2, and the heavy chain CDR3 comprise the amino acid sequences set forth in SEQ ID NOs: 19, 22, and 24, or amino acid sequences having at least 80% identity to the amino acid sequences set forth in SEQ ID NOs: 19, 22, and 24, respectively;
(7) the heavy chain CDR1, the heavy chain CDR2, and the heavy chain CDR3 comprise the amino acid sequences set forth in SEQ ID NOs: 19, 23, and 24, or amino acid sequences having at least 80% identity to the amino acid sequences set forth in SEQ ID NOs: 19, 23, and 24, respectively;

(8) the heavy chain CDR1, the heavy chain CDR2, and the heavy chain CDR3 comprise the amino acid sequences set forth in SEQ ID NOs: 37, 38, and 39, or amino acid sequences having at least 80% identity to the amino acid sequences set forth in SEQ ID NOs: 37, 38, and 39, respectively;

(9) the heavy chain CDR1, the heavy chain CDR2, and the heavy chain CDR3 comprise the amino acid sequences set forth in SEQ ID NOs: 45, 46, and 47, or amino acid sequences having at least 80% identity to the amino acid sequences set forth in SEQ ID NOs: 45, 46, and 47, respectively; or

(10) the heavy chain CDR1, the heavy chain CDR2, and the heavy chain CDR3 comprise the amino acid sequences set forth in SEQ ID NOs: 53, 54, and 55, or amino acid sequences having at least 80% identity to the amino acid sequences set forth in SEQ ID NOs: 53, 54, and 55, respectively; and/or

(ii) a light chain CDR1, a light chain CDR2, and a light chain CDR3, wherein

(1) the light chain CDR1, the light chain CDR2, and the light chain CDR3 comprise the amino acid sequences set forth in SEQ ID NOs: 8, 9, and 10, or amino acid sequences having at least 80% identity to the amino acid sequences set forth in SEQ ID NOs: 8, 9, and 10, respectively;

(2) the light chain CDR1, the light chain CDR2, and the light chain CDR3 comprise the amino acid sequences set forth in SEQ ID NOs: 25, 26, and 27, or amino acid sequences having at least 80% identity to the amino acid sequences set forth in SEQ ID NOs: 25, 26, and 27, respectively;

(3) the light chain CDR1, the light chain CDR2, and the light chain CDR3 comprise the amino acid sequences set forth in SEQ ID NOs: 25, 26, and 28, or amino acid sequences having at least 80% identity to the amino acid sequences set forth in SEQ ID NOs: 25, 26, and 28, respectively;

(4) the light chain CDR1, the light chain CDR2, and the light chain CDR3 comprise the amino acid sequences set forth in SEQ ID NOs: 40, 41, and 42, or amino acid sequences having at least 80% identity to the amino acid sequences set forth in SEQ ID NOs: 40, 41, and 42, respectively;

(5) the light chain CDR1, the light chain CDR2, and the light chain CDR3 comprise the amino acid sequences set forth in SEQ ID NOs: 48, 49, and 50, or amino acid sequences having at least 80% identity to the amino acid sequences set forth in SEQ ID NOs: 48, 49, and 50, respectively; or

(6) the light chain CDR1, the light chain CDR2, and the light chain CDR3 comprise the amino acid sequences set forth in SEQ ID NOs: 56, 57, and 58, or amino acid sequences having at least 80% identity to the amino acid sequences set forth in SEQ ID NOs: 56, 57, and 58, respectively.

3. The antibody or the antigen-binding fragment thereof according to claim 1 or 2, wherein the antibody or the antigen-binding fragment thereof comprises a heavy chain CDR1, a heavy chain CDR2, a heavy chain CDR3, a light chain CDR1, a light chain CDR2, and a light chain CDR3, wherein

(1) the heavy chain CDR1 comprises the heavy chain CDR1 amino acid sequence in SEQ ID NO: 11, the heavy chain CDR2 comprises the heavy chain CDR2 amino acid sequence in SEQ ID NO: 11, the heavy chain CDR3 comprises the heavy chain CDR3 amino acid sequence in SEQ ID NO: 11, the light chain CDR1 comprises the light chain CDR1 amino acid sequence in SEQ ID NO: 16, the light chain CDR2 comprises the light chain CDR2 amino acid sequence in SEQ ID NO: 16, and the light chain CDR3 comprises the light chain CDR3 amino acid sequence in SEQ ID NO: 16;

(2) the heavy chain CDR1 comprises the heavy chain CDR1 amino acid sequence in SEQ ID NO: 12, the heavy chain CDR2 comprises the heavy chain CDR2 amino acid sequence in SEQ ID NO: 12, the heavy chain CDR3 comprises the heavy chain CDR3 amino acid sequence in SEQ ID NO: 12, the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;

(3) the heavy chain CDR1 comprises the heavy chain CDR1 amino acid sequence in SEQ ID NO: 13, the heavy chain CDR2 comprises the heavy chain CDR2 amino acid sequence in SEQ ID NO: 13, the heavy chain CDR3 comprises the heavy chain CDR3 amino acid sequence in SEQ ID NO: 13, the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;

(4) the heavy chain CDR1 comprises the heavy chain CDR1 amino acid sequence in SEQ ID NO: 14, the heavy chain CDR2 comprises the heavy chain CDR2 amino acid sequence in SEQ ID NO: 14, the heavy chain CDR3 comprises the heavy chain CDR3 amino acid sequence in SEQ ID NO: 14, the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;

(5) the heavy chain CDR1 comprises the heavy chain CDR1 amino acid sequence in SEQ ID NO: 15, the heavy chain CDR2 comprises the heavy chain CDR2 amino acid sequence in SEQ ID NO: 15, the heavy chain CDR3 comprises the heavy chain CDR3 amino acid sequence in SEQ ID NO: 15, the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;

(6) the heavy chain CDR1 comprises the heavy chain CDR1 amino acid sequence in SEQ ID NO: 12, the heavy chain CDR2 comprises the heavy chain CDR2 amino acid sequence in SEQ ID NO: 12, the heavy chain CDR3 comprises the heavy chain CDR3 amino acid sequence in SEQ ID NO: 12, the light chain CDR1 comprises the light chain CDR1 amino acid sequence in SEQ ID NO: 18, the light chain CDR2 comprises the light chain CDR2 amino acid sequence in SEQ ID NO: 18, and the light chain CDR3 comprises the light chain CDR3 amino acid sequence in SEQ ID NO: 18;

(7) the heavy chain CDR1 comprises the heavy chain CDR1 amino acid sequence in SEQ ID NO: 13, the heavy chain CDR2 comprises the heavy chain CDR2 amino acid sequence in SEQ ID NO: 13, the heavy chain CDR3 comprises the heavy chain CDR3 amino acid sequence in SEQ ID NO: 13, the light chain CDR1 comprises the light chain CDR1 amino acid sequence in SEQ ID NO: 18, the light chain CDR2 comprises the light chain CDR2 amino acid sequence in SEQ ID NO: 18, and the light chain CDR3 comprises the light chain CDR3 amino acid sequence in SEQ ID NO: 18;

(8) the heavy chain CDR1 comprises the heavy chain CDR1 amino acid sequence in SEQ ID NO: 14, the heavy chain CDR2 comprises the heavy chain CDR2 amino acid sequence in SEQ ID NO: 14, the heavy chain CDR3 comprises the heavy chain CDR3 amino acid sequence in SEQ ID NO: 14, the light chain CDR1 comprises the light chain CDR1 amino acid sequence in SEQ ID NO: 18, the light chain CDR2 comprises the light chain CDR2 amino acid sequence in SEQ ID NO: 18, and the light chain CDR3 comprises the light chain CDR3 amino acid sequence in SEQ ID NO: 18;

(9) the heavy chain CDR1 comprises the heavy chain CDR1 amino acid sequence in SEQ ID NO: 15, the heavy chain CDR2 comprises the heavy chain CDR2 amino acid sequence in SEQ ID NO: 15, the heavy chain CDR3 comprises the heavy chain CDR3 amino acid sequence in SEQ ID NO: 15, the light chain CDR1 comprises the light chain CDR1 amino acid sequence in SEQ ID NO: 18, the light chain CDR2 comprises the light chain CDR2 amino acid sequence in SEQ ID NO: 18, and the light chain CDR3 comprises the light chain CDR3 amino acid sequence in SEQ ID NO: 18;

(10) the heavy chain CDR1 comprises the heavy chain CDR1 amino acid sequence in SEQ ID NO: 29, the heavy chain CDR2 comprises the heavy chain CDR2 amino acid sequence in SEQ ID NO: 29, the heavy chain CDR3 comprises the heavy chain CDR3 amino acid sequence in SEQ ID NO: 29, the light chain CDR1 comprises the light chain CDR1 amino acid sequence in SEQ ID NO: 34, the light chain CDR2 comprises the light chain CDR2 amino acid sequence in SEQ ID NO: 34, and the light chain CDR3 comprises the light chain CDR3 amino acid sequence in SEQ ID NO: 34;

(11) the heavy chain CDR1 comprises the heavy chain CDR1 amino acid sequence in SEQ ID NO: 30, the heavy chain CDR2 comprises the heavy chain CDR2 amino acid sequence in SEQ ID NO: 30, the heavy chain CDR3 comprises the heavy chain CDR3 amino acid sequence in SEQ ID NO: 30, the light chain CDR1 comprises the light chain CDR1 amino acid sequence in SEQ ID NO: 35, the light chain CDR2 comprises the light chain CDR2 amino acid sequence in SEQ ID NO: 35, and the light chain CDR3 comprises the light chain CDR3 amino acid sequence in SEQ ID NO: 35;

(12) the heavy chain CDR1 comprises the heavy chain CDR1 amino acid sequence in SEQ ID NO: 31, the heavy chain CDR2 comprises the heavy chain CDR2 amino acid sequence in SEQ ID NO: 31, the heavy chain CDR3 comprises the heavy chain CDR3 amino acid sequence in SEQ ID NO: 31, the light chain CDR1 comprises the light chain CDR1 amino acid sequence in SEQ ID NO: 35, the light chain CDR2 comprises the light chain CDR2 amino acid sequence in SEQ ID NO: 35, and the light chain CDR3 comprises the light chain CDR3 amino acid sequence in SEQ ID NO: 35;

(13) the heavy chain CDR1 comprises the heavy chain CDR1 amino acid sequence in SEQ ID NO: 32, the heavy chain CDR2 comprises the heavy chain CDR2 amino acid sequence in SEQ ID NO: 32, the heavy chain CDR3 comprises the heavy chain CDR3 amino acid sequence in SEQ ID NO: 32, the light chain CDR1 comprises the light chain CDR1 amino acid sequence in SEQ ID NO: 35, the light chain CDR2 comprises the light chain CDR2 amino acid sequence in SEQ ID NO: 35, and the light chain CDR3 comprises the light chain CDR3 amino acid sequence in SEQ ID NO: 35;

(14) the heavy chain CDR1 comprises the heavy chain CDR1 amino acid sequence in SEQ ID NO: 33, the heavy chain CDR2 comprises the heavy chain CDR2 amino acid sequence in SEQ ID NO: 33, the heavy chain CDR3 comprises the heavy chain CDR3 amino acid sequence in SEQ ID NO: 33, the light chain CDR1 comprises the light chain CDR1 amino acid sequence in SEQ ID NO: 35, the light chain CDR2 comprises the light chain CDR2 amino acid sequence in SEQ ID NO: 35, and the light chain CDR3 comprises the light chain CDR3 amino acid sequence in SEQ ID NO: 35;

(15) the heavy chain CDR1 comprises the heavy chain CDR1 amino acid sequence in SEQ ID NO: 30, the heavy chain CDR2 comprises the heavy chain CDR2 amino acid sequence in SEQ ID NO: 30, the heavy chain CDR3 comprises the heavy chain CDR3 amino acid sequence in SEQ ID NO: 30, the light chain CDR1 comprises the light chain CDR1 amino acid sequence in SEQ ID NO: 36, the light chain CDR2 comprises the light chain CDR2 amino acid sequence in SEQ ID NO: 36, and the light chain CDR3 comprises the light chain CDR3 amino acid sequence in SEQ ID NO: 36;

(16) the heavy chain CDR1 comprises the heavy chain CDR1 amino acid sequence in SEQ ID NO: 31, the heavy chain CDR2 comprises the heavy chain CDR2 amino acid sequence in SEQ ID NO: 31, the heavy chain CDR3 comprises the heavy chain CDR3 amino acid sequence in SEQ ID NO: 31, the light chain CDR1 comprises the light chain CDR1 amino acid sequence in SEQ ID NO: 36, the light chain CDR2 comprises the light chain CDR2 amino acid sequence in SEQ ID NO: 36, and the light chain CDR3 comprises the light chain CDR3 amino acid sequence in SEQ ID NO: 36;

(17) the heavy chain CDR1 comprises the heavy chain CDR1 amino acid sequence in SEQ ID NO: 32, the heavy chain CDR2 comprises the heavy chain CDR2 amino acid sequence in SEQ ID NO: 32, the heavy chain CDR3 comprises the heavy chain CDR3 amino acid sequence in SEQ ID NO: 32, the light chain CDR1 comprises the light chain CDR1 amino acid sequence in SEQ ID NO: 36, the light chain CDR2 comprises the light chain CDR2 amino acid sequence in SEQ ID NO: 36, and the light chain CDR3 comprises the light chain CDR3 amino acid sequence in SEQ ID NO: 36;

(18) the heavy chain CDR1 comprises the heavy chain CDR1 amino acid sequence in SEQ ID NO: 33, the heavy chain CDR2 comprises the heavy chain CDR2 amino acid sequence in SEQ ID NO: 33, the heavy chain CDR3 comprises the heavy chain CDR3 amino acid sequence in SEQ ID NO: 33, the light chain CDR1 comprises the light chain CDR1 amino acid sequence in SEQ ID NO: 36, the light chain CDR2 comprises the light chain CDR2 amino acid sequence in SEQ ID NO: 36, and the light chain CDR3 comprises the light chain CDR3 amino acid sequence in SEQ ID NO: 36;

(19) the heavy chain CDR1 comprises the heavy chain CDR1 amino acid sequence in SEQ ID NO: 43, the heavy chain CDR2 comprises the heavy chain CDR2 amino acid sequence in SEQ ID NO: 43, the heavy chain CDR3 comprises the heavy chain CDR3 amino acid sequence in SEQ ID NO: 43, the light chain CDR1 comprises the light chain CDR1 amino acid sequence in SEQ ID NO: 44, the light chain CDR2 comprises the light chain CDR2 amino acid sequence in SEQ ID NO: 44, and the light chain CDR3 comprises the light chain CDR3 amino acid sequence in SEQ ID NO: 44;

(20) the heavy chain CDR1 comprises the heavy chain CDR1 amino acid sequence in SEQ ID NO: 51, the heavy chain CDR2 comprises the heavy chain CDR2 amino acid sequence in SEQ ID NO: 51, the heavy chain CDR3 comprises the heavy chain CDR3 amino acid sequence in SEQ ID NO: 51, the light chain CDR1 comprises the light chain CDR1 amino acid sequence in SEQ ID NO: 52, the light chain CDR2 comprises the light chain CDR2 amino acid sequence in SEQ ID NO: 52, and the light chain CDR3 comprises the light chain CDR3 amino acid sequence in SEQ ID NO: 52; or

(21) the heavy chain CDR1 comprises the heavy chain CDR1 amino acid sequence in SEQ ID NO: 59, the heavy chain CDR2 comprises the heavy chain CDR2 amino acid sequence in SEQ ID NO: 59, the heavy chain CDR3 comprises the heavy chain CDR3 amino acid sequence in SEQ ID NO: 59, the light chain CDR1 comprises the light chain CDR1 amino acid sequence in SEQ ID NO: 60, the light chain CDR2 comprises the light chain CDR2 amino acid sequence in SEQ ID NO: 60, and the light chain CDR3 comprises the light chain CDR3 amino acid sequence in SEQ ID NO: 60; or

the antibody or the antigen-binding fragment thereof comprises a heavy chain CDR1, a heavy chain CDR2, a heavy chain CDR3, a light chain CDR1, a light chain CDR2, and a light chain CDR3, wherein

(1) the heavy chain CDR1, the heavy chain CDR2, the heavy chain CDR3, the light chain CDR1, the light chain CDR2, and the light chain CDR3 comprise the amino acid sequences set forth in SEQ ID NOs: 3, 4, 7, 8, 9, and 10, or amino acid sequences having at least 80% identity to the amino acid sequences set forth in SEQ ID NOs: 3, 4, 7, 8, 9, and 10, respectively;

(2) the heavy chain CDR1, the heavy chain CDR2, the heavy chain CDR3, the light chain CDR1, the light chain CDR2, and the light chain CDR3 comprise the amino acid sequences set forth in SEQ ID NOs: 3, 5, 7, 8, 9, and 10, or amino acid sequences having at least 80% identity to the amino acid sequences set forth in SEQ ID NOs: 3, 5, 7, 8, 9, and 10, respectively;

(3) the heavy chain CDR1, the heavy chain CDR2, the heavy chain CDR3, the light chain CDR1, the light chain CDR2, and the light chain CDR3 comprise the amino acid sequences set forth in SEQ ID NOs: 3, 6, 7, 8, 9, and 10, or amino acid sequences having at least 80% identity to the amino acid sequences set forth in SEQ ID NOs: 3, 6, 7, 8, 9, and 10, respectively;

(4) the heavy chain CDR1, the heavy chain CDR2, the heavy chain CDR3, the light chain CDR1, the light chain CDR2, and the light chain CDR3 comprise the amino acid sequences set forth in SEQ ID NOs: 19, 20, 24, 25, 26, and 27, or amino acid sequences having at least 80% identity to the amino acid sequences set forth in SEQ ID NOs: 19, 20, 24, 25, 26, and 27, respectively;

(5) the heavy chain CDR1, the heavy chain CDR2, the heavy chain CDR3, the light chain CDR1, the light chain CDR2, and the light chain CDR3 comprise the amino acid sequences set forth in SEQ ID NOs: 19, 21, 24, 25, 26, and 27, or amino acid sequences having at least 80% identity to the amino acid sequences set forth in SEQ ID NOs: 19, 21, 24, 25, 26, and 27, respectively;

(6) the heavy chain CDR1, the heavy chain CDR2, the heavy chain CDR3, the light chain CDR1, the light chain CDR2, and the light chain CDR3 comprise the amino acid sequences set forth in SEQ ID NOs: 19, 22, 24, 25, 26, and 27, or amino acid sequences having at least 80% identity to the amino acid sequences set forth in SEQ ID NOs: 19, 22, 24, 25, 26, and 27, respectively;

(7) the heavy chain CDR1, the heavy chain CDR2, the heavy chain CDR3, the light chain CDR1, the light chain CDR2, and the light chain CDR3 comprise the amino acid sequences set forth in SEQ ID NOs: 19, 23, 24, 25, 26, and 27, or amino acid sequences having at least 80% identity to the amino acid sequences set forth in SEQ ID NOs: 19, 23, 24, 25, 26, and 27, respectively;

(8) the heavy chain CDR1, the heavy chain CDR2, the heavy chain CDR3, the light chain CDR1, the light chain CDR2, and the light chain CDR3 comprise the amino acid sequences set forth in SEQ ID NOs: 19, 20, 24, 25, 26, and 28, or amino acid sequences having at least 80% identity to the amino acid sequences set forth in SEQ ID NOs: 19, 20, 24, 25, 26, and 28, respectively;

(9) the heavy chain CDR1, the heavy chain CDR2, the heavy chain CDR3, the light chain CDR1, the light chain CDR2, and the light chain CDR3 comprise the amino acid sequences set forth in SEQ ID NOs: 19, 21, 24, 25, 26, and 28, or amino acid sequences having at least 80% identity to the amino acid sequences set forth in SEQ ID NOs: 19, 21, 24, 25, 26, and 28, respectively;

(10) the heavy chain CDR1, the heavy chain CDR2, the heavy chain CDR3, the light chain CDR1, the light chain CDR2, and the light chain CDR3 comprise the amino acid sequences set forth in SEQ ID NOs: 19, 22, 24, 25, 26, and 28, or amino acid sequences having at least 80% identity to the amino acid sequences set forth in SEQ ID NOs: 19, 22, 24, 25, 26, and 28, respectively;

(11) the heavy chain CDR1, the heavy chain CDR2, the heavy chain CDR3, the light chain CDR1, the light chain CDR2, and the light chain CDR3 comprise the amino acid sequences set forth in SEQ ID NOs: 19, 23, 24, 25, 26, and 28, or amino acid sequences having at least 80% identity to the amino acid sequences set forth in SEQ ID NOs: 19, 23, 24, 25, 26, and 28, respectively;

(12) the heavy chain CDR1, the heavy chain CDR2, the heavy chain CDR3, the light chain CDR1, the light chain CDR2, and the light chain CDR3 comprise the amino acid sequences set forth in SEQ ID NOs: 37, 38, 39, 40, 41, and 42, or amino acid sequences having at least 80% identity to the amino acid sequences set forth in SEQ ID NOs: 37, 38, 39, 40, 41, and 42, respectively;

(13) the heavy chain CDR1, the heavy chain CDR2, the heavy chain CDR3, the light chain CDR1, the light chain CDR2, and the light chain CDR3 comprise the amino acid sequences set forth in SEQ ID NOs: 45, 46, 47, 48, 49, and 50, or amino acid sequences having at least 80% identity to the amino acid sequences set forth in SEQ ID NOs: 45, 46, 47, 48, 49, and 50, respectively; or

(14) the heavy chain CDR1, the heavy chain CDR2, the heavy chain CDR3, the light chain CDR1, the light chain CDR2, and the light chain CDR3 comprise the amino acid sequences set forth in SEQ ID NOs: 53, 54, 55, 56, 57, and 58, or amino acid sequences having at least 80% identity to the amino acid sequences set forth in SEQ ID NOs: 53, 54, 55, 56, 57, and 58, respectively.

4. The antibody or the antigen-binding fragment thereof according to any one of claims 1-3, wherein the antibody or the antigen-binding fragment thereof is chimeric or humanized.

5. The antibody or the antigen-binding fragment thereof according to any one of claims 1-4, wherein the antibody or the antigen-binding fragment thereof comprises:

(i) a heavy chain variable region comprising the amino acid sequence set forth in SEQ ID NO: 11, 12, 13, 14, 15, 29, 30, 31, 32, 33, 43, 51, or 59, or an amino acid sequence having at least 80% identity to the amino acid sequence set forth in SEQ ID NO: 11, 12, 13, 14, 15, 29, 30, 31, 32, 33, 43, 51, or 59; and/or
(ii) a light chain variable region comprising the amino acid sequence set forth in SEQ ID NO: 16, 17, 18, 34, 35, 36, 44, 52, or 60, or an amino acid sequence having at least 80% identity to the amino acid sequence set forth in SEQ ID NO: 16, 17, 18, 34, 35, 36, 44, 52, or 60.

6. The antibody or the antigen-binding fragment thereof according to any one of claims 1-5, wherein the antibody or the antigen-binding fragment thereof comprises a heavy chain variable region and a light chain variable region, wherein

(1) the heavy chain variable region and the light chain variable region comprise the amino acid sequences set forth in SEQ ID NOs: 11 and 16, or amino acid sequences having at least 80% identity to the amino acid sequences set forth in SEQ ID NOs: 11 and 16, respectively;
(2) the heavy chain variable region and the light chain variable region comprise the amino acid sequences set forth in SEQ ID NOs: 12 and 17, or amino acid sequences having at least 80% identity to the amino acid sequences set forth in SEQ ID NOs: 12 and 17, respectively;
(3) the heavy chain variable region and the light chain variable region comprise the amino acid sequences set forth in SEQ ID NOs: 13 and 17, or amino acid sequences having at least 80% identity to the amino acid sequences set forth in SEQ ID NOs: 13 and 17, respectively;
(4) the heavy chain variable region and the light chain variable region comprise the amino acid sequences set forth in SEQ ID NOs: 14 and 17, or amino acid sequences having at least 80% identity to the amino acid sequences set forth in SEQ ID NOs: 14 and 17, respectively;
(5) the heavy chain variable region and the light chain variable region comprise the amino acid sequences set forth in SEQ ID NOs: 15 and 17, or amino acid sequences having at least 80% identity to the amino acid sequences set forth in SEQ ID NOs: 15 and 17, respectively;
(6) the heavy chain variable region and the light chain variable region comprise the amino acid sequences set forth in SEQ ID NOs: 12 and 18, or amino acid sequences having at least 80% identity to the amino acid sequences set forth in SEQ ID NOs: 12 and 18, respectively;
(7) the heavy chain variable region and the light chain variable region comprise the amino acid sequences set forth in SEQ ID NOs: 13 and 18, or amino acid sequences having at least 80% identity to the amino acid sequences set forth in SEQ ID NOs: 13 and 18, respectively;
(8) the heavy chain variable region and the light chain variable region comprise the amino acid sequences set forth in SEQ ID NOs: 14 and 18, or amino acid sequences having at least 80% identity to the amino acid sequences set forth in SEQ ID NOs: 14 and 18, respectively;
(9) the heavy chain variable region and the light chain variable region comprise the amino acid sequences set forth in SEQ ID NOs: 15 and 18, or amino acid sequences having at least 80% identity to the amino acid sequences set forth in SEQ ID NOs: 15 and 18, respectively;
(10) the heavy chain variable region and the light chain variable region comprise the amino acid sequences set forth in SEQ ID NOs: 29 and 34, or amino acid sequences having at least 80% identity to the amino acid sequences set forth in SEQ ID NOs: 29 and 34, respectively;
(11) the heavy chain variable region and the light chain variable region comprise the amino acid sequences set forth in SEQ ID NOs: 30 and 35, or amino acid sequences having at least 80% identity to the amino acid sequences set forth in SEQ ID NOs: 30 and 35, respectively;
(12) the heavy chain variable region and the light chain variable region comprise the amino acid sequences set forth in SEQ ID NOs: 31 and 35, or amino acid sequences having at least 80% identity to the amino acid sequences set forth in SEQ ID NOs: 31 and 35, respectively;
(13) the heavy chain variable region and the light chain variable region comprise the amino acid sequences set forth in SEQ ID NOs: 32 and 35, or amino acid sequences having at least 80% identity to the amino acid sequences set forth in SEQ ID NOs: 32 and 35, respectively;
(14) the heavy chain variable region and the light chain variable region comprise the amino acid sequences set forth in SEQ ID NOs: 33 and 35, or amino acid sequences having at least 80% identity to the amino acid sequences set forth in SEQ ID NOs: 33 and 35, respectively;
(15) the heavy chain variable region and the light chain variable region comprise the amino acid sequences set forth in SEQ ID NOs: 30 and 36, or amino acid sequences having at least 80% identity to the amino acid sequences set forth in SEQ ID NOs: 30 and 36, respectively;
(16) the heavy chain variable region and the light chain variable region comprise the amino acid sequences set forth in SEQ ID NOs: 31 and 36, or amino acid sequences having at least 80% identity to the amino acid sequences set forth in SEQ ID NOs: 31 and 36, respectively;
(17) the heavy chain variable region and the light chain variable region comprise the amino acid sequences set forth in SEQ ID NOs: 32 and 36, or amino acid sequences having at least 80% identity to the amino acid sequences set forth in SEQ ID NOs: 32 and 36, respectively;

(18) the heavy chain variable region and the light chain variable region comprise the amino acid sequences set forth in SEQ ID NOs: 33 and 36, or amino acid sequences having at least 80% identity to the amino acid sequences set forth in SEQ ID NOs: 33 and 36, respectively;

(19) the heavy chain variable region and the light chain variable region comprise the amino acid sequences set forth in SEQ ID NOs: 43 and 44, or amino acid sequences having at least 80% identity to the amino acid sequences set forth in SEQ ID NOs: 43 and 44, respectively;

(20) the heavy chain variable region and the light chain variable region comprise the amino acid sequences set forth in SEQ ID NOs: 51 and 52, or amino acid sequences having at least 80% identity to the amino acid sequences set forth in SEQ ID NOs: 51 and 52, respectively; or

(21) the heavy chain variable region and the light chain variable region comprise the amino acid sequences set forth in SEQ ID NOs: 59 and 60, or amino acid sequences having at least 80% identity to the amino acid sequences set forth in SEQ ID NOs: 59 and 60, respectively.

7. The antibody or the antigen-binding fragment thereof according to any one of claims 1-6, wherein the antibody or the antigen-binding fragment thereof is of the IgG1, IgG2, or IgG4 isotype.

8. The antibody or the antigen-binding fragment thereof according to any one of claims 1-7, wherein the antibody or the antigen-binding fragment thereof further comprises a heavy chain constant region and a light chain constant region, wherein the heavy chain constant region comprises the amino acid sequence set forth in SEQ ID NO: 61 or an amino acid sequence comprising 1, 2, 3, 4, or 5 amino acid substitutions, deletions, or additions compared with the amino acid sequence set forth in SEQ ID NO: 61, and the light chain constant region comprises the amino acid sequence set forth in SEQ ID NO: 62 or an amino acid sequence comprising 1, 2, 3, 4, or 5 amino acid substitutions, deletions, or additions compared with the amino acid sequence set forth in SEQ ID NO: 62.

9. The antibody or the antigen-binding fragment thereof according to any one of claims 1-8, wherein the antibody or the antigen-binding fragment thereof (a) binds to human CLDN18.2; (b) induces antibody-dependent cellular cytotoxicity (ADCC) against cells expressing CLDN18.2; and/or (c) induces complement-dependent cytotoxicity (CDC) against cells expressing CLDN18.2.

10. The antibody or the antigen-binding fragment thereof according to any one of claims 1-9, wherein the antibody or the antigen-binding fragment thereof is selected from a monoclonal antibody, a monospecific antibody, a nanobody, a Fab fragment, a F(ab')2 fragment, an Fd fragment, an Fv fragment, a dAb fragment, an isolated CDR region, a single-chain Fv molecule, and a combination thereof.

11. An isolated nucleic acid molecule encoding the antibody or the antigen-binding fragment thereof according to any one of claims 1-10.

12. An expression vector, comprising the nucleic acid molecule according to claim 11.

13. A host cell, comprising the nucleic acid molecule according to claim 11 or the expression vector according to claim 12.

14. A multispecific antibody, comprising the antibody or the antigen-binding fragment thereof according to any one of claims 1-10.

15. A recombinant polypeptide or fusion protein, comprising the antibody or the antigen-binding fragment thereof according to any one of claims 1-10 and a functional fragment linked thereto.

16. An immunoconjugate, comprising the antibody or the antigen-binding fragment thereof according to any one of claims 1-10 and a therapeutic agent linked thereto.

17. A pharmaceutical composition, comprising the antibody or the antigen-binding fragment thereof according to any one of claims 1-10 and one or more pharmaceutically acceptable carriers or excipients.

18. A method for preventing, alleviating, or treating a CLDN18.2-positive disease in a subject in need thereof, comprising administering to the subject a therapeutically effective amount of the antibody or the antigen-binding fragment thereof according to any one of claims 1-10 or the pharmaceutical composition according to claim 17.

19. The method according to claim 18, wherein the CLDN18.2-positive disease comprises cancer.

**20.** The method according to claim 19, wherein the cancer comprises gastric cancer, esophageal cancer, gastrointestinal cancer, pancreatic cancer, liver cancer, lung cancer, bronchial cancer, mesothelioma, kidney cancer, ovarian cancer, breast cancer, bladder cancer, uterus cancer, prostate cancer, testicular cancer, anal cancer, vaginal cancer, bile duct cancer, gall bladder cancer, head and neck cancer, spinal tumor, otorhinolaryngological tumor, thyroid cancer, mesothelioma, bone cancer, skin cancer, melanoma, adenocarcinoma, sarcoma, neuroblastoma, glioblastoma, brain cancer, central nervous system cancer, neuroendocrine tumor, leukemia, lymphoma, and myeloma.

FIG. 1

FIG. 2

**A**

**ADCC
[KATOIII/PBMC=1:20]**

**B**

**ADCC
[NUGC-4/PBMC=1:20]**

FIG. 3

FIG. 4

**A**

**Internalization activity**
**NUGC-4**

**B**

**Internalization activity**
**NIH-3T3-CLDN18.2**

FIG. 5

**Non-specific ADCC**
**NIH-3T3-CLDN18.1/Jurkat-CD16a v158-NFAT-luciferase**

FIG. 6

Binding activity
[NUGC-4]

A

Binding activity
[KATOIII]

B

FIG. 7

A

**Binding activity**
**[NUGC-4]**

B

**Binding activity**
**[KATOIII]**

FIG. 8

A

ADCC
(U2OS-CLDN18.2/Jurkat-CD16a v158-NFAT-luciferase)

B

ADCC
(KATOIII/Jurkat-CD16a v158-NFAT-luciferase)

FIG. 9

FIG. 10

FIG. 11

FIG. 12

FIG. 13

FIG. 14

## INTERNATIONAL SEARCH REPORT

| International application No. |
|---|
| **PCT/CN2022/129834** |

**A.    CLASSIFICATION OF SUBJECT MATTER**

C07K 16/28(2006.01)i;    A61K 39/395(2006.01)i;    A61P 35/00(2006.01)i

According to International Patent Classification (IPC) or to both national classification and IPC

**B.    FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNABS, SIPOABS, DWPI, CNTXT, ENTXT, ENTXTC, WOTXT, EPTXT, USTXT, 中国专利生物序列检索系统, Chinese Patent Biological Sequence Retrieval System, CNKI, GenBank, EBI-EMBL, PubMed, ISI Web of Knowledge: 申请人/发明人, CLDN18.2, Claudin18.2, CLDN18, 密蛋白18.2, 抗体, mAb, 抗原结合片段, SEQ ID NOs: 3-18

**C.    DOCUMENTS CONSIDERED TO BE RELEVANT**

| Category* | Citation of document, with indication, where appropriate, of the relevant passages | Relevant to claim No. |
|---|---|---|
| A | CN 110862454 A (NANJING SANHOME PHARMACEUTICAL CO., LTD.) 06 March 2020 (2020-03-06)<br>    see claims 1-16, and description, embodiment 10 | 1-20 |
| A | WO 2020063988 A1 (CARSGEN THERAPEUTICS (SHANGHAI) CO., LTD.) 02 April 2020 (2020-04-02)<br>    entire document | 1-20 |
| A | CN 113423735 A (ZAI LAB SHANGHAI CO., LTD.) 21 September 2021 (2021-09-21)<br>    entire document | 1-20 |
| A | WO 2018006882 A1 (CARSGEN THERAPEUTICS (SHANGHAI) CO., LTD. et al.) 11 January 2018 (2018-01-11)<br>    entire document | 1-20 |
| A | WO 2019170147 A1 (CARSGEN THERAPEUTICS (SHANGHAI) CO., LTD. et al.) 12 September 2019 (2019-09-12)<br>    entire document | 1-20 |

☑ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **05 January 2023** | **16 January 2023** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2022/129834** |

### C. DOCUMENTS CONSIDERED TO BE RELEVANT

| Category* | Citation of document, with indication, where appropriate, of the relevant passages | Relevant to claim No. |
| --- | --- | --- |
| A | CN 109762067 A (BEIJING MABWORKS BIOTECH CO., LTD.) 17 May 2019 (2019-05-17)<br>entire document | 1-20 |
| A | CN 112480248 A (SANYOU BIOPHARMACEUTICALS CO., LTD.) 12 March 2021 (2021-03-12)<br>entire document | 1-20 |
| A | CN 113416260 A (NANJING KAEDI MEDICAL TECHNOLOGY CO., LTD.) 21 September 2021 (2021-09-21)<br>entire document | 1-20 |
| A | WO 2019242505 A1 (L&L BIOPHARMA CO., LTD.) 26 December 2019 (2019-12-26)<br>entire document | 1-20 |
| A | 徐良额 等 (XU, Liang'e et al.). "CLDN18.2蛋白在恶性肿瘤治疗中的研究进展 (Advances of CLDN18.2 Protein in the Therapy of Malignant Tumors)"<br>中国肿瘤临床 (Chinese Journal of Clinical Oncology),<br>Vol. 46, No. 9, 31 December 2019 (2019-12-31),<br>pp. 311-315 | 1-20 |
| A | BAEK, J. H. et al. "Clinical Implications of Claudin18.2 Expression in Patients With Gastric Cancer"<br>ANTICANCER RESEARCH, Vol. 39, 31 December 2019 (2019-12-31),<br>pp. 6973-6979 | 1-20 |
| A | SCHULER, M. et al. "Final results of the FAST study, an international, multicenter, randomized, phase II trial of epirubicin, oxaliplatin, and capecitabine (EOX) with or without the anti-CLDN18.2 antibody IMAB362 as first-line therapy in patients with advanced CLDN18.2+ gastric and gastroesophageal junction (GEJ) adenocarcinoma"<br>Annals of Oncology, Vol. 27, No. Supplement 6, 31 December 2016 (2016-12-31),<br>Abstract 614O | 1-20 |

Form PCT/ISA/210 (second sheet) (January 2015)

**INTERNATIONAL SEARCH REPORT**

| International application No. |
|---|
| **PCT/CN2022/129834** |

**Box No. I          Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑  forming part of the international application as filed:

        ☑  in the form of an Annex C/ST.25 text file.

        ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

        ☐  in the form of an Annex C/ST.25 text file (Rule 13*ter*.1(a)).

        ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

    [1]  The actually submitted sequence table is an XML file in Standard ST.26.

Form PCT/ISA/210 (continuation of first sheet) (January 2015)

<table>
<tr><td colspan="2" align="center"><strong>INTERNATIONAL SEARCH REPORT</strong></td><td>International application No.<br><strong>PCT/CN2022/129834</strong></td></tr>
</table>

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **18-20**
   because they relate to subject matter not required to be searched by this Authority, namely:

   [1] Claims 18-20 relate to a method for preventing, alleviating or treating CLDN18.2 positive related diseases in a subject in need, and thus said claims do not comply with PCT Rule 39.1(iv). The present report was formed on the basis that claims 18-20 are amended to relate to a use of the antibody or the antigen-binding fragment thereof according to any one of claims 1-10 or the pharmaceutical composition according to claim 17 in the preparation of drugs for preventing, relieving or treating CLDN18.2 positive related diseases.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |
| --- | --- |

This International Searching Authority found multiple inventions in this international application, as follows:

[1] I: Claims 1-20 (in part) relate to an anti-CLDN18.2 antibody derived from clone 28G3 or an antigen-binding fragment thereof, which is specifically defined by VH as shown in SEQ ID NOs: 11-15 and VL as shown in SEQ ID NOs: 16-18, and/or HCDR1-3 as shown in SEQ ID NOs: 3-7 and LCDR1-3 as shown in SEQ ID NOs: 8-10; and a corresponding nucleic acid molecule, expression vector, host cell, multispecific antibody, recombinant polypeptide or fusion protein, immunoconjugate, pharmaceutical composition and treatment method;

[2] II: Claims 1-20 (in part) relate to an anti-CLDN18.2 antibody derived from clone 40F6 or an antigen-binding fragment thereof, which is specifically defined by VH as shown in SEQ ID NOs: 29-33 and VL as shown in SEQ ID NOs: 34-36, and/or HCDR1-3 as shown in SEQ ID NOs: 19-24 and LCDR1-3 as shown in SEQ ID NOs: 25-28; and a corresponding nucleic acid molecule, expression vector, host cell, multispecific antibody, recombinant polypeptide or fusion protein, immunoconjugate, pharmaceutical composition and treatment method;

[3] III: Claims 1-20 (in part) relate to an anti-CLDN18.2 antibody derived from clone 34G6 or an antigen-binding fragment thereof, which is specifically defined by VH as shown in SEQ ID NO: 43 and VL as shown in SEQ ID NO: 44, and/or HCDR1-3 as shown in SEQ ID NOs: 37-39 and LCDR1-3 as shown in SEQ ID NOs: 40-42; and a corresponding nucleic acid molecule, expression vector, host cell, multispecific antibody, recombinant polypeptide or fusion protein, immunoconjugate, pharmaceutical composition and treatment method;

[4] IV: Claims 1-20 (in part) relate to an anti-CLDN18.2 antibody derived from clone 10D8 or an antigen-binding fragment thereof, which is specifically defined by VH as shown in SEQ ID NO: 51 and Vl as shown in SEQ ID NO: 52, and/or HCDR1-3 as shown in SEQ ID NOs: 45-47 and LCDR1-3 as shown in SEQ ID NOs: 48-50; and a corresponding nucleic acid molecule, expression vector, host cell, multispecific antibody, recombinant polypeptide or fusion protein, immunoconjugate, pharmaceutical composition and treatment method;

[5] V: Claims 1-20 (in part) relate to an anti-CLDN18.2 antibody derived from clone 22F12 or an antigen-binding fragment thereof, which is specifically defined by VH as shown in SEQ ID NO: 59 and VL as shown in SEQ ID NO: 60, and/or HCDR1-3 as shown in SEQ ID NOs: 53-55 and LCDR1-3 as shown in SEQ ID NOs: 56-58; and a corresponding nucleic acid molecule, expression vector, host cell, multispecific antibody, recombinant polypeptide or fusion protein, immunoconjugate, pharmaceutical composition and treatment method.

[6] The same or corresponding technical feature between the inventions I to V is: the anti-CLDN18.2 antibody or the antigen-binding fragment thereof. However, the anti-CLDN18.2 antibody or the antigen-binding fragment

**INTERNATIONAL SEARCH REPORT**

| International application No. |
| --- |
| **PCT/CN2022/129834** |

**Box No. III    Observations where unity of invention is lacking (Continuation of item 3 of first sheet)**

thereof is known in the art, and does not belong to the specific technical features defined in PCT Rule 13.2. Therefore, the inventions I to V lack unity of invention as defined in PCT Rule 13.1.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-20 (in part)**

**Remark on Protest**        ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

| International application No. |
|---|
| **PCT/CN2022/129834** |

| Patent document<br>cited in search report | | | Publication date<br>(day/month/year) | Patent family member(s) | | | Publication date<br>(day/month/year) |
|---|---|---|---|---|---|---|---|
| CN | 110862454 | A | 06 March 2020 | KR | 20210050547 | A | 07 May 2021 |
| | | | | US | 2022119517 | A1 | 21 April 2022 |
| | | | | CN | 112654638 | A | 13 April 2021 |
| | | | | TW | 202023613 | A | 01 July 2020 |
| | | | | JP | 2021535744 | A | 23 December 2021 |
| | | | | EP | 3878863 | A1 | 15 September 2021 |
| | | | | WO | 2020043044 | A1 | 05 March 2020 |
| | | | | CA | 3110593 | A1 | 05 March 2020 |
| WO | 2020063988 | A1 | 02 April 2020 | EP | 3858384 | A1 | 04 August 2021 |
| | | | | US | 2022033491 | A1 | 03 February 2022 |
| | | | | JP | 2022511394 | A | 31 January 2022 |
| | | | | KR | 20210072027 | A | 16 June 2021 |
| | | | | CN | 112888458 | A | 01 June 2021 |
| CN | 113423735 | A | 21 September 2021 | JP | 2022512132 | A | 02 February 2022 |
| | | | | KR | 20210100655 | A | 17 August 2021 |
| | | | | IL | 283754 | A | 29 July 2021 |
| | | | | AU | 2019391204 | A1 | 24 June 2021 |
| | | | | SG | 11202105885 W | A | 29 July 2021 |
| | | | | CA | 3122135 | A1 | 11 June 2020 |
| | | | | BR | 112021011014 | A2 | 31 August 2021 |
| | | | | EP | 3891183 | A1 | 13 October 2021 |
| | | | | WO | 2020114480 | A1 | 11 June 2020 |
| | | | | US | 2021380680 | A1 | 09 December 2021 |
| WO | 2018006882 | A1 | 11 January 2018 | JP | 2019531084 | A | 31 October 2019 |
| | | | | CL | 2019000061 | A1 | 03 May 2019 |
| | | | | EP | 3483182 | A1 | 15 May 2019 |
| | | | | CA | 3030257 | A1 | 11 January 2018 |
| | | | | RU | 2019101430 | A | 10 August 2020 |
| | | | | CN | 109790222 | A | 21 May 2019 |
| | | | | IL | 264144 | A | 28 February 2019 |
| | | | | US | 2019233511 | A1 | 01 August 2019 |
| | | | | KR | 20190038564 | A | 08 April 2019 |
| | | | | BR | 112019000327 | A2 | 13 August 2019 |
| | | | | AU | 2017294276 | A1 | 28 February 2019 |
| | | | | US | 2022185880 | A1 | 16 June 2022 |
| | | | | SG | 11201900171 Q | A | 27 February 2019 |
| WO | 2019170147 | A1 | 12 September 2019 | JP | 2021523090 | A | 02 September 2021 |
| | | | | EP | 3778649 | A1 | 17 February 2021 |
| | | | | KR | 20210013013 | A | 03 February 2021 |
| | | | | US | 2022110973 | A1 | 14 April 2022 |
| | | | | CN | 111989344 | A | 24 November 2020 |
| CN | 109762067 | A | 17 May 2019 | CA | 3104383 | A1 | 23 July 2020 |
| | | | | AU | 2019422603 | A1 | 07 January 2021 |
| | | | | CN | 112399974 | A | 23 February 2021 |
| | | | | WO | 2020147321 | A1 | 23 July 2020 |
| | | | | KR | 20210116207 | A | 27 September 2021 |
| | | | | US | 10421817 | B1 | 24 September 2019 |
| | | | | EP | 3683239 | A1 | 22 July 2020 |
| | | | | JP | 2021509889 | A | 08 April 2021 |

Form PCT/ISA/210 (patent family annex) (January 2015)

INTERNATIONAL SEARCH REPORT
Information on patent family members

International application No.

**PCT/CN2022/129834**

| Patent document cited in search report | | | Publication date (day/month/year) | Patent family member(s) | | | Publication date (day/month/year) |
|---|---|---|---|---|---|---|---|
| CN | 112480248 | A | 12 March 2021 | None | | | |
| CN | 113416260 | A | 21 September 2021 | None | | | |
| WO | 2019242505 | A1 | 26 December 2019 | JP | 2021527441 | A | 14 October 2021 |
| | | | | US | 2021230272 | A1 | 29 July 2021 |
| | | | | EP | 3808376 | A1 | 21 April 2021 |
| | | | | CN | 111867630 | A | 30 October 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202111307619
- WO 2020211792 A
- US 5530101 A
- US 5585089 A
- US 5693762 A
- US 6180370 B
- US 20030153043 A
- US 4816567 A
- US 5225539 A
- DD 120303

### Non-patent literature cited in the description

- **NIIMI et al.** *Mol Cell Biol,* 2001, vol. 21 (21), 7380-7390
- **SAHIN, U. et al.** *Clin Cancer Res.,* 2008, vol. 14, 7624-34
- **TANAKA et al.** *J Histochem Cytochem,* 2011, vol. 59 (10), 942-952
- **MICKE et al.** *Int J Cancer,* 2014, vol. 135 (9), 2206-2214
- **SHIMOBABA et al.** *Biochim Biophys Acta,* 2016, vol. 1863, 1170-1178
- **SING H et al.** *J Hema tol Oncol,* 2017, vol. 10 (1), 105
- **TOKUMITSU et al.** *Cytopathology,* 2017, vol. 28 (2), 116-121
- **SAHIN et al.** *Eur J Cancer,* 2018, vol. 100, 17-26
- **WARD et al.** *Nature,* 1989, vol. 341, 544-546
- **BIRD et al.** *Science,* 1988, vol. 242, 423-426
- **HUSTON et al.** *Proc. Natl. Acad. Sci.,* 1988, vol. 85, 5879-5883
- **KABAT et al.** Sequences of Proteins of Immunological Interest. Public Health Service, National Institutes of Health, 1991
- **AL-LAZIKANI et al.** *JMB,* 1997, vol. 273, 927-948
- **LEFRANC M.P.** *Immunologist,* 1999, vol. 7, 132-136
- **LEFRANC, M.P. et al.** *Dev. Comp. Immunol.,* 2003, vol. 27, 55-77
- **KOHLER ; MILSTEIN.** *Nature,* 1975, vol. 256, 495
- **MORRISON, S.** *Science,* 1985, vol. 229, 1202
- **URLAUB ; CHASIN.** *Proc. Natl. Acad. Sci. USA,* 1980, vol. 77, 4216-4220
- **R. J. KAUFMAN ; P. A. SHARP.** *J. Mol. Biol.,* 1982, vol. 159, 601-621